# EUROPEAN PATENT SPECIFICATION

(11) **EP 3 110 409 B1**
(45) Date of publication and mention of the grant of the patent: **15.08.2018**
(21) Application number: 15710994.3
(22) Date of filing: 27.02.2015
(51) Int. Cl.: A61K 31/00, A61K 31/519, A61K 31/497, A61K 45/06, A61P 35/02

(54) **JAK1 INHIBITORS FOR THE TREATMENT OF MYELODYSPLASTIC SYNDROMES**
JAK1-INHIBITOREN ZUR BEHANDLUNG VON MYELODYSPLASTISCHEN SYNDROMEN
INHIBITEURS DES JAK1 POUR LE TRAITEMENT DE SYNDROMES MYÉLODYSPLASIQUES

(30) Priority: 28.02.2014 US 201461946124 P
(43) Date of publication of application: 04.01.2017
(60) Divisional application: 18179758.0
(73) Proprietor: Incyte Corporation, Wilmington, DE 19803 (US)
(72) Inventor: VADDI, Krishna, Kennett Square, Pennsylvania 19348 (US)
(74) Representative: Carpmaels & Ransford LLP
(86) International application number: PCT/US2015/017963
(87) International publication number: WO 2015/131031

(56) References cited:
- WO-A1-2012/071612
- WO-A1-2014/071031
- WO-A1-2014/186706
- US-A1- 2012 149 681
- US-A1- 2014 004 516
- US-A1- 2014 005 166
- EGHTEDAR A ET AL: "Phase 2 study of the JAK kinase inhibitor ruxolitinib in patients with refractory leukemias, including postmyeloproliferative neoplasm acute myeloid leukemia", BLOOD, vol. 119, no. 20, 17 May 2012 (2012-05-17) , pages 4614-4618, XP002695253, AMERICAN SOCIETY OF HEMATOLOGY, US ISSN: 0006-4971, DOI: 10.1182/BLOOD-2011-12-400051 [retrieved on 2012-03-15]
- Anonymous: "Ruxolitinib for Patients With Low or Intermediate-1 Risk Myelodysplastic Syndrome (MDS)", ClinicalTrials.gov archive , 19 August 2013 (2013-08-19), XP002739581, Retrieved from the Internet: URL:clinicaltrials.gov/archive/NCT01895842 /2013_08_19 [retrieved on 2015-04-30]

## Description

This application claims the benefit of priority of U.S. Prov. Appl. No. 61/946,124, filed February 28, 2014.

### TECHNICAL FIELD

This invention relates to JAK1 selective inhibitors and their use in treating myelodysplastic syndromes (MDS).

### BACKGROUND

Myelodysplastic syndromes (MDS), known previously as dysmyelopoietic syndromes or preleukemia, are heterogeneous and clonal hematopoietic disorders that are characterized by ineffective hematopoiesis on one or more of the major myeloid cell lineages. Myelodysplastic syndromes are associated with bone marrow failure, peripheral blood cytopenias, and a propensity to progress to acute myeloid leukemia (AML). Moreover, clonal cytogenetic abnormalities can be detected in about 50% of cases with MDS. In the general population, MDS occurs in 5 per 100,000 and the incidence increases with age, reaching to about 22 to 45 per 100,000 in individuals older than 70 years (Greenberg, "The myelodysplastic syndromes" in Hoffman, et al, eds. Hematology: Basic Principles and Practice (3rd ed.), Churchill Livingston; 2000:1106-1129; Liesveld and Lichtman, Chapter 88. "Myelodysplastic Syndromes (Clonal Cytopenias and Oligoblastic Myelogenous Leukemia)", in Prchal et al, eds. Williams Hematology. 8th ed., New York: McGraw-Hill; 2010). Despite scientific advances in our knowledge of the pathophysiology of MDS, there are few therapeutic options available and are mostly palliative, especially when affected patients are not candidates for hematopoietic stem cell transplant (HSCT).

The standard of care for MDS includes supportive care that involves observation and clinical monitoring, psychosocial support, and efforts to improve QOL (Cheson, et al, Blood 2000;96:3671-3674; Venugopal et al. Cancer Treat Res 2001;108:257-265; Greenberg, Int J Ped Hem-Onc 1997;4:231-238). In addition, RBC transfusions for symptomatic anemia and platelet transfusions for bleeding episodes from thrombocytopenia are needed. Myelodysplastic syndrome patients requiring RBC transfusions may develop complications including development of alloantibodies requiring increasing transfusion frequency, and iron overload with end-organ damage to the liver, heart, and endocrine organs requiring iron-chelation to maintain serum ferritin at < 1000 µg/L (Venugopal et al 2001 (*supra*), Greenberg 1997 (*supra*)). In cases with refractory symptomatic cytopenia, hematopoietic cytokine support is needed, such as the use of recombinant human granulocyte colony-stimulating factor (G CSF) or granulocyte-monocyte CSF (GM-CSF) for neutropenic MDS with infectious complications, and erythropoiesis-stimulating agent (ESA) for symptomatic anemia (Cheson et al 2000 (*supra*), Jädersten et al, Blood 2005;106:803-811; Schiffer, Hematology Am Soc Hematol Educ Program 2006:205-210). In early stage MDS, ie, IPSS low and IPSS Intermediate-1, symptomatic anemia is the most common reason requiring therapeutic intervention. The ESA benefits only a fraction of these patients with the highest response seen in patients who are not RBC transfusion dependent or those with a low endogenous EPO level (< 500 IU) (Cheson et al 2000 (*supra*), Jädersten et al 2005 (*supra*), Schiffer 2006 (*supra*), Fenaux, et al., Lancet Oncol 2009;10:223-232). Eventually, patients stop responding to ESA therapy and require RBC transfusion support, although ESA is typically continued even when RBC transfusions are needed and reticulocyte counts are low. Transfusion requirement may vary and may be influenced by concomitant medical issues requiring a higher Hgb level such as angina, development of alloantibodies to RBC, splenomegaly, and occult gastrointestinal hemorrhage from thrombocytopenia or platelet dysfunction (Venugopal et al 2001 (*supra*), Greenberg 1997 (*supra*), Fenaux et al 2009 (*supra*)).

Low-intensity therapy includes the use of low-intensity chemotherapy or biological response modifiers (BRM). Hypomethylating agents such as DNA methyltransferase inhibitors 5 azacytidine and decitabine (5-aza-2'-deoxycytidine) have been shown to reduce the risk of leukemic transformation in randomized Phase 3 studies and improve overall survival in a proportion of patients (Fenaux et al 2009 (*supra*), Silverman, J Clin Oncol 2002;20:2429-2440; Silverman, J Clin Oncol 2006;24:3895-3903). Similarly, decitabine has demonstrated a higher disease response rate, duration of remission, time to AML progression, and survival benefit in MDS patients with intermediate-risk and high risk disease. In addition, decitabine demonstrated significant improvement in patient-reported QOL (based on The European Organisation for Research and Treatment of Cancer [EORTC QLQ C30]) for the dimensions of fatigue and physical functioning (Kantarjian, et al., Cancer 2006;106:1794-1803; Lübbert, et al., Br J Haematol 2001;114:349-357; Lübbert, et al., J Clin Oncol 2011;29:1987-1996). Both 5-azacytidine and decitabine are approved for MDS treatment and specifically provides clinical benefit and recommended by the NCCN MDS panel for patients with IPSS intermediate 2 and high-risk MDS (National Comprehensive Cancer Network (NCCN). Myelodysplastic Syndromes Guidelines Version 1. 2012. www.nccn.org/professionals/physician_gls/f_guidelines.asp).

Inflammatory molecules have been implicated as regulatory cues driving the proliferation and apoptotic death of hematopoietic progenitors in MDS. Chronic immune stimulation, coupled with senescence dependent changes in both hematopoietic stem/progenitor cells (HSPC) and the BM microenvironment, are believed to be critical to the pathogenesis of the disease. Increasing evidence implicates the activation of innate immune signaling in both hematopoietic senescence and the pathobiology of MDS (Chen et al., 2014). As such, immune modifiers that include T cell inhibitors such as antithymocyte globulin (ATG), cyclosporine, and thalidomide and its analog lenalidomide (Molldrem, et al., Br J Haematol 1997;99:699-705; Sloand, et al., J Clin Oncol 2008;26:2505-2511; Raza, et al., Blood 2008; 111:86-93; Fenaux, et al., Blood 2011;118:3765-3776; List, et al., N Engl J Med 2005;352:549-557) are used as low intensity agents MDS. High-intensity therapy for MDS include intensive induction chemotherapy, as is used for treating AML and HSCT. Different intensive chemotherapeutic regimens have been tested as they have the potential for altering the natural history of the disease and comparative studies have failed to show benefit; this approach remains investigational and a possible option for MDS patients with high-risk disease. Allogeneic HSCT, the only curative treatment modality for MDS and preferably from a matched sibling donor, is a preferred option for high-risk MDS patients, but the lack of a suitable donor and comorbidities related to advancing age often preclude these patients from undergoing this procedure (NCCN 2012 (*supra*); Larson, Best Pract Res Clin Hematol 2006;19:293-300; Schiffer, Best Pract Res Clin Hematol 2007;20:49-5 A. Eghtedar et al. discloses the treatment of myelodysplastic syndrome with the JAK inhibitor ruxolitinib (Blood, 2012; 119(2): 4614-4618).

Accordingly, there is a need to develop new therapies for the treatment of myelodysplastic syndromes. This application addresses this need and others.

### SUMMARY

The present application provides a JAK1 selective inhibitor as defined in the claims for use in a method of treating a myelodysplastic syndrome in a patient in need thereof.

### DETAILED DESCRIPTION

The scope of the invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human (or animal) body by therapy. The methods described herein utilize JAK1 selective inhibitors. A JAK1 selective inhibitor is a compound that inhibits JAK1 activity preferentially over other Janus kinases. JAK1 plays a central role in a number of cytokine and growth factor signaling pathways that, when dysregulated, can result in or contribute to disease states. For example, IL-6 levels are elevated in rheumatoid arthritis, a disease in which it has been suggested to have detrimental effects (Fonesca, et al., Autoimmunity Reviews, 8:538-42, 2009). Because IL-6 signals, at least in part, through JAK1, antagonizing IL-6 directly or indirectly through JAK1 inhibition is expected to provide clinical benefit (Guschin, et al Embo J 14:1421, 1995; Smolen, et al. Lancet 371:987, 2008). Moreover, in some cancers JAK1 is mutated resulting in constitutive undesirable tumor cell growth and survival (Mullighan, Proc Natl Acad Sci U S A. 106:9414-8, 2009; Flex, J Exp Med. 205:751-8, 2008). In other autoimmune diseases and cancers elevated systemic levels of inflammatory cytokines that activate JAK1 may also contribute to the disease and/or associated symptoms. Therefore, patients with such diseases may benefit from JAK1 inhibition. Selective inhibitors of JAK1 may be efficacious while avoiding unnecessary and potentially undesirable effects of inhibiting other JAK kinases.

Selective inhibitors of JAK1, relative to other JAK kinases, may have multiple therapeutic advantages over less selective inhibitors. With respect to selectivity against JAK2, a number of important cytokines and growth factors signal through JAK2 including, for example, erythropoietin (Epo) and thrombopoietin (Tpo) (Parganas, et al. Cell. 93:385-95, 1998). Epo is a key growth factor for red blood cells production; hence a paucity of Epo-dependent signaling can result in reduced numbers of red blood cells and anemia (Kaushansky, NEJM 354:2034-45, 2006). Tpo, another example of a JAK2-dependent growth factor, plays a central role in controlling the proliferation and maturation of megakaryocytes - the cells from which platelets are produced (Kaushansky, NEJM 354:2034-45, 2006). As such, reduced Tpo signaling would decrease megakaryocyte numbers (megakaryocytopenia) and lower circulating platelet counts (thrombocytopenia). This can result in undesirable and/or uncontrollable bleeding. Reduced inhibition of other JAKs, such as JAK3 and Tyk2, may also be desirable as humans lacking functional version of these kinases have been shown to suffer from numerous maladies such as severe-combined immunodeficiency or hyperimmunoglobulin E syndrome (Minegishi, et al. Immunity 25:745-55, 2006; Macchi, et al. Nature, 377:65-8, 1995). Therefore a JAK1 inhibitor with reduced affinity for other JAKs would have significant advantages over a less-selective inhibitor with respect to reduced side effects involving immune suppression, anemia and thrombocytopenia.

Inflammatory cytokines play a significant role in the pathogenesis of MDS, which results in cytopenias and dysplastic hematopoiesis. It is hypothesized that curbing the activity of these inflammatory cytokines will promote normal hematopoiesis and relieve the marrow precursors from premature apoptosis. The inflammatory cytokines mediate downstream effects by JAK activation which involves juxtapositioning of JAKs from ligand-mediated receptor dimerization and trans/autophosphorylation. The resultant JAK heterodimers, comprised of JAK1 and JAK2 or JAK1 and JAK3, transduce signals and mediate cellular responses of these cytokines. Moreover, JAK homodimers, comprised of only JAK2, transduce signals from bone marrow growth factors such as EPO, which is responsible for stimulating erythropoiesis, and TPO, which is responsible for stimulating thrombopoiesis. Therefore, a selective JAK1 inhibitor would result in abrogation of the inflammatory cytokine signaling without inhibiting JAK2-mediated erythropoiesis and thrombopoiesis, resulting in the reestablishment of normal hematopoiesis and alleviation of myeloid cytopenias.

Despite scientific advances in our knowledge of the pathophysiology of MDS, there are few therapeutic options available and are mostly palliative, especially when affected patients are not candidates for HSCT. A number of studies have indicated that MDS is a clonal disease and demonstrated that the expanded clone was a result of excessive proliferation of hematopoietic progenitors in the bone marrow. The paradox of a hyperproliferative state in the marrow leading to peripheral cytopenias was investigated and revealed that there was an excessive amount of intramedullary programmed cell death or apoptosis of the hematopoietic cells. This apoptosis was seen in patients with all the FAB categories but decreased in patients with increasing blast counts. It appeared that a clonal population progressively became resistant to apoptosis and gained a proliferative advantage over the normal hematopoietic precursors that led to the increase in blast counts and evolution to AML. It also became evident that the excessive apoptosis was largely mediated by a number of proinflammatory cytokines that are overexpressed in the marrows of patients with MDS. The cytokines that have been implicated in the pathobiology of MDS include tumor necrosis factor-alpha (TNF-α), interferon-gamma (IFN γ) and IL 1β. High plasma concentration of TNF-α, a classic proapoptotic cytokine, has been observed in the peripheral blood and the bone marrow of patients with MDS and a higher expression of TNF receptors and messenger ribonucleic acid (mRNA) has been observed in bone marrow mononuclear cells derived from MDS patients. Similarly, an increased amount of IFN γ and IL 1β has been found in MDS bone marrow mononuclear cells and IL-1β has been implicated in the evolution of AML from MDS. The IL-1β has variable regulatory effects on the hematopoietic cells as it stimulates GM-CSF and IL-3, whereas in higher concentrations as seen in inflammatory states, it leads to suppression of hematopoiesis by induction of TNF α and prostaglandin E2, the latter being a potent suppressor of myeloid stem cell proliferation. In addition, high levels of IL-6, fibroblast growth factor, hepatocyte growth factor, and transforming growth factor β has been seen in myeloid cells from MDS patients. Moreover, the very cytokines that suppress the normal hematopoietic proliferation and maturation fail to exert this proapoptotic effect on the evolving abnormal clone that results in selective proliferation of these abnormal cells. There is evidence that the source of these proinflammatory cytokines is the altered bone marrow microenvironment that normally nurtures the normal hematopoietic cells to proliferate and differentiate, and may also be the reason for the infiltration of immune regulatory cells and angiogenesis that contributes to the pathobiology of MDS (Raza, et al., Blood 1995;86:268-276; Raza, et al, Int J Hematol 1996a;63:265-278; Raza, et al., Leuk Res 1996b;20:881-890; Mundle, et al. Am J Hematol 1999;60:36-47; Claessens, et al., Blood 2002;99:1594-1601).

The concept that the cytokine-mediated proinflammatory state is responsible for the etiology of MDS has led to the novel approach of anticytokine therapy to improve the cytopenias in MDS by protecting the differentiating hematopoietic cells from premature apoptosis. There has been demonstration that anti-TNF-a agents like thalidomide and its analog lenalidomide, infliximab, and etanercept have been effective in improving cytopenias in MDS patients (NCCN 2012 (s*upra*), Larson 2006 (*supra*), Schiffer 2007(*supra*)). In a study of 14 MDS patients, etanercept showed erythroid hematologic improvement in 25% of patients along with improvement in platelet counts and ANC in 12.5% of evaluable patients. Additionally combining intermittent etanercept with ATG resulted in a higher erythroid hematologic improvement and 5 of the 14 MDS patients requiring transfusion became RBC and platelet transfusion independent that lasted beyond 2 years. In a study investigating thalidomide in MDS, of the 83 patients enrolled, 51 completed 12 weeks of therapy and 16 patients showed hematologic improvement with 10 previously transfusion-dependent patients becoming transfusion independent and most of the responders were in either the IPSS low-risk or intermediate 1 risk category (NCCN 2012 (*supra*)). Moreover, higher risk category patients, especially with a high blast percentage, tended to discontinue treatment early. Another approach to curb the effects of the proinflammatory cytokines is to inhibit their cellular responses. A considerable number of cytokine and growth factor receptors utilize the JAK family of nonreceptor TYKs to transmit extracellular ligand binding into a cellular response via the transcription factor STAT signaling.

There are 4 members of JAKs: JAK1, JAK2, JAK3, and TYK2. The JAKs are constitutively associated with cytokine and growth factor receptors and have become activated as an immediate consequence of ligand-induced receptor dimerization, JAK activation occurs upon the subsequent juxtapositioning of the JAKs and the trans/autophosphorylation of conserved tyrosines found in the activation loop of the JAK catalytic domain. Upon phosphorylation of these tyrosines, the JAKs enter a high-activity state and are then able to phosphorylate specific tyrosine residues on the cytokine receptors, which serve as docking sites for multiple proteins, including the STAT proteins. The JAKs are the principal family of kinases associated with STAT activation. Activated STATs translocate to the nucleus where they function as transcription factors and drive the expression of multiple genes important for cell activation, localization, survival, and proliferation.

Ruxolitinib, a JAK1 and JAK2 inhibitor has demonstrated marked reduction in spleen size in patients with myelofibrosis and improvement of symptoms. These improvements were apparent in subjects with and without the presence of the V617F mutation in JAK2 and are likely related to inhibition of proinflammatory cytokines. The primary adverse events (AEs) observed with ruxolitinib are thrombocytopenia and anemia; both were infrequently the cause of study discontinuation in a double-blind, placebo-controlled Phase 3 study, and both are due at least in part to JAK2-mediated myelosuppression. It is therefore hypothesized that selective inhibition of JAK1 would exert a salutary effect of inhibiting proinflammatory cytokines in patients with MDS and result in the amelioration of the cytopenias resulting from premature apoptosis of the hematopoietic precursors. Moreover, sparing of JAK2 activity would allow the physiological activity of hematopoietic cytokines namely EPO and TPO to allow physiological proliferation and differentiation of the normal hematopoietic cells.

Further, iron overload frequently occurs in MDS patients, with recent data suggesting an impact on both overall and leukemia-free survival. It has been postulated that an altered production of hepcidin, the recently discovered key hormone regulating iron homeostasis, may play a role in this regard and be regulated by inflammatory cytokines like IL-6. It has recently been shown in MDS that both hepcidin and CRP, as a marker of general inflammation, are elevated. Santini, et al., PLoS One, 6(8), e23109, pages 1-8 (2011). These data suggest that JAK inhibition, which can reduce CRP and hepcidin levels, may reverse the inflammation and iron overload that occurs in MDS.

Accordingly, the present application provides, *inter alia,* a method of treating a myelodysplastic syndrome in a patient in need thereof, comprising administering to said patient a therapeutically effective amount of a JAK1 selective inhibitor, or a pharmaceutically acceptable salt thereof. As used herein, a myelodysplastic syndrome refers to the classification of MDS proposed by the World Health Organization in 2008 (see e.g., Table 1). In particular, in 1997, the World Health Organization (WHO) in conjunction with the Society for Hematopathology (SH) and the European Association of Hematopathology (EAHP) proposed new classifications for hematopoietic neoplasms (Harris, et al., J Clin Oncol 1999;17:3835-3849; Vardiman, et al., Blood 2002;100:2292-2302). For MDS, the WHO utilized not only the morphologic criteria from the French-American-British (FAB) classification but also incorporated available genetic, biologic, and clinical characteristics to define subsets of MDS (Bennett, et al., Br J Haematol 1982;51:189-199). In 2008, the WHO classification of MDS (Table 1) was further refined to allow precise and prognostically relevant subclassification of unilineage dysplasia by incorporating new clinical and scientific information (Vardiman, et al., Blood 2009;114:937-951; Swerdlow, et al., WHO Classification of Tumours of Haematopoietic and Lymphoid Tissues. 4th Edition. Lyon France: IARC Press; 2008:88-103; Bunning and Germing, "Myelodysplastic syndromes/neoplasms" in Chapter 5, Swerdlow, et al, eds. WHO Classification of Tumours of Haematopoietic and Lymphoid Tissues. (ed. 4th edition): Lyon, France: IARC Press;2008:88-103).

**Table 1: 2008 WHO Classification for De Novo Myelodysplastic Syndrome**

| **Subtype** | **Blood** | **Bone Marrow** |
|---|---|---|
| Refractory cytopenia with unilineage dysplasia (RCUD) | Single or Bicytopenia | Dysplasia in ≥ 10% of 1 cell line, < 5% blasts |
| Refractory anemia with ring sideroblasts (RARS) | Anemia, no blasts | ≥ 15% of erythroid precursors w/ring sideroblasts, erythroid dysplasia only, < 5% blasts |
| Refractory cytopenia with multilineage dysplasia | Cytopenia(s), < 1 × 10⁹/L monocytes | Dysplasia in ≥ 10% of cells in ≥ 2 hematopoietic lineages, ± 15% ring sideroblasts, < 5% blasts |
| Refractory anemia with excess blasts-1 (RAEB-1) | Cytopenia(s), ≤ 2% to 4% blasts, < 1 × 10⁹/L monocytes | Unilineage or multilineage dysplasia, No Auer rods, 5% to 9% blasts |
| Refractory anemia with excess blasts-2 (RAEB-2) | Cytopenia(s), ≤ 5% to 19% blasts, < 1 × 10⁹/L monocytes | Unilineage or multilineage dysplasia, ± Auer rods, 10% to 19% blasts |
| Myelodysplastic syndrome, unclassified (MDS-U) | Cytopenias | Unilineage or no dysplasia but characteristic MDS cytogenetics, < 5% blasts |
| MDS associated with isolated del(5q) | Anemia, platelets normal or increased | Unilineage erythroid. Isolated del(5q), < 5% blasts |

In some embodiments, the JAK1 selective inhibitor is selective for JAK1 over JAK2, JAK3, and TYK2. For example, the compounds described herein, or a pharmaceutically acceptable salt thereof, preferentially inhibit JAK1 over one or more of JAK2, JAK3, and TYK2. In some embodiments, the compounds inhibit JAK1 preferentially over JAK2 (e.g., have a JAK1/JAK2 ICso ratio >1). In some embodiments, the compounds or salts are about 10-fold more selective for JAK1 over JAK2. In some embodiments, the compounds or salts are about 3-fold, about 5-fold, about 10-fold, about 15-fold, or about 20-fold more selective for JAK1 over JAK2 as calculated by measuring IC₅₀ at 1 mM ATP (e.g., see Example A). According to the present invention, the JAK1 selective inhibitor is a compound of Table 2, or a pharmaceutically acceptable salt thereof.

**Table 2**

| Prep in Ex. No. | Name | Structure | JAK1 IC₅₀ (nM) | JAK2/ JAK1 |
|---|---|---|---|---|
| 1^{a} | 3-[1-(6-chloropyridin-2-yl)pyrrolidin-3-yl]-3-[4-(7H-pyrrolo[2,3-d]pyrimidin-4-yl)-1H-pyrazol-1-yl]propanenitrile | 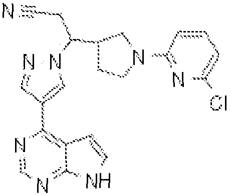 | + | >10 |
| 2 | 3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile | 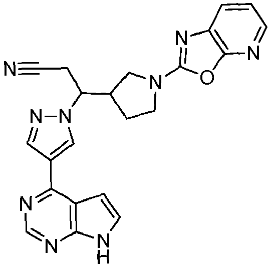 | + | >10 |
| 3 | 4-[(4-{3-cyano-2-[4-(7H-pyrrolo[2,3-d]pyrimidin-4-yl)-1H-pyrazol-1-yl]propyl}piperazin-1-yl)carbonyl]-3-fluorobenzonitrile | 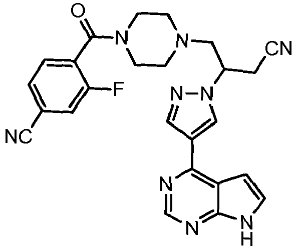 | + | >10 |
| 4 | 4-[(4-{3-cyano-2-[3-(7H-pyrrolo[2,3-d]pyrimidin-4-yl)-1H-pyrrol-1-yl]propyl}piperazin-1-yl)carbonyl]-3-fluorobenzonitrile | 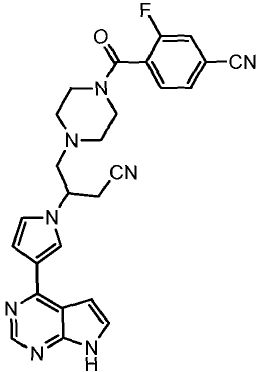 | + | >10 |
| 5 | {1-{1-[3-Fluoro-2-(trifluoromethyl)isonicotino yl]piperidin-4-yl}-3-[4-(7H-pyrrolo[2,3-d]pyrimidin-4-yl)-1H-pyrazol-1-yl]azetidin-3-yl}acetonitrile | 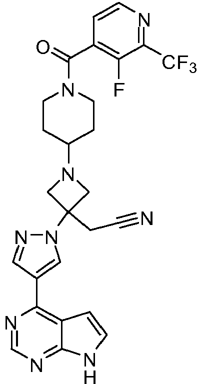 | + | >10 |
| 6 | 4-{3-(Cyanomethyl)-3-[4-(7H-pyrrolo[2,3-d]pyrimidin-4-yl)-1H-pyrazol-1-yl]azetidin-1-yl}-N-[4-fluoro-2-(trifluoromethyl)phenyl]pip eridine-1-carboxamide | 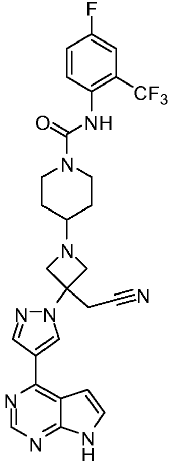 | + | >10 |
| 7 | [3-[4-(7H-pyrrolo[2,3-d]pyrimidin-4-yl)-1H-pyrazol-1-yl]-1-(1-{[2-(trifluoromethyl)pyrimidin-4-yl]carbonyl}piperidin-4-yl)azetidin-3-yl]acetonitrile | 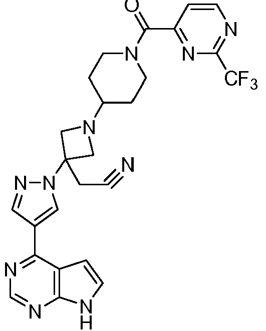 | + | >10 |
| 8 | [*trans*-1-[4-(7H-pyrrolo[2,3-d]pyrimidin-4-yl)-1H-pyrazol-1-yl]-3-(4-{[2-(trifluoromethyl)pyrimidin-4-yl]carbonyl}piperazin-1-yl)cyclobutyl]acetonitrile | 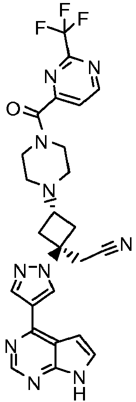 | + | >10 |
| 9 | {*trans*-3-(4-{[4-[(3-hydroxyazetidin-1-yl)methyl]-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile | 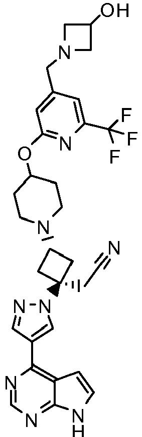 | + | >10 |
| 10 | {*trans*-3-(4-{[4-{[(2S)-2-(hydroxymethyl)pyrrolidin-1-yl]methyl}-6-(trifluoromethyl)pyridin-2-yl]oxy}piperidin-1-yl)-1-[4-(7H-pyrrolo[2,3-d]pyrimidin-4-yl)-1H-pyrazol-1-yl] cyclobutyl}acetonitrile | 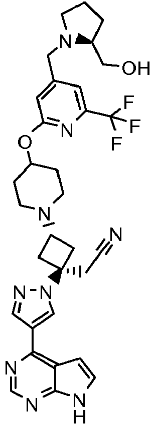 | + | >10 |
| 11 | {*trans-*3-(4-{[4-{[(2R)-2-(hydroxymethyl)pyrrolidin-1-yl]methyl}-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile | 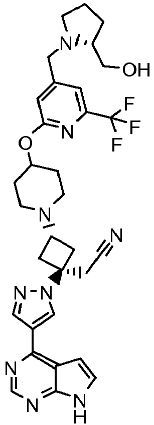 | + | >10 |
| 12^{b} | 4-(4-{3-[(dimethylamino)methyl]-5-fluorophenoxy}piperidin-1-yl)-3-[4-(7H-pyrrolo[2,3-d]pyrimidin-4-yl)-1H-pyrazol-1-yl]butanenitrile | 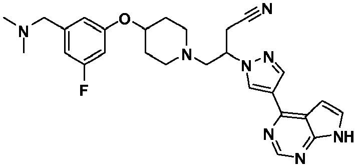 | + | >10 |
| 13 | 5-{3-(cyanomethyl)-3-[4-(7H-pyrrolo[2,3-d]pyrimidin-4-yl)-1H-pyrazol-1-yl]azetidin-1-yl}-N-isopropylpyrazine-2-carboxamide | 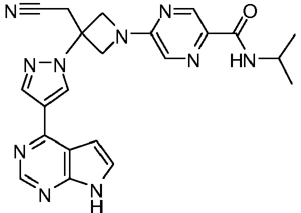 | + | >10 |
| 14 | 4-{3-(cyanomethyl)-3-[4-(7H-pyrrolo[2,3-d]pyrimidin-4-yl)-1H-pyrazol-1-yl]azetidin-1-yl}-2,5-difluoro-N-[(1S)-2,2,2-trifluoro-1-methylethyl]benzamide | 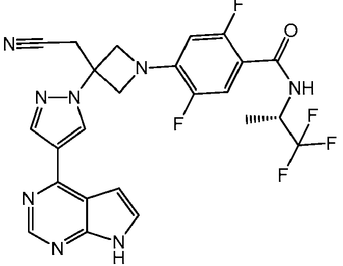 | + | >10 |
| 15 | 5-{3-(cyanomethyl)-3-[4-(1H-pyrrolo [2,3-b]pyridin-4-yl)-1H-pyrazol-1-yl]azetidin-1-yl}-N-isopropylpyrazine-2-carboxamide | 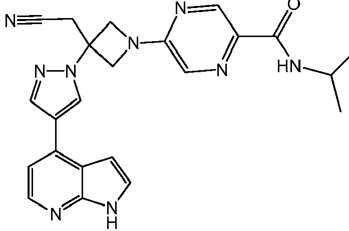 | + | >10 |
| 16 | {1-(*cis*-4-{[6-(2-hydroxyethyl)-2-(trifluoromethyl)pyrimidin-4-yl]oxy}cyclohexyl)-3-[4-(7H-pyrrolo[2,3-d]pyrimidin-4-yl)-1H-pyrazol-1-yl]azetidin-3-yl}acetonitrile | 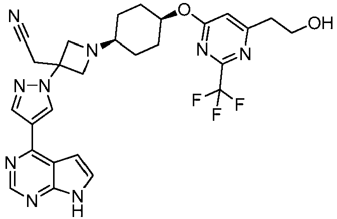 | + | >10 |
| 17 | {1-(*cis*-4-{[4-[(ethylamino)methyl]-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile | 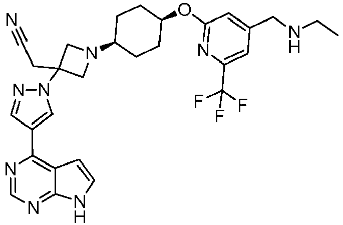 | + | >10 |
| 18 | {1-(*cis-*4-{[4-(1-hydroxy-1-methylethyl)-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile | 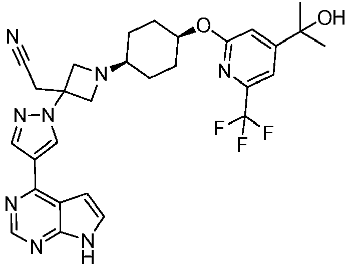 | + | >10 |
| 19 | {1-(*cis*-4-{[4-{[(3R)-3-hydroxypyrrolidin-1-yl]methyl}-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile | 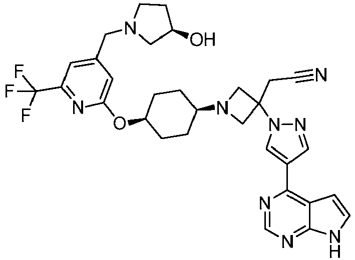 | + | >10 |
| 20 | {1-(*cis*-4-{[4-{[(3S)-3-hydroxypyrrolidin-1-yl]methyl}-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile | 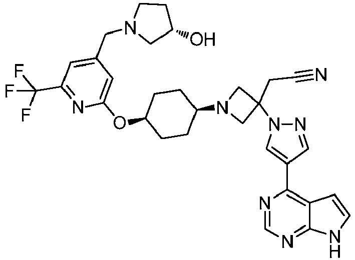 | + | >10 |
| 21 | {*trans*-3-(4-{[4-({[(1S)-2-hydroxy-1-methylethyl]amino}methyl) -6-(trifluoromethyl)pyridin-2-yl]oxy}pipendm-1-yl)-1-[4-(7H-pyrrolo[2,3-d]pyrimidin-4-yl)-1H-pyrazol-1-yl] cyclobutyl}acetonitrile | 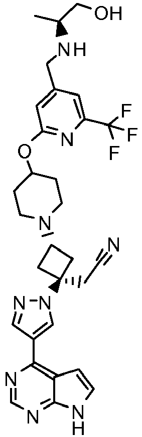 | + | >10 |
| 22 | {*trans*-3-(4-{[4-({[(2R)-2-hydroxypropyl]amino}meth yl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile | 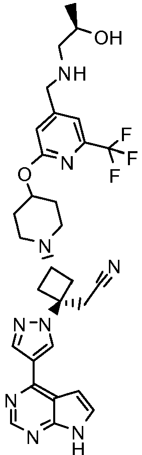 | + | >10 |
| 23 | {*trans*-3-(4-{[4-({[(2S)-2-hydroxypropyl] amino} meth yl)-6-(trifluoromethyl)pyridin-2-yl]oxy}piperidin-1-yl)-1-[4-(7H-pyrrolo[2,3-d]pyrimidin-4-yl)-1H-pyrazol-1-yl] cyclobutyl}acetonitrile | 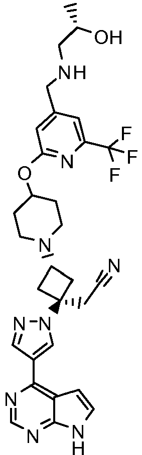 | + | >10 |
| 24 | {*trans*-3-(4-{[4-(2-hydroxyethyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile | 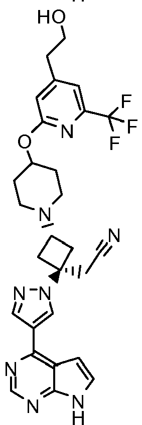 | + | >10 |

| | | | | |
|---|---|---|---|---|
| + means <10 nM (see Example A for assay conditions) ^{a}Data for enantiomer 1 ^{b}Data for enantiomer 2 | | | | |

In some embodiments, the JAK1 selective inhibitor is {1-{1-[3-Fluoro-2-(trifluoromethyl)isonicotinoyl]piperidin-4-yl}-3-[4-(7H-pyrrolo[2,3-d]pyrimidin-4-yl)-1H-pyrazol-1-yl]azetidin-3-yl}acetonitrile adipic acid salt.

In some embodiments, the JAK1 selective inhibitor is selected from (R)-3-[1-(6-chloropyridin-2-yl)pyrrolidin-3-yl]-3-[4-(7H-pyrrolo[2,3-d]pyrimidin-4-yl)-1H-pyrazol-1-yl]propanenitrile, (R)-3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile, (R)-4-[(4-{3-cyano-2-[4-(7H-pyrrolo[2,3-d]pyrimidin-4-yl)-1H-pyrazol-1-yl]propyl}piperazin-1-yl)carbonyl]-3-fluorobenzonitrile, (R)-4-[(4-{3-cyano-2-[3-(7H-pyrrolo[2,3-d]pyrimidin-4-yl)-1H-pyrrol-1-yl]propyl}piperazin-1-yl)carbonyl]-3-fluorobenzonitrile, or (R)-4-(4-{3-[(dimethylamino)methyl]-5-fluorophenoxy}piperidin-1-yl)-3-[4-(7H-pyrrolo[2,3-d]pyrimidin-4-yl)-1H-pyrazol-1-yl]butanenitrile; or a pharmaceutically acceptable salt of any of the aforementioned.

In some embodiments, the JAK1 selective inhibitor is selected from (S)-3-[1-(6-chloropyridin-2-yl)pyrrolidin-3-yl]-3-[4-(7H-pyrrolo[2,3-d]pyrimidin-4-yl)-1H-pyrazol-1-yl]propanenitrile, (S)-3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile, (S)-4-[(4-{3-cyano-2-[4-(7H-pyrrolo[2,3-d]pyrimidin-4-yl)-1H-pyrazol-1-yl]propyl}piperazin-1-yl)carbonyl]-3-fluorobenzonitrile, (S)-4-[(4-{3-cyano-2-[3-(7H-pyrrolo[2,3-d]pyrimidin-4-yl)-1H-pyrrol-1-yl]propyl}piperazin-1-yl)carbonyl]-3-fluorobenzonitrile, or (S)-4-(4-{3-[(dimethylamino)methyl]-5-fluorophenoxy}piperidin-1-yl)-3-[4-(7H-pyrrolo[2,3-d]pyrimidin-4-yl)-1H-pyrazol-1-yl]butanenitrile; or a pharmaceutically acceptable salt of any of the aforementioned. In a non-claimed aspect of the disclosure, the JAK1 selective inhibitor is GLPG0634 (Galapagos).

In some embodiments, the compounds of Table 2 are prepared by the synthetic procedures described in US Patent Publ. No. 2010/0298334, filed May 21, 2010, US Patent Publ. No. 2011/0059951, filed August 31, 2010, US Patent Publ. No. 2011/0224190, filed March 9, 2011, US Patent Publ. No. 2012/0149681, filed November 18, 2011, US Patent Publ. No. 2012/0149682, filed November 18, 2011, US Patent Publ. 2013/0018034, filed June 19, 2012, US Patent Publ. No. 2013/0045963, filed August 17, 2012, and US Patent Publ. No. 2014/0005166, filed May 17, 2013. In some aspects, the JAK1 inhibitor is selected from the compounds of US Patent Publ. No. 2010/0298334, filed May 21, 2010, US Patent Publ. No. 2011/0059951, filed August 31, 2010, US Patent Publ. No. 2011/0224190, filed March 9, 2011, US Patent Publ. No. 2012/0149681, filed November 18, 2011, US Patent Publ. No. 2012/0149682, filed November 18, 2011, US Patent Publ. 2013/0018034, filed June 19, 2012, US Patent Publ. No. 2013/0045963, filed August 17, 2012, and US Patent Publ. No. 2014/0005166, filed May 17, 2013. In some embodiments, the myelodysplastic syndrome is refractory cytopenia with unilineage dysplasia (RCUD).

In some embodiments, the myelodysplastic syndrome is refractory anemia with ring sideroblasts (RARS).

In some embodiments, the myelodysplastic syndrome is refractory cytopenia with multilineage dysplasia.

In some embodiments, the myelodysplastic syndrome is refractory anemia with excess blasts-1 (RAEB-1).

In some embodiments, the myelodysplastic syndrome is refractory anemia with excess blasts-2 (RAEB-2).

In some embodiments, the myelodysplastic syndrome is myelodysplastic syndrome, unclassified (MDS-U).

In some embodiments, the myelodysplastic syndrome is myelodysplastic syndrome associated with isolated del(5q).

In some embodiments, the myelodysplastic syndrome is refractory to erythropoiesis-stimulating agents (ESAs). In some embodiments, refractory to ESAs means no improvement in Hgb of at least 1.5 g/dL after 8 weeks of at least 40,000 IU/week of erythropoietin (EPO) (or equivalent).

In some embodiments, the patient is red blood cell (RBC) transfusion dependent. In some embodiments, red blood cell transfusion dependent means the patient requires at least 4 units of packed RBCs for a Hgb of < 9 g/dL over the 8 weeks prior to treatment.

In some embodiments, the patient has elevated serum hepcidin levels as compared to a control group of healthy individuals. In some embodiments, the patient has an elevated serum c-reactive protein (CRP) concentration as compared to a control group of healthy individuals. In some embodiments, an elevated serum concentration of CRP is one that is equal to or greater than about 10 µg/mL. In some embodiments, healthy individuals are as defined in Santini, et al., PLoS One, 6(8), e23109, pages 1-8 (2011). In some embodiments, the patient has a modified Glasgow Prognostic Score of 1 or 2. The modified Glasgow Prognosis Score (GPS) is described in McMillian, Cancer Treatment Reviews, 39 (5):534-540 (2013) (and in particular, the scores as shown in Table 1).

In some embodiments, the present invention provides a compound described herein, or a pharmaceutically acceptable salt thereof, as described in any of the embodiments herein, for use in a method of treating any of the diseases or disorders described herein. In some embodiments, the present invention provides the use of s a compound described herein, or a pharmaceutically acceptable salt thereof, as described in any of the embodiments herein, for the preparation of a medicament for use in a method of treating any of the diseases or disorders described herein.

The JAK1 selective inhibitors also include pharmaceutically acceptable salts of the compounds described herein. As used herein, "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the non-toxic salts of the parent compound formed, for example, from non-toxic inorganic or organic acids. The pharmaceutically acceptable salts can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, alcohols (e.g., methanol, ethanol, isopropanol, or butanol) or acetonitrile (ACN) are preferred. Lists of suitable salts are found in Remington's Pharmaceutical Sciences, 17th ed., Mack Publishing Company, Easton, Pa., 1985, p. 1418 and Journal of Pharmaceutical Science, 66, 2 (1977). In some embodiments, the compounds described herein include the N-oxide forms.

The compounds described herein can be asymmetric (e.g., having one or more stereocenters). All stereoisomers, such as enantiomers and diastereomers, are intended unless otherwise indicated. Compounds described herein that contain asymmetrically substituted carbon atoms can be isolated in optically active or racemic forms. Methods on how to prepare optically active forms from optically inactive starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. *Cis* and *trans* geometric isomers of the compounds of the present invention are described and may be isolated as a mixture of isomers or as separated isomeric forms.

Resolution of racemic mixtures of compounds can be carried out by any of numerous methods known in the art. An example method includes fractional recrystallizaion using a chiral resolving acid which is an optically active, salt-forming organic acid. Suitable resolving agents for fractional recrystallization methods are, for example, optically active acids, such as the D and L forms of tartaric acid, diacetyltartaric acid, dibenzoyltartaric acid, mandelic acid, malic acid, lactic acid or the various optically active camphorsulfonic acids such as β-camphorsulfonic acid. Other resolving agents suitable for fractional crystallization methods include stereoisomerically pure forms of α-methylbenzylamine (*e.g., S* and *R* forms, or diastereomerically pure forms), 2-phenylglycinol, norephedrine, ephedrine, N-methylephedrine, cyclohexylethylamine, 1,2-diaminocyclohexane, and the like.

Resolution of racemic mixtures can also be carried out by elution on a column packed with an optically active resolving agent (e.g., dinitrobenzoylphenylglycine). Suitable elution solvent composition can be determined by one skilled in the art.

Compounds described herein also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, enamine - imine pairs, and annular forms where a proton can occupy two or more positions of a heterocyclic system, for example, 1H- and 3H-imidazole, 1H-, 2H- and 4H- 1,2,4-triazole, 1H- and 2H-isoindole, and 1H- and 2H-pyrazole. Tautomeric forms can be in equilibrium or sterically locked into one form by appropriate substitution.

Compounds of the invention can also include all isotopes of atoms occurring in the intermediates or final compounds. Isotopes include those atoms having the same atomic number but different mass numbers. For example, isotopes of hydrogen include deuterium.

The term, "compound," as used herein is meant to include all stereoisomers, geometric iosomers, tautomers, and isotopes of the structures depicted. Further, compounds herein identified by name or structure as one particular tautomeric form are intended to include other tautomeric forms unless otherwise specified.

All compounds, and pharmaceutically acceptable salts thereof, can be found together with other substances such as water and solvents (e.g., hydrates and solvates) or can be isolated.

In some embodiments, the compounds described herein, or salts thereof, are substantially isolated. By "substantially isolated" is meant that the compound is at least partially or substantially separated from the environment in which it was formed or detected. Partial separation can include, for example, a composition enriched in the compounds of the invention. Substantial separation can include compositions containing at least about 50%, at least about 60%, at least about 70%, at least about 80%, at least about 90%, at least about 95%, at least about 97%, or at least about 99% by weight of the compounds of the invention, or salt thereof. Methods for isolating compounds and their salts are routine in the art.

As used herein, the term "individual" or "patient," used interchangeably, refers to any animal, including mammals, preferably mice, rats, other rodents, rabbits, dogs, cats, swine, cattle, sheep, horses, or primates, and most preferably humans.

As used herein, the phrase "therapeutically effective amount" refers to the amount of active compound or pharmaceutical agent that elicits the biological or medicinal response that is being sought in a tissue, system, animal, individual or human by a researcher, veterinarian, medical doctor or other clinician. In some embodiments, the therapeutically effective amount is about 1 mg to about 100 mg, about 1 mg to about 20 mg, about 4 mg to about 10 mg, about 5 mg to about 1000 mg, or about 10 mg to about 500 mg. In some embodiments, the therapeutically effective amount is 4 mg, 6 mg, or 10 mg QD.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "treating" or "treatment" refers to one or more of (1) preventing the disease; for example, preventing a disease, condition or disorder in an individual who may be predisposed to the disease, condition or disorder but does not yet experience or display the pathology or symptomatology of the disease; (2) inhibiting the disease; for example, inhibiting a disease, condition or disorder in an individual who is experiencing or displaying the pathology or symptomatology of the disease, condition or disorder (i.e., arresting further development of the pathology and/or symptomatology); and (3) ameliorating the disease; for example, ameliorating a disease, condition or disorder in an individual who is experiencing or displaying the pathology or symptomatology of the disease, condition or disorder (i.e., reversing the pathology and/or symptomatology) such as decreasing the severity of disease.

### Combination Therapies

The methods described herein can further comprise administering one or more additional therapeutic agents. The one or more additional therapeutic agents can be administered to a patient simultaneously or sequentially.

In some embodiments, the method further comprises administering an additional therapeutic agent selected from IMiDs, an anti-IL-6 agent, an anti-TNF-α agent, a hypomethylating agent, and a biologic response modifier (BRM).

Generally, a BRM is a substances made from living organisms to treat disease, which may occur naturally in the body or may be made in the laboratory. Examples of BRMs include IL-2, interferon, various types of colony-stimulating factors (CSF, GM-CSF, G-CSF), monoclonal antibodies such as abciximab, etanercept, infliximab, rituximab, trasturzumab, and high dose ascorbate.

In some embodiments, the anti-TNF-a agent is infliximab, and etanercept.

In some embodiments, the hypomethylating agent is a DNA methyltransferase inhibitor. In some embodiments, the DNA methyltransferase inhibitor is selected from 5 azacytidine and decitabine.

Generally, IMiDs are as immunomodulatory agents. In some embodiments, the IMiD is selected from thalidomide, lenalidomide, pomalidomide, CC-11006, and CC-10015.

In some embodiments, the method further comprises administering an additional therapeutic agent selected from anti-thymocyte globulin, recombinant human granulocyte colony-stimulating factor (G CSF), granulocyte-monocyte CSF (GM-CSF), a erythropoiesis-stimulating agent (ESA), and cyclosporine.

In some embodiments, the method further comprises administering an additional JAK inhibitor to the patient. In some embodiments, the additional JAK inhibitor is tofacitinib or ruxolitinib.

One or more additional therapeutic agents may include chemotherapeutics, anti-inflammatory agents, steroids, immunosuppressants, as well as Bcr-Abl, Flt-3, RAF and FAK kinase inhibitors such as, for example, those described in WO 2006/056399, or other agents can be used in combination with the compounds described herein.

Example chemotherapeutics include proteosome inhibitors (*e.g.,* bortezomib), thalidomide, revlimid, and DNA-damaging agents such as melphalan, doxorubicin, cyclophosphamide, vincristine, etoposide, carmustine, and the like.

Example steroids include coriticosteroids such as dexamethasone or prednisone.

Example Bcr-Abl inhibitors include the compounds, and pharmaceutically acceptable salts thereof, of the genera and species disclosed in U.S. Pat. No. 5,521,184, WO 04/005281, and U.S. Ser. No. 60/578,491. Example suitable Flt-3 inhibitors include compounds, and their pharmaceutically acceptable salts, as disclosed in WO 03/037347, WO 03/099771, and WO 04/046120. Example suitable RAF inhibitors include compounds, and their pharmaceutically acceptable salts, as disclosed in WO 00/09495 and WO 05/028444. Example suitable FAK inhibitors include compounds, and their pharmaceutically acceptable salts, as disclosed in WO 04/080980, WO 04/056786, WO 03/024967, WO 01/064655, WO 00/053595, and WO 01/014402. In some embodiments, one or more of the compounds of the invention can be used in combination with one or more other kinase inhibitors including imatinib, particularly for treating patients resistant to imatinib or other kinase inhibitors.

In some embodiments, a suitable chemotherapeutical agent can be selected from antimetabolite agents, topoisomerase 1 inhibitors, platinum analogs, taxanes, anthracyclines, and EGFR inhibitors, and combinations thereof.

In some embodiments, antimetabolite agents include capecitabine, gemcitabine, and fluorouracil (5-FU).

In some embodiments, taxanes include paclitaxel, Abraxane® (paclitaxel protein-bound particles for injectable suspension), and Taxotere® (docetaxel).

In some embodiments, platinum analogs include oxaliplatin, cisplatin, and carboplatin.

In some embodiments, topoisomerase 1 inhibitors include irinotecan and topotecan.

In some embodiment, anthracyclines include doxorubicin or liposomal formulations of doxorubicin.

In some embodiments, the chemotherapeutic is FOLFIRINOX (5-FU, lecovorin, irinotecan and oxaliplatin). In some embodiments, the chemotherapeutic agent is gemcitabine and Abraxane® (paclitaxel protein-bound particles for injectable suspension).

In some embodiments, the additional therapeutic agent is melphalan, melphalan plus prednisone [MP], doxorubicin, dexamethasone, and Velcade (bortezomib). Further additional agents used in the treatment of multiple myeloma include Bcr-Abl, Flt-3, RAF and FAK kinase inhibitors. The agents can be combined with the present compounds in a single or continuous dosage form, or the agents can be administered simultaneously or sequentially as separate dosage forms.

In some embodiments, a corticosteroid such as dexamethasone is administered to a patient in combination with at least one JAK1 selective inhibitor where the dexamethasone is administered intermittently as opposed to continuously.

In some further embodiments, combinations of one or more JAK1 selective inhibitors with other therapeutic agents can be administered to a patient prior to, during, and/or after a bone marrow transplant or stem cell transplant.

In some embodiments, the additional therapeutic agent is fluocinolone acetonide (Retisert®), or rimexolone (AL-2178, Vexol, Alcon).

In some embodiments, the additional therapeutic agent is cyclosporine (Restasis®).

In some embodiments, the additional therapeutic agent is a corticosteroid. In some embodiments, the corticosteroid is triamcinolone, dexamethasone, fluocinolone, cortisone, prednisolone, or flumetholone.

In some embodiments, the additional therapeutic agent is selected from Dehydrex™ (Holles Labs), Civamide (Opko), sodium hyaluronate (Vismed, Lantibio/TRB Chemedia), cyclosporine (ST-603, Sirion Therapeutics), ARG101(T) (testosterone, Argentis), AGR1012(P) (Argentis), ecabet sodium (Senju-Ista), gefarnate (Santen), 15-(s)-hydroxyeicosatetraenoic acid (15(S)-HETE), cevilemine, doxycycline (ALTY-0501, Alacrity), minocycline, iDestrin™ (NP50301, Nascent Pharmaceuticals), cyclosporine A (Nova22007, Novagali), oxytetracycline (Duramycin, MOLI1901, Lantibio), CF101 (2S,3S,4R,5R)-3,4-dihydroxy-5-[6-[(3-iodophenyl)methylamino]purin-9-yl]-N-methyl-oxolane-2-carbamyl, Can-Fite Biopharma), voclosporin (LX212 or LX214, Lux Biosciences), ARG103 (Agentis), RX-10045 (synthetic resolvin analog, Resolvyx), DYN15 (Dyanmis Therapeutics), rivoglitazone (DE011, Daiichi Sanko), TB4 (RegeneRx), OPH-01 (Ophtalmis Monaco), PCS101 (Pericor Science), REV1-31 (Evolutec), Lacritin (Senju), rebamipide (Otsuka-Novartis), OT-551 (Othera), PAI-2 (University of Pennsylvania and Temple University), pilocarpine, tacrolimus, pimecrolimus (AMS981, Novartis), loteprednol etabonate, rituximab, diquafosol tetrasodium (INS365, Inspire), KLS-0611 (Kissei Pharmaceuticals), dehydroepiandrosterone, anakinra, efalizumab, mycophenolate sodium, etanercept (Embrel®), hydroxychloroquine, NGX267 (TorreyPines Therapeutics), actemra, gemcitabine, oxaliplatin, L-asparaginase, or thalidomide.

In some embodiments, the additional therapeutic agent is an anti-angiogenic agent, cholinergic agonist, TRP-1 receptor modulator, a calcium channel blocker, a mucin secretagogue, MUC1 stimulant, a calcineurin inhibitor, a corticosteroid, a P2Y2 receptor agonist, a muscarinic receptor agonist, an mTOR inhibitor, another JAK inhibitor, Bcr-Abl kinase inhibitor, Flt-3 kinase inhibitor, RAF kinase inhibitor, and FAK kinase inhibitor such as, for example, those described in WO 2006/056399. In some embodiments, the additional therapeutic agent is a tetracycline derivative (e.g., minocycline or doxycline). In some embodiments, the additional therapeutic agent binds to FKBP12.

In some embodiments, the additional therapeutic agent is an alkylating agent or DNA cross-linking agent; an anti-metabolite/demethylating agent (e.g., 5-flurouracil, capecitabine or azacitidine); an anti-hormone therapy (e.g., hormone receptor antagonists, SERMs, or aromotase inhibitor); a mitotic inhibitor (e.g. vincristine or paclitaxel); an topoisomerase (I or II) inhibitor (e.g. mitoxantrone and irinotecan); an apoptotic inducers (e.g. ABT-737); a nucleic acid therapy (e.g. antisense or RNAi); nuclear receptor ligands (e.g., agonists and/or antagonists: all-trans retinoic acid or bexarotene); epigenetic targeting agents such as histone deacetylase inhibitors (e.g. vorinostat), hypomethylating agents (e.g. decitabine); regulators of protein stability such as Hsp90 inhibitors, ubiquitin and/or ubiquitin like conjugating or deconjugating molecules; or an EGFR inhibitor (erlotinib).

### Pharmaceutical Formulations and Dosage Forms

When employed as pharmaceuticals, the JAK1 selective inhibitors can be administered in the form of pharmaceutical compositions. These compositions can be prepared in a manner well known in the pharmaceutical art, and can be administered by a variety of routes, depending upon whether local or systemic treatment is desired and upon the area to be treated. Administration may be topical (including transdermal, epidermal, ophthalmic and to mucous membranes including intranasal, vaginal and rectal delivery), pulmonary (*e.g.,* by inhalation or insufflation of powders or aerosols, including by nebulizer; intratracheal or intranasal), oral or parenteral. Parenteral administration includes intravenous, intraarterial, subcutaneous, intraperitoneal intramuscular or injection or infusion; or intracranial, *e.g.,* intrathecal or intraventricular, administration. Parenteral administration can be in the form of a single bolus dose, or may be, for example, by a continuous perfusion pump. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

This invention also includes pharmaceutical compositions which contain, as the active ingredient, the JAK1 selective inhibitor described herein, or a pharmaceutically acceptable salt thereof, in combination with one or more pharmaceutically acceptable carriers (excipients). In some embodiments, the composition is suitable for topical administration. In making the compositions, the active ingredient is typically mixed with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, the active compound can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, e.g. about 40 mesh.

The JAK1 selective inhibitors may be milled using known milling procedures such as wet milling to obtain a particle size appropriate for tablet formation and for other formulation types. Finely divided (nanoparticulate) preparations of the JAK1 selective inhibitors can be prepared by processes known in the art, e.g., see International App. No. WO 2002/000196.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions can be formulated so as to provide quick, sustained or delayed release of the active ingredient after administration to the patient by employing procedures known in the art.

In some embodiments, the pharmaceutical composition comprises silicified microcrystalline cellulose (SMCC) and at least one compound described herein, or a pharmaceutically acceptable salt thereof. In some embodiments, the silicified microcrystalline cellulose comprises about 98% microcrystalline cellulose and about 2% silicon dioxide w/w.

In some embodiments, the composition is a sustained release composition comprising at least one JAK1 selective inhibitor described herein, or a pharmaceutically acceptable salt thereof, and at least one pharmaceutically acceptable carrier. In some embodiments, the composition comprises at least one JAK1 selective inhibitor described herein, or a pharmaceutically acceptable salt thereof, and at least one component selected from microcrystalline cellulose, lactose monohydrate, hydroxypropyl methylcellulose, and polyethylene oxide. In some embodiments, the composition comprises at least one JAK1 selective inhibitor described herein, or a pharmaceutically acceptable salt thereof, and microcrystalline cellulose, lactose monohydrate, and hydroxypropyl methylcellulose. In some embodiments, the composition comprises at least one JAK1 selective inhibitor described herein, or a pharmaceutically acceptable salt thereof, and microcrystalline cellulose, lactose monohydrate, and polyethylene oxide. In some embodiments, the composition further comprises magnesium stearate or silicon dioxide. In some embodiments, the microcrystalline cellulose is Avicel PH102™. In some embodiments, the lactose monohydrate is Fast-flo 316™. In some embodiments, the hydroxypropyl methylcellulose is hydroxypropyl methylcellulose 2208 K4M (e.g., Methocel K4 M Premier™) and/or hydroxypropyl methylcellulose 2208 K100LV (e.g., Methocel K00LV™). In some embodiments, the polyethylene oxide is polyethylene oxide WSR 1105 (e.g., Polyox WSR 1105™).

In some embodiments, a wet granulation process is used to produce the composition. In some embodiments, a dry granulation process is used to produce the composition.

The compositions can be formulated in a unit dosage form, each dosage containing from about 5 to about 1,000 mg (1 g), more usually about 100 mg to about 500 mg, of the active ingredient. In some embodiments, each dosage contains about 10 mg of the active ingredient. In some embodiments, each dosage contains about 50 mg of the active ingredient. In some embodiments, each dosage contains about 25 mg of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

In some embodiments, the compositions contain from about 2 mg to about 10 mg, or about 5 mg to about 50 mg of the active ingredient. One having ordinary skill in the art will appreciate that this embodies compounds or compositions containing about 2 mg to about 10 mg, 5 mg to about 10 mg, about 10 mg to about 15 mg, about 15 mg to about 20 mg, about 20 mg to about 25 mg, about 25 mg to about 30 mg, about 30 mg to about 35 mg, about 35 mg to about 40 mg, about 40 mg to about 45 mg, or about 45 mg to about 50 mg of the active ingredient.

In some embodiments, the compositions contain from about 50 mg to about 500 mg of the active ingredient. One having ordinary skill in the art will appreciate that this embodies compounds or compositions containing about 50 mg to about 100 mg, about 100 mg to about 150 mg, about 150 mg to about 200 mg, about 200 mg to about 250 mg, about 250 mg to about 300 mg, about 350 mg to about 400 mg, or about 450 mg to about 500 mg of the active ingredient.

In some embodiments, the compositions contain from about 500 mg to about 1,000 mg of the active ingredient. One having ordinary skill in the art will appreciate that this embodies compounds or compositions containing about 500 mg to about 550 mg, about 550 mg to about 600 mg, about 600 mg to about 650 mg, about 650 mg to about 700 mg, about 700 mg to about 750 mg, about 750 mg to about 800 mg, about 800 mg to about 850 mg, about 850 mg to about 900 mg, about 900 mg to about 950 mg, or about 950 mg to about 1,000 mg of the active ingredient.

The active compound may be effective over a wide dosage range and is generally administered in a pharmaceutically effective amount. It will be understood, however, that the amount of the compound actually administered will usually be determined by a physician, according to the relevant circumstances, including the condition to be treated, the chosen route of administration, the actual compound administered, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound described herein, or a pharmaceutically acceptable salt thereof. When referring to these preformulation compositions as homogeneous, the active ingredient is typically dispersed evenly throughout the composition so that the composition can be readily subdivided into equally effective unit dosage forms such as tablets, pills and capsules. This solid preformulation is then subdivided into unit dosage forms of the type described above containing from, for example, about 0.1 to about 1000 mg of the active ingredient of the present invention.

The tablets or pills can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the compounds and compositions can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* In some embodiments, the compositions are administered by the oral or nasal respiratory route for local or systemic effect. Compositions in can be nebulized by use of inert gases. Nebulized solutions may be breathed directly from the nebulizing device or the nebulizing device can be attached to a face masks tent, or intermittent positive pressure breathing machine. Solution, suspension, or powder compositions can be administered orally or nasally from devices which deliver the formulation in an appropriate manner.

Topical formulations can contain one or more conventional carriers. In some embodiments, ointments can contain water and one or more hydrophobic carriers selected from, for example, liquid paraffin, polyoxyethylene alkyl ether, propylene glycol, white Vaseline, and the like. Carrier compositions of creams can be based on water in combination with glycerol and one or more other components, e.g. glycerinemonostearate, PEG-glycerinemonostearate and cetylstearyl alcohol. Gels can be formulated using isopropyl alcohol and water, suitably in combination with other components such as, for example, glycerol, hydroxyethyl cellulose, and the like. In some embodiments, topical formulations contain at least about 0.1, at least about 0.25, at least about 0.5, at least about 1, at least about 2, or at least about 5 wt % of the compound described herein, or a pharmaceutically acceptable salt thereof. The topical formulations can be suitably packaged in tubes of, for example, 100 g which are optionally associated with instructions for the treatment of the select indication, e.g., psoriasis or other skin condition.

The amount of compound or composition administered to a patient will vary depending upon what is being administered, the purpose of the administration, such as prophylaxis or therapy, the state of the patient, the manner of administration, and the like. In therapeutic applications, compositions can be administered to a patient already suffering from a disease in an amount sufficient to cure or at least partially arrest the symptoms of the disease and its complications. Effective doses will depend on the disease condition being treated as well as by the judgment of the attending clinician depending upon factors such as the severity of the disease, the age, weight and general condition of the patient, and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers, or stabilizers will result in the formation of pharmaceutical salts.

The therapeutic dosage of a JAK1 selective inhibitor described herein, or a pharmaceutically acceptable salt thereof, can vary according to, for example, the particular use for which the treatment is made, the manner of administration of the compound, the health and condition of the patient, and the judgment of the prescribing physician. The proportion or concentration of a compound described herein, or a pharmaceutically acceptable salt thereof, in a pharmaceutical composition can vary depending upon a number of factors including dosage, chemical characteristics (*e.g.,* hydrophobicity), and the route of administration. For example, the JAK1 selective inhibitor can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from *in vitro* or animal model test systems.

The compositions of the invention can further include one or more additional pharmaceutical agents such as a chemotherapeutic, steroid, anti-inflammatory compound, or immunosuppressant, examples of which are listed hereinabove.

### Kits

The present invention also includes pharmaceutical kits useful, for example, in the treatment or prevention of a myelodysplastic syndrome, which include one or more containers containing a pharmaceutical composition comprising a therapeutically effective amount of a compound described herein, or a pharmaceutically acceptable salt thereof. Such kits can further include, if desired, one or more of various conventional pharmaceutical kit components, such as, for example, containers with one or more pharmaceutically acceptable carriers, additional containers, etc., as will be readily apparent to those skilled in the art. Instructions, either as inserts or as labels, indicating quantities of the components to be administered, guidelines for administration, and/or guidelines for mixing the components, can also be included in the kit.

### EXAMPLES

The invention will be described in greater detail by way of specific examples. The following examples are offered for illustrative purposes. Those of skill in the art will readily recognize a variety of non-critical parameters which can be changed or modified to yield essentially the same results. The compounds of the Examples have been found to be JAK inhibitors according to at least one assay described herein.

### Example 1. 3-[1-(6-chloropyridin-2-yl)pyrrolidin-3-yl]-3-[4-(7H-pyrrolo[2,3-d]pyrimidin-4-yl)-1H-pyrazol-1-yl]propanenitrile (two different enantiomers isolated)

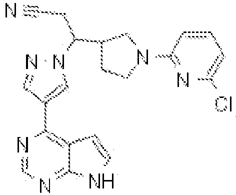

### Step 1. benzyl 3-{2-cyano-1-[4-(1-{[2-(trimethylsilyl)ethoxy]methyl}-7H-pyrrolo[2,3-d]pyrimidin-4-yl)-1H-pyrazol-1-yl]ethyl}pyrrolidine-1-carboxylate

Benzyl 3-[2-cyanovinyl]pyrrolidine-1-carboxylate (4.3 g, 0.017 mol, mixture of E and Z isomers prepared as described in WO 2007/070514 Ex.742) was dissolved in acetonitrile (270 mL). 1,8-Diazabicyclo[5.4.0]undec-7-ene (5.02 mL, 0.0336 mol) was added, followed by 4-(1H-pyrazol-4-yl)-7-{[2-(trimethylsilyl)ethoxy]methyl}-7H-pyrrolo[2,3-d]pyrimidine (5.6 g, 0.017 mol, prepared as described in WO 2007/070514, Ex.65). The mixture was stirred at RT overnight. The solvent was removed by rotary evaporation, and the residue was redissolved in ethyl acetate. The solution was washed successively with IN HCl, water, saturated sodium bicarbonate, and brine, dried over sodium sulfate and concentrated in vacuo. The product was purified by flash column chromatography on silica gel, eluting with a gradient of 0-100% ethyl acetate in hexanes to afford diastereomer 1 (first to elute) (3.5g, 36%) and diastereomer 2 (second to elute) (2.5g, 25%). LCMS (M+H)⁺: 572.2.

### Step 2. 3-pyrrolidin-3-yl-3-[4-(7-{[2-(trimethylsilyl)ethoxy]methyl}-7H-pyrrolo[2,3-d]pyrimidin-4-yl)-1H-pyrazol-1-yl]propanenitrile

Benzyl 3-{2-cyano-1-[4-(7-{[2-(trimethylsilyl)ethoxy]methyl}-7H-pyrrolo[2,3-d]pyrimidin-4-yl)-1H-pyrazol-1-yl]ethyl}pyrrolidine-1-carboxylate (3.5 g, 6.1 mmol) (diastereomer 1 from Example 1, Step 1) was dissolved in 100 mL methanol, and a catalytic amount of 10% Pd-C was added. The mixture was shaken under 50 psi of hydrogen for 24 h. The mixture was then filtered and the solvent removed *in vacuo.* The product was used without further purification. LCMS (M+H)⁺: 438.2.

### Step 3. 3-[1-(6-chloropyridin-2-yl)pyrrolidin-3-yl]-3-[4-(1-{[2-(trimethylsilyl)ethoxy]methyl}-7H-pyrrolo[2,3-d]pyrimidin-4-yl)-1H-pyrazol-1-yl]propanenitrile

A mixture of 3-pyrrolidin-3-yl-3-[4-(7-{[2-(trimethylsilyl)ethoxy]methyl}-7H-pyrrolo[2,3-d]pyrimidin-4-yl)-1H-pyrazol-1-yl]propanenitrile (150 mg, 0.27 mmol) and 2,6-dichloropyridine (48.7 mg, 0.329 mmol) in NMP (1.6 mL) and N,N-diisopropylethylamine (96 microL, 0.55 mmol) was heated to 135°C for 20 min in the microwave. Purification by flash column chromatography on silica gel, eluting with a gradient from 0-80% ethyl acetate in hexanes, afforded the title product (28 mg, 18%). ¹H NMR (400 MHz, CDCl₃): δ 8.85 (s, 1H), 8.36 (s, 1H), 8.36 (s, 1H), 7.41 (d, 1H), 7.37 (dd, 1H), 6.79 (d, 1H), 6.57 (d, 1H), 6.22 (d, 1H), 5.68 (s, 2H), 4.45 (dt, 1H), 3.91 (dd, 1H), 3.57-3.46 (m, 3H), 3.39-3.29 (m, 2H), 3.24 (dd, 1H), 3.13-3.01 (m, 1H), 3.01 (dd, 1H), 1.98-1.88 (m, 1H), 1.82-1.69 (m, 1H), 0.95-0.88 (m, 2H), - 0.06 (s, 9H); LCMS (M+H)⁺: 549.1.

This racemic product was separated into its enantiomers by chiral HPLC (Chiral Technologies Chiralcel OJ-H, 5µ, 30 x 250mm, 45% EtOH/Hexanes, 20 mL/min) to afford enantiomer 1 (first to elute, retention time 40.7 min) and enantiomer 2 (second to elute, retention time 51.6 min), which were deprotected separately in Steps 4a/4b.

### Step 4a. 3-[1-(6-chloropyridin-2-yl)pyrrolidin-3-yl]-3-[4-(7H-pyrrolo[2,3-d]pyrimidin-4-yl)-1H-pyrazol-1-yl]propanenitrile (enantiomer 1)

3-[1-(6-Chloropyridin-2-yl)pyrrolidin-3-yl]-3-[4-(7-{[2-(trimethylsilyl)ethoxy]methyl}-7H-pyrrolo[2,3-d]pyrimidin-4-yl)-1H-pyrazol-1-yl]propanenitrile (enantiomer 1 from Step 3) was stirred in a solution containing 1:1 TFA/DCM (2 mL) for 2 h, and then concentrated. The residue was dissolved in 1 mL MeOH, and 0.2 mL EDA was added. Purification via preparative-HPLC/MS (C18 column eluting with a gradient of ACN/H₂O containing 0.15% NH₄OH) afforded product. ¹H NMR (400 MHz, CDCl₃): δ 9.44 (br s, 1H), 8.84 (s, 1H), 8.37 (s, 2H), 7.39 (dd, 1H), 7.38 (dd, 1H), 6.79 (dd, 1H), 6.58 (d, 1H), 6.22 (d, 1H), 4.46 (dt, 1H), 3.92 (dd, 1H), 3.55-3.48 (m, 1H), 3.39-3.31 (m, 2H), 3.25 (dd, 1H), 3.13-3.02 (m, 1H), 3.02 (dd, 1H), 2.00-1.88 (m, 1H), 1.84-1.71 (m, 1H); LCMS (M+H)⁺: 419.1.

### Step 4b. 3-[1-(6-chloropyridin-2-yl)pyrrolidin-3-yl]-3-[4-(7H-pyrrolo[2,3-d]pyrimidin-4-yl)-1H-pyrazol-1-yl]propanenitrile(enantiomer 2)

Performed as in Step 4a, using enantiomer 2 from Step 3: ¹H NMR (400 MHz, CDCl₃): δ 9.59 (br s, 1H), 8.84 (s, 1H), 8.37 (s, 2H), 7.40 (dd, 1H), 7.38 (dd, 1H), 6.79 (dd, 1H), 6.58 (d, 1H), 6.22 (d, 1H), 4.46 (dt, 1H), 3.92 (dd, 1H), 3.55-3.48 (m, 1H), 3.39-3.31 (m, 2H), 3.25 (dd, 1H), 3.14-3.02 (m, 1H), 3.02 (dd, 1H), 1.99-1.90 (m, 1H), 1.83-1.72 (m, 1H); LCMS (M+H)⁺: 419.1.

### Example 2. 3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile (one enantiomer isolated)

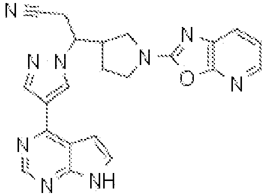

### Step 1. 3-(1-[1,3]oxazolo[5,4-b]pyridin-2-ylpyrrolidin-3-yl)-3-[4-(7-{[2-(trimethylsilyl)ethoxy]methyl}-7H-pyrrolo[2,3-d]pyrimidin-4-yl)-1H-pyrazol-1-yl]propanenitrile

Oxazolo[5,4-b]pyridine-2(1H)-thione (1.17 g, 7.68 mmol, prepared as in Example 33 of US 2010/0298334, Step 4) and 3-pyrrolidin-3-yl-3-[4-(7-{[2-(trimethylsilyl)ethoxy]methyl}-7H-pyrrolo[2,3-d]pyrimidin-4-yl)-1H-pyrazol-1-yl]propanenitrile (2.80 g, 6.40 mmol from Example 15, Step 3) in 1,4-dioxane (30 mL) was heated to 70 °C for 2 h. The solvent was removed in vacuo. The crude product was reconstituted in ethanol (40 mL) and treated with silver nitrate (3 g, 15 mmol) and aqueous ammonium hydroxide (6 mL) portionwise over the course of 20 h. Into the reaction was added water, IN NaOH and brine. Insoluble material was removed by filtration. The layers of the filtrate were separated. The aqueous portion was extracted with three portions of ethyl acetate. The extracts were dried over sodium sulfate, decanted and concentrated. The crude product was purified by flash column chromatography on silica gel, eluting with 10% MeOH/DCM to afford the product as an off-white foam (2.84 g, 80%). ¹H NMR (300 MHz, CDCl₃): δ 8.83 (s, 1H), 8.37 (s, 1H), 8.36 (s, 1H), 7.92 (dd, 1H), 7.57 (dd, 1H), 7.40 (d, 1H), 7.13 (dd, 1H), 6.78 (d, 1H), 5.67 (s, 2H), 4.52 (dt, 1H), 4.05 (dd, 1H), 3.82 (ddd, 1H), 3.67-3.44 (m, 4H), 3.25 (dd, 1H), 3.24-3.09 (m, 1H), 2.98 (dd, 1H), 2.06-1.74 (m, 2H), 0.97-0.88 (m, 2H), -0.06 (s, 9H); LCMS (M+H)⁺: 556.1.

### Step 2. 3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile

3-(1-[1,3]Oxazolo[5,4-b]pyridin-2-ylpyrrolidin-3-yl)-3-[4-(7-{[2-(trimethylsilyl)ethoxy]methyl}-7H-pyrrolo[2,3-d]pyrimidin-4-yl)-1H-pyrazol-1-yl]propanenitrile (5.35 g, 9.63 mmol, prepared by the method of Step 1) was stirred in a 2:1 mixture of DCM and TFA (60 mL) for 6 h. The solvents were removed by rotary evaporation. The crude residue was dissolved in methanol (50 mL) containing EDA (5.15 mL, 77.0 mmol) and was stirred overnight. After removal of solvent, the product was purified by flash column chromatography on silica gel, eluting with a gradient from 0-15% MeOH/DCM (3.59 g, 88%). ¹H NMR (300 MHz, CDCl₃): δ 8.72 (s, 1H), 8.40 (s, 1H), 8.34 (s, 1H), 7.89 (dd, 1H), 7.54 (dd, 1H), 7.36 (d, 1H), 7.12 (dd, 1H), 6.75 (d, 1H), 4.56 (dt, 1H), 4.01 (dd, 1H), 3.80 (ddd, 1H), 3.60 (ddd, 1H), 3.48 (dd, 1H), 3.26 (dd, 1H), 3.21-3.06 (m, 1H), 3.02 (dd, 1H), 2.03-1.76 (m, 2H); LCMS (M+H)⁺: 426.1.

### Example 3. 4-[(4-{3-cyano-2-[4-(7H-pyrrolo[2,3-d]pyrimidin-4-yl)-1H-pyrazol-1-yl]propyl}piperazin-1-yl)carbonyl]-3-fluorobenzonitrile trifluoroacetate salt (single enantiomer isolated)

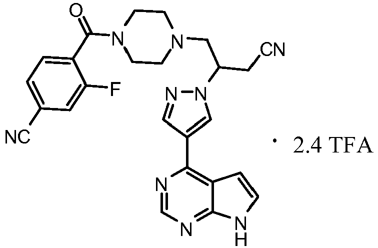

### Step 1. (R)- and (S)- tert-butyl 4-{3-cyano-2-[4-(7-{[2-(trimethylsilyl)ethoxy]methyl}-7H-pyrrolo[2,3-d]pyrimidin-4-yl)-1H-pyrazol-1-yl]propyl}piperazine-1-carboxylate

1,8-Diazabicyclo[5.4.0]undec-7-ene (5.5 mL, 0.037 mol) was added to a solution of (E)- and (Z)-tert-butyl 4-(3-cyanoallyl)piperazine-1-carboxylate (11.1 g, 0.0441 mol, prepared as in Example 1 of US 2011/0059951, Steps 1-2) and 4-(1H-pyrazol-4-yl)-7-{[2-(trimethylsilyl)ethoxy]methyl}-7H-pyrrolo[2,3-d]pyrimidine (11.6 g, 0.0368 mol, prepared as described in WO2007/070514, Example 65) in acetonitrile (70 mL). The mixture was stirred at 50 °C for 15 hours. Solvents were removed *in vacuo.* The residue was dissolved in ethyl acetate, washed with water (3 times), brine (once), dried over sodium sulfate and concentrated. Flash column chromatography, followed by preparative HPLC-MS (eluting with a gradient of MeCN/H₂O containing 0.15% NH₄OH) afforded product as a white foam (8.20 g, 39%).

Chiral HPLC was used to separate the racemic mixture into single enantiomers (Phenomenex Lux-Cellulose-2, 21.2 x 250 mm, 5 µm, eluting with 30%EtOH/70%Hexanes, at 20mL/min). Peak 1 (first to elute): 4.0 g and peak 2 (second to elute): 4.0 g. ¹H NMR Peak 1 (400 MHz, CDCl₃): δ 8.84 (s, 1H), 8.33 (s, 1H), 8.31 (s, 1H), 7.40 (d, 1H), 6.79 (d, 1H), 5.68 (s, 2H), 4.70-4.62 (m, 1H), 3.58-3.51 (m, 2H), 3.44-3.35 (br m, 4H), 3.16 (dd, 1H), 3.10 (dd, 1H), 2.99 (dd, 1H), 2.89 (dd, 1H), 2.50-2.40 (br m, 4H), 1.44 (s, 9H), 0.95-0.89 (m, 2H), -0.06 (s, 9H); LCMS (M+H)⁺: 567.3. ¹H NMR Peak 2 (400 MHz, CDCl₃): δ 8.84 (s, 1H), 8.32 (s, 1H), 8.31 (s, 1H), 7.40 (d, 1H), 6.79 (d, 1H), 5.68 (s, 2H), 4.70-4.62 (m, 1H), 3.58-3.51 (m, 2H), 3.45-3.34 (br m, 4H), 3.16 (dd, 1H), 3.10 (dd, 1H), 2.99 (dd, 1H), 2.90 (dd, 1H), 2.50-2.40 (br m, 4H), 1.44 (s, 9H), 0.95-0.89 (m, 2H), -0.06 (s, 9H); LCMS (M+H)⁺: 567.3.

### Step 2. 4-piperazin-1-yl-3-[4-(7-{[2-(trimethylsilyl)ethoxy]methyl}-7H-pyrrolo[2,3-d]pyrimidin-4-yl)-1H-pyrazol-1-yl]butanenitrile hydrochloride salt

tert-Butyl 4-{3-cyano-2-[4-(7-{[2-(trimethylsilyl)ethoxy]methyl}-7H-pyrrolo[2,3-d]pyrimidin-4-yl)-1H-pyrazol-1-yl]propyl}piperazine-1-carboxylate (4.0 g, 7.0 mmol; Peak 2 from Step 1) was dissolved in 1,4-dioxane (40 mL), and 4.0 M of HCl in dioxane (25 mL, 100 mmol) was added. The mixture was stirred at room temperature for 80 min. Solvent was removed *in vacuo* to afford the product as the hydrochloride salt. LCMS (M+H)⁺: 467.3.

### Step 3. 4-[(4-{3-cyano-2-[4-(7H-pyrrolo[2,3-d]pyrimidin-4-yl)-1H-pyrazol-1-yl]propyl}piperazin-1-yl)carbonyl]-3-fluorobenzonitrile trifluoroacetate salt

A mixture of 4-cyano-2-fluorobenzoic acid (138 mg, 0.836 mmol, Alfa Aesar), N,N,N',N'-tetramethyl-O-(7-azabenzotriazol-1-yl)uronium hexafluorophosphate (254 mg, 0.669 mmol) and triethylamine (0.466 mL, 3.34 mmol) in THF (10.0 mL) was stirred at room temperature for 15 minutes. 4-Piperazin-1-yl-3-[4-(7-{[2-(trimethylsilyl)ethoxy]methyl}-7H-pyrrolo[2,3-d]pyrimidin-4-yl)-1H-pyrazol-1-yl]butanenitrile hydrochloride (0.33 g, 0.56 mmol; from Step 2) was added. The reaction was stirred at room temperature for one hour. The reaction was diluted with ethyl acetate and water. The layers were separated and the organic layer was washed successively with water, 0.1N NaOH and brine, dried over sodium sulfate and concentrated. The residue was dissolved in a 2:1 mixture of DCM:TFA, stirred for 3 hours, concentrated, then in a mixture of 8 mL methanol to which 0.8 mL of ethylenediamine was added. After stirring for one hour, the product was purified via HPLC-MS, eluting with a gradient of MeCN and H₂O containing 0.2% TFA. Eluent frozen and lyophilized to afford a white powder (200 mg, 47%). ¹H NMR (400 MHz, d₆-dmso): δ 12.64 (br s, 1H), 8.97 (s, 1H), 8.83 (s, 1H), 8.51 (s, 1H), 7.99 (dd, 1H), 7.82-7.76 (m, 2H), 7.61 (t, 1H), 7.15-7.11 (m, 1H), 5.13 (br m, 1H), 3.82-2.37 (br, 12H); ¹⁹F NMR (400 MHz, d₆-dmso): δ -74.97 (s, 7.2 F), -114.49 (br s, 1F); LCMS (M+H)⁺: 484.2.

### Example 4. 4-[(4-{3-cyano-2-[3-(7H-pyrrolo[2,3-d]pyrimidin-4-yl)-1H-pyrrol-1-yl]propyl}piperazin-1-yl)carbonyl]-3-fluorobenzonitrile trifluoroacetate salt (single enantiomer isolated)

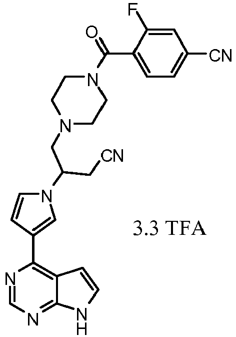

### Step 1. tert-butyl 4-{3-cyano-2-[3-(7-{[2-(trimethylsilyl)ethoxy]methyl}-7H-pyrrolo[2,3-d]pyrimidin-4-yl)-1H-pyrrol-1-yl]propyl}piperazine-1-carboxylate

To a mixture of (E)- and (Z)-tert-butyl 4-(3-cyanoallyl)piperazine-1-carboxylate (4.0 g, 0.016 mol; prepared as in Example 1 of US 2011/0059951, Steps 1-2) and 4-(1H-pyrrol-3-yl)-7-{[2-(trimethylsilyl)ethoxy]methyl}-7H-pyrrolo[2,3-d]pyrimidine (4.2 g, 0.013 mol, prepared as in WO2009/114512, Example 82) in N,N-Dimethylformamide (25 mL) was added potassium carbonate (5.540 g, 0.0401 mol). The mixture was stirred at 60 °C for 17 hours. Additional (E)- and (Z)-tert-butyl 4-(3-cyanoallyl)piperazine-1-carboxylate (4.0 g, 0.016 mol) was added and the reaction was stirred at 60 °C for 24 hours. A further portion of (E)- and (Z)-tert-butyl 4-(3-cyanoallyl)piperazine-1-carboxylate (4.0 g, 0.016 mol) was added. After 3 nights of heating, most of the starting material had been converted to desired product as determined by LCMS. The mixture was then filtered, diluted with EtOAc, washed with water (3 times), brine (once), dried over sodium sulfate, decanted and concentrated. Purification via preparative HPLC-MS (eluting with a gradient of MeCN/H₂O containing 0.15% NH₄OH) afforded a brown foam (4.20 g, 55%). ¹H NMR (300 MHz, CDCl₃): δ 8.81 (s, 1H), 7.66 (t, 1H), 7.34 (d, 1H), 6.97 (dd, 1H), 6.89 (t, 1H), 6.84 (d, 1H), 5.66 (s, 2H), 4.47-4.36 (m, 1H), 3.57-3.50 (m, 2H), 3.45-3.37 (m, 4H), 3.06 (dd, 1H), 3.00-2.90 (m, 2H), 2.83 (dd, 1H), 2.57-2.35 (m, 4H), 1.45 (s, 9H), 0.96-0.86 (m, 2H), -0.06 (s, 9H); LCMS (M+H)⁺: 566.3.

Chiral HPLC was used to separate the racemate into single enantiomers (Chiral Technologies ChiralPAK IA 20x250mm, 5µm, mobile phase 30%EtOH/70%hexanes, flow rate 12mL/min). Peak 1 (first enantiomer to elute), 1.8 g; Peak 2 (second enantiomer to elute): 1.9 g.

### Step 2. 4-piperazin-1-yl-3-[3-(7-{[2-(trimethylsilyl)ethoxy]methyl}-7H-pyrrolo[2,3-d]pyrimidin-4-yl)-1H-pyrrol-1-yl]butanenitrile hydrochloride salt

To a solution of tert-butyl 4-{3-cyano-2-[3-(7-{[2-(trimethylsilyl)ethoxy]methyl}-7H-pyrrolo[2,3-d]pyrimidin-4-yl)-1H-pyrrol-1-yl]propyl}piperazine-1-carboxylate (1.9 g, 0.0034 mol; peak 2 of Step 1) in 1,4-dioxane (20 mL) was added 4.0 M of HCl in p-dioxane (12 mL, 48 mmol). The mixture was stirred at room temperature for 80 minutes. Solvent was removed *in vacuo,* to afford product as a light yellow solid (1.90 g, 100%). LCMS (M+H)⁺: 466.3.

### Step 3. 4-[(4-{3-cyano-2-[3-(7H-pyrrolo[2,3-d]pyrimidin-4-yl)-1H-pyrrol-1-yl]propyl}piperazin-1-yl)carbonyl]-3-fluorobenzonitrile trifluoroacetate salt

A mixture of 4-cyano-2-fluorobenzoic acid (44 mg, 0.26 mmol, Alfa Aesar), N,N,N',N'-Tetramethyl-O-(7-azabenzotriazol-1-yl)uronium Hexafluorophosphate (93 mg, 0.24 mmol) and triethylamine (171 uL, 1.22 mmol) in THF (2.4 mL) was stirred at room temperature for 15 minutes. 4-Piperazin-1-yl-3-[3-(7-{[2-(trimethylsilyl)ethoxy]methyl}-7H-pyrrolo[2,3-d]pyrimidin-4-yl)-1H-pyrrol-1-yl]butanenitrile hydrochloride salt (110 mg, 0.20 mmol; from Step 2) was added. The reaction was stirred for 2 hours. Ethyl acetate and water were added. The layers were separated and the organic layer was washed successively with water, IN NaOH and brine, dried over sodium sulfate and concentrated. The residue was dissolved first in a 1:1 mixture of DCM:TFA for 1 hour, was concentrated, then was stirred in methanol (2 mL) containing ethylenediamine (0.2 mL) for one hour. Purification via preparative HPLC-MS (eluting with a gradient of MeCN/H₂O containing 0.1% TFA afforded product as the 3.3xTFA salt (84 mg, 48%). ¹H NMR (300 MHz, d₆-dmso): δ 13.22 (br s, 1H), 8.90 (s, 1H), 8.38 (s, 1H), 8.00 (dd, 1H), 7.97-7.93 (m, 1H), 7.80 (dd, 1H), 7.61 (t, 1H), 7.35 (s, 2H), 7.18-7.13 (m, 1H), 5.00-4.80 (m, 1H), 3.75-3.49 (br m, 2H), 3.35-2.33 (m, 10H); ¹⁹F NMR (300 MHz, d₆-dmso): δ -74.82 (s, 10F), -114.53 (s, IF); LCMS (M+H)⁺: 483.2.

### Example 5. {1-{1-[3-Fluoro-2-(trifluoromethyl)isonicotinoyl]piperidin-4-yl}-3-[4-(7H-pyrrolo[2,3-d]pyrimidin-4-yl)-1H-pyrazol-1-yl]azetidin-3-yl}acetonitrile

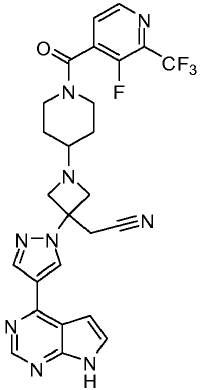

### Step A: tert-Butyl 3-Oxoazetidine-1-carboxylate

To a mixture of *tert*-butyl 3-hydroxyazetidine-1-carboxylate (10.0 g, 57.7 mmol), dimethyl sulfoxide (24.0 mL, 338 mmol), triethylamine (40 mL, 300 mmol) and methylene chloride (2.0 mL) was added sulfur trioxide-pyridine complex (40 g, 200 mmol) portionwise at 0 °C. The mixture was stirred for 3 hours, quenched with brine, and extracted with methylene chloride. The combined extracts were dried over anhydrous Na₂SO₄, filtered, and concentrated under reduced pressure. The residue was purified by silica gel column (0-6% ethyl acetate (EtOAc) in hexanes) to give *tert*-butyl 3-oxoazetidine-1-carboxylate (5.1 g, 52% yield).

### Step B: tert-Butyl 3-(Cyanomethylene)azetidine-1-carboxylate

An oven-dried 1 L 4-neck round bottom flask fitted with stir bar, septa, nitrogen inlet, 250 ml addition funnel and thermocouple was charged with sodium hydride (5.6 g, 0.14 mol) and tetrahydrofuran (THF) (140 mL) under a nitrogen atmosphere. The mixture was chilled to 3 °C, and then charged with diethyl cyanomethylphosphonate (22.4 mL, 0.138 mol) dropwise via a syringe over 20 minutes. The solution became a light yellow slurry. The reaction was then stirred for 75 minutes while warming to 18.2 °C. A solution of *tert*-butyl 3-oxoazetidine-1-carboxylate (20 g, 0.1 mol) in tetrahydrofuran (280 mL) was prepared in an oven-dried round bottom, charged to the addition funnel via canula, then added to the reaction mixture dropwise over 25 minutes. The reaction solution became red in color. The reaction was allowed to stir overnight. The reaction was checked after 24 hours by TLC (70% hexane/EtOAc) and found to be complete. The reaction was diluted with 200 mL of 20% brine and 250 mL of EtOAc. The solution was partitioned and the aqueous phase was extracted with 250 mL of EtOAc.The combined organic phase was dried over MgSO₄ and filtered, evaporated under reduced pressure, and purified by flash chromatography (0% to 20% EtOAc/hexanes, 150 g flash column) to give the desired product, *tert*-butyl 3-(cyanomethylene)azetidine-1-carboxylate (15 g, 66.1% yield).

### Step C: 4-Chloro-7-{[2-(trimethylsilyl)ethoxy]methyl}-7H-pyrrolo[2,3-d]pyrimidine

To a suspension of sodium hydride (36.141 g, 903.62 mmol) in N,N-dimethylacetamide (118 mL) at -5 °C (ice/salt bath) was added a dark solution of 4-chloropyrrolo[2,3-d]pyrimidine (119.37 g, 777.30 mmol) in N,N-dimethylacetamide (237 mL) slowly. The flask and addition funnel were rinsed with N,N-dimethylacetamide (30 mL). A large amount of gas was evolved immediately. The mixture became a slightly cloudy orange mixture. The mixture was stirred at 0 °C for 60 min to give a light brown turbid mixture. To the mixture was slowly added [2-(trimethylsilyl)ethoxy]methyl chloride (152.40 g, 914.11 mmol) and the reaction was stirred at 0 °C for 1 h. The reaction was quenched by addition of 12 mL of H₂O slowly. More water (120 mL) was added followed by methyl *tert*-butyl ether (MTBE) (120 mL). The mixture was stirred for 10 min. The organic layer was separated. The aqueous layer was extracted with another portion of MTBE (120 mL). The organic extracts were combined, washed with brine (120 mL × 2) and concentrated under reduced pressure to give the crude product 4-chloro-7-{[2-(trimethylsilyl)ethoxy]methyl}-7H-pyrrolo[2,3-d]pyrimidine as a dark oil. Yield: 85.07 g (97%); LC-MS: 284.1 (M+H)⁺. It was carried to the next reaction without purification.

### Step D: 4-(1H-Pyrazol-4-yl)-7-{[2-(trimethylsilyl)ethoxy]methyl}-7H-pyrrolo[2,3-d]pyrimidine

A 1000 mL round bottom flask was charged with 4-chloro-7-{[2-(trimethylsilyl)ethoxy]methyl}-7H-pyrrolo[2,3-d]pyrimidine (10.00 g, 35.23 mmol), 1-butanol (25.0 mL), 1-(1-ethoxyethyl)-4-(4,4,5,5-tetramethyl-1,3,2-dioxaborolan-2-yl)-1H-pyrazole (15.66 g, 52.85 mmol), water (25.0 mL) and potassium carbonate (12.17 g, 88.08 mmol). This solution was degased 4 times, filling with nitrogen each time. To the solution was added tetrakis(triphenylphosphine)palladium(0) (4.071 g, 3.523 mmol). The solution was degased 4 times, filling with nitrogen each time. The mixture was stirred overnight at 100 °C. After being cooled to room temperature, the mixture was filtered through a bed of celite and the celite was rinsed with ethyl acetate (42 mL). The filtrate was combined, and the organic layer was separated. The aqueous layer was extracted with ethyl acetate. The organic extracts were combined and concentrated under vacuum with a bath temerature of 30-70 °C to give the final compound 4-(1H-pyrazol-4-yl)-7-{[2-(trimethylsilyl)ethoxy]methyl}-7H-pyrrolo[2,3-d]pyrimidine. Yield: 78%. LC-MS: 316.2 (M+H)⁺.

### Step E: tert-Butyl 3-(Cyanomethyl)-3-[4-(7-{[2-(trimethylsilyl)ethoxy]methyl}-7H-pyrrolo[2,3-d]pyrimidin-4-yl)-1H-pyrazol-1-yl]azetidine-1-carboxylate

A 2 L round bottom flask fitted with overhead stirring, septa and nitrogen inlet was charged with *tert*-butyl 3-(cyanomethylene)azetidine-1-carboxylate (9.17 g, 0.0472 mol), 4-(1H-pyrazol-4-yl)-7-{[2-(trimethylsilyl)ethoxy]methyl}-7H-pyrrolo[2,3-d]pyrimidine (14.9 g, 0.0472 mol) and acetonitrile (300 mL). The resulting solution was heterogeneous. To the solution was added 1,8-diazabicyclo[5.4.0]undec-7-ene (8.48 mL, 0.0567 mol) portionwise via syringe over 3 min at room temperature. The solution slowly became homogeneous and yellow in color. The reaction was allowed to stir at room temperature for 3 h. The reaction was complete by HPLC and LC/MS and was concentrated by rotary evaporation to remove acetonitrile (∼150 mL). EtOAc (100 mL) was added followed by 100 ml of 20% brine. The two phases were partitioned. The aqueous phase was extracted with 150 mL of EtOAC. The combine organic phases were dried over MgSO₄, filtered and concentrated to yield an orange oil. Purification by flash chromatography (150 grams silica, 60% EtOAc/hexanes, loaded with CH₂Cl₂) yielded the title compound *tert-*butyl 3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidine-1-carboxylate as a yellow oil (21.1 g, 88% yield). LC-MS: [M+H]⁺ = 510.3.

### Step F: {3-[4-(7-{[2-(Trimethylsilyl)ethoxy]methyl}-7H-pyrrolo[2,3-d]pyrimidin-4-yl)-1H-pyrazol-1-yl]azetidin-3-yl}acetonitrile dihydrochloride

To a solution of *tert*-butyl 3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidine-1-carboxylate (2 g, 3.9 mmol) in 10 mL of THF was added 10 mL of 4 N HCl in dioxane. The solution was stirred at room temperature for 1 hour and concentrated *in vacuo* to provide 1.9 g (99%) of the title compound as a white powder solid, which was used for the next reaction without purification. LC-MS: [M+H]⁺ = 410.3.

### Step G: tert-Butyl 4-{3-(Cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}piperidine-1-carboxylate

Into the solution of {3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile dihydrochloride (2.6 g, 6.3 mmol), *tert*-butyl 4-oxo-1-piperidinecarboxylate (1.3 g, 6.3 mmol) in THF (30 mL) were added N,N-diisopropylethylamine (4.4 mL, 25 mmol) and sodium triacetoxyborohydride (2.2 g, 10 mmol). The mixture was stirred at room temperature overnight. After adding 20 mL of brine, the solution was extracted with EtOAc. The extract was dried over anhydrous Na₂SO₄ and concentrated. The residue was purified by combiflash column eluting with 30-80 % EtOAc in hexanes to give the desired product, *tert*-butyl 4-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}piperidine-1-carboxylate. Yield: 3.2 g (86%); LC-MS: [M+H]⁺= 593.3.

### Step H: {1-Piperidin-4-yl-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile trihydrochloride

To a solution of *tert-butyl* 4-{3-(cyanomethyl)-3-[4-(7-{[2-(trimethylsilyl) ethoxy]methyl}-7H-pyrrolo[2,3-d]pyrimidin-4-yl)-1H-pyrazol-1-yl]azetidin-1-yl}piperidine-1-carboxylate (3.2 g, 5.4 mmol) in 10 mL of THF was added 10 mL of 4 N HCl in dioxane. The reaction mixture was stirred at room temperature for 2 hours. Removing solvents under reduced pressure yielded 3.25 g (100%) of {1-piperidin-4-yl-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile trihydrochloride as a white powder solid, which was used directly in the next reaction. LC-MS: [M+H]⁺ = 493.3. ¹H NMR (400 MHz, DMSO-d₆): δ 9.42 (s 1H), 9.21 (s, 1H), 8.89 (s, 1H), 8.69 (s, 1H), 7.97 (s, 1H), 7.39 (d, 1H), 5.68 (s, 2H), 4.96 (d, 2H), 4.56 (m, 2H), 4.02-3.63 (m, 2H), 3.55 (s, 2H), 3.53 (t, 2H), 3.49-3.31 (3, 3H), 2.81 (m, 2H), 2.12 (d, 2H), 1.79 (m, 2H), 0.83 (t, 2H), -0.10 (s, 9H).

### Step I: {1-{1-[3-Fluoro-2-(trifluoromethyl)isonicotinoyl]piperidin-4-yl}-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile

A mixture of {1-piperidin-4-yl-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile trihydrochloride (1.22 g, 2.03 mmol), 3-fluoro-2-(trifluoromethyl)isonicotinic acid (460 mg, 2.2 mmol), benzotriazol-1-yloxytris(dimethylamino)phosphonium hexafluorophosphate (1.07 g, 2.42 mmol), and triethylamine (2.0 mL, 14 mmol) in dimethylformamide (DMF) (20.0 mL) was stirred at room temperature overnight. LS-MS showed the reaction was complete. EtOAc (60 mL) and saturated NaHCO₃ aqueous solution (60 mL) were added to the reaction mixture. After stirring at room temperature for 10 minutes, the organic phase was seperated and the aqueous layer was extracted with EtOAc three times. The combined organic phase was washed with brine, dried over anhydrous Na₂SO₄, filtered and evaporated under reduced pressure. Purification by flash chromatography provided the desired product {1-{1-[3-fluoro-2-(trifluoromethyl)isonicotinoyl]piperidin-4-yl}-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile. LC-MS: 684.3 (M+H)⁺.

### Step J: {1-{1-[3-Fluoro-2-(trifluoromethyl)isonicotinoyl]piperidin-4-yl}-3-[4-(7H-pyrrolo[2,3-d]pyrimidin-4-yl)-1H-pyrazol-1-yl]azetidin-3-yl}acetonitrile

Into a solution of {1-{1-[3-fluoro-2-(trifluoromethyl)isonicotinoyl]piperidin-4-yl}-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile (56 mg, 0.1 mmol) in methylene chloride (1.5 mL) was added trifluoroacetic acid (1.5 mL). The mixture was stirred at room temperature for 2 hours. After removing the solvents in vacuum, the residue was dissolved in a methanol solution containing 20% ethylenediamine. After being stirred at room temperature for 1 hour, the solution was purified by HPLC (method B) to give the title compound. LC-MS: 554.3 (M+H)⁺; ¹H NMR (400 MHz, CDCl₃): 9.71 (s, 1H), 8.82 (s, 1H), 8.55 (d, J=4.6 Hz, 1H), 8.39 (s, 1H), 8.30 (s, 1H), 7.52 (t, J=4.6 Hz, 1H), 7.39 (dd, J₁=3.4 Hz, J₂=1.5 Hz, 1H), 6.77 (dd, J₁=3.6 Hz, J₂=0.7 Hz, 1H), 4.18 (m, 1H), 3.75 (m, 2H), 3.63 (dd, J₁=7.8 Hz, J₂=3.7 Hz, 2H), 3.45 (m, 2H), 3.38 (s, 2H), 3.11 (m, 1H), 2.57 (m, 1H), 1.72 (m, 1H), 1.60 (m, 1H), 1.48 (m, 1H), 1.40 (m, 1H).

### Example 6. 4-{3-(Cyanomethyl)-3-[4-(7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide

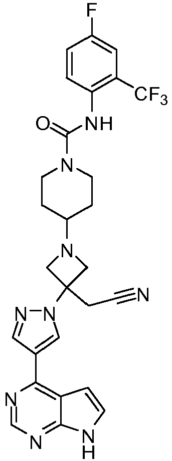

### Step A: 4-{3-(Cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide

To a solution of {1-piperidin-4-yl-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile trihydrochloride (500 mg, 1 mmol) in tetrahydrofuran (30 mL) were added triethylamine (0.29 g, 2.8 mmol) and 4-fluoro-1-isocyanato-2-(trifluoromethyl)benzene (190 mg, 0.95 mmol). The mixture was stirred at room temperature for 1 hour. The solvent was removed under reduced pressure. Purification by combi-flash using 30-100% EtOAc/hexanes gave 4-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide as a powder. LC-MS: 698.1 (M+H)⁺.

### Step B: 4-{3-(Cyanomethyl)-3-[4-(7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide

4-{3-(Cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide (210 mg, 0.3 mmol) was dissolved in a 50 M solution of trifluoroacetic acid in methylene chloride (20 mL). After being stirred at room temperature for one hour, the solvents were removed under reduced pressure. The residue was dissolved in methanol (20 mL) and ethylenediamine (1.0 g, 17 mmol). After being stirred at room temperature for one hour, the mixture was purified by HPLC (method B) to give 4-{3-(cyanomethyl)-3-[4-(7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide as a white powder. LC-MS: 568.1 (M+H)⁺. ¹H NMR (400 MHz, DMSO-d₆): δ 12.10 (s, 1H), 8.76 (s, 1H), 8.63 (s, 1H), 8.36 (s, 1H), 8.18 (s, 1H), 7.55 (d, J=3.6 Hz, 1H), 7.50 (m, 1H), 7.43 (m, 1H), 7.34 (m, 1H), 7.01(d, J=3.6 Hz, 1H), 3.79 (m, 2H), 3.67 (d, J=8 Hz, 2H), 3.51 (m, 4H), 2.92(m, 2H), 2.38 (m, 1H), 1.62 (m, 2H), 1.09 (m, 2H).

### Example 7. [3-[4-(7H-pyrrolo[2,3-d]pyrimidin-4-yl)-1H-pyrazol-1-yl]-1-(1-{[2-(trifluoromethyl)pyrimidin-4-yl]carbonyl}piperidin-4-yl)azetidin-3-yl]acetonitrile

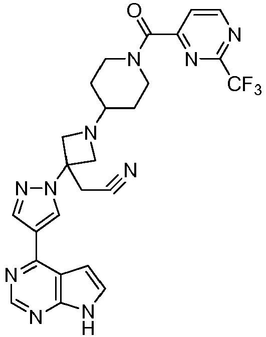

The title compound was prepared by a method analogous to the one used to prepare Example 5. LC-MS (M+H)+: 537.2.

### Example 8. [trans-1-[4-(7H-pyrrolo[2,3-d]pyrimidin-4-yl)-1H-pyrazol-1-yl]-3-(4-{[2-(trifluoromethyl)pyrimidin-4-yl]carbonyl}piperazin-1-yl)cyclobutyl] acetonitrile

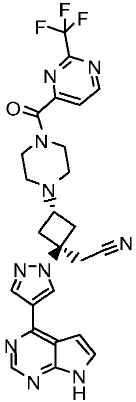

A mixture of 2-(trifluoromethyl)pyrimidine-4-carboxylic acid (0.225 g, 1.17 mmol, prepared by hydrolysis of the methyl ester obtained from Apollo as described in WO2006/067445), N,N,N',N'-tetramethyl-O-(7-azabenzotriazol-1-yl)uronium hexafluorophosphate (0.29 g, 0.76 mmol, Aldrich), and triethylamine (0.26 mL, 1.9 mmol) in tetrahydrofuran (6 mL) was prestirred for 15 minutes, followed by the addition of {*trans*-3-piperazin-1-yl-1-[4-(7-{[2-(trimethylsilyl)ethoxy]methyl}-7H-pyrrolo[2,3-d]pyrimidin-4-yl)-1H-pyrazol-1-yl]cyclobutyl}acetonitrile (0.188 g, 0.380 mmol, prepared as in Example 1b of US 2012/0149681, Step 1) in tetrahydrofuran (10 mL). The reaction was stirred overnight. THF was removed *in vacuo.* The residue was partitioned between saturated sodium bicarbonate and ethyl acetate. The aqueous portion was extracted a total of three times. The combined organic extracts were dried over sodium sulfate, decanted and concentrated. Flash chromatography, eluting with a gradient from 0-10% MeOH in DCM was used to purify the SEM-protected intermediate. Deprotection was effected by first stirring with trifluoroacetic acid (10 mL) in methylene chloride (10 mL) for 2 hours, followed by evaporation of solvent *in vacuo,* then stirring with methanol (6 mL, 200 mmol) containing ethylenediamine (0.5 mL, 7 mmol) overnight. The reaction mixture was partitioned between water and ethyl acetate, and the aqueous portion was extracted a further two times with ethyl acetate. The combined extracts were dried over sodium sulfate, filtered and concentrated. Flash chromatography was used to purify product, eluting with a gradient from 0-10% MeOH in DCM. The product was repurified preparative HPLC-MS (C18, eluting with a gradient of H₂O/MeCN containing 0.1% TFA).Acetonitrile was removed from the eluent containing the desired mass via rotary evaporation, then the remaining aqueous solution was neutralized by the addition of sodium bicarbonate and extracted with ethyl acetate several times. The combined organic extracts were dried over sodium sulfate, filtered and concentrated. The product was re-purified by preparative HPLC-MS (C18, eluting with a gradient of H₂O/MeCN containing 0.15% NH₄OH). The eluent containing the desired mass was frozen and lyophilized to afford product as the free base (99 mg, 48%). ¹H NMR (300 MHz, CD₃OD): δ 9.13 (d, 1H), 8.71 (s, 1H), 8.66 (s, 1H), 8.39 (s, 1H), 7.88 (d, 1H), 7.50 (d, 1H), 6.98 (d, 1H), 3.89-3.81 (m, 2H), 3.59-3.52 (m, 2H), 3.34 (s, 2H), 3.13-3.03 (m, 2H), 2.97 (tt, 1H), 2.59-2.42 (m, 6H); ¹⁹F NMR (282 MHz, CD₃OD): δ-72.43 (s, 3F); LCMS (M+H)⁺: 537.0.

### Example 9. {trans-3-(4-{[4-[(3-hydroxyazetidin-1-yl)methyl]-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

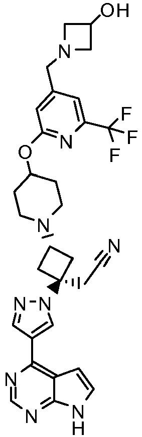

The procedure of Example 153 of US 2012/0149681 was followed, using N,N-diisopropylethylamine (64 µL, 0.37 mmol) and azetidin-3-ol hydrochloride (30 mg, 0.3 mmol, Oakwood) in the displacement step. After stirring overnight at room temperature, methanol (0.20 mL) was added to afford a homogenous solution, which was stirred for a further 2.5 hours at room temperature and treated according to the deprotection and purification conditions given in Example 153 of US 2012/0149681 to afford product as the free base (9.7 mg, 44%). ¹H NMR (400 MHz, dmso) δ 12.12 (br s, 1H), 8.81 (s, 1H), 8.67 (s, 1H), 8.40 (s, 1H), 7.59 (d, *J* = 3.6 Hz, 1H), 7.29 (s, 1H), 7.06 (d, *J* = 3.6 Hz, 1H), 6.93 (s, 1H), 5.34 (d, *J* = 6.4 Hz, 1H), 5.05 - 4.77 (m, 1H), 4.19 (h, *J* = 6.1 Hz, 1H), 3.60 (s, 2H), 3.50 (td, *J* = 6.1, 2.0 Hz, 2H), 3.40 (s, 2H), 3.06 - 2.92 (m, 2H), 2.86 - 2.71 (m, 3H), 2.68 - 2.53 (m, 2H), 2.38 - 2.22 (m, 2H), 2.22-2.07 (br m, 2H), 2.05-1.95 (br m, 2H), 1.75 - 1.48 (m, 2H); ¹⁹F NMR (376 MHz, dmso) δ-67.36 (s); LCMS (M+H)⁺: 608.2.

### Example 10. {trans-3-(4-{[4-{[(2S)-2-(hydroxymethyl)pyrrolidin-1-yl]methyl}-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

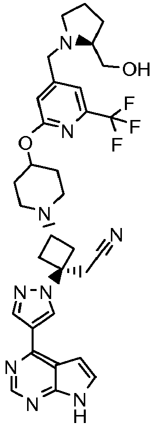

The method of Example 158 of US 2012/0149681 was followed, except that the displacement of mesylate with amine was carried out using (2*S*)-pyrrolidin-2-ylmethanol (20 µL, 0.2 mmol, Aldrich), at room temperature overnight (8.3 mg, 59%). ¹H NMR (500 MHz, DMSO) δ 12.09 (br s, 1H), 8.81 (s, 1H), 8.69 (s, 1H), 8.41 (s, 1H), 7.59 (d, *J* = 3.5 Hz, 1H), 7.39 (s, 1H), 7.06 (d, *J* = 3.6 Hz, 1H), 7.03 (s, 1H), 5.00 (tt, *J* = 8.4, 3.9 Hz, 1H), 4.48 (s, 1H), 4.12 (d, *J* = 14.8 Hz, 1H), 3.45 (d, *J* = 15.0 Hz, 1H), 3.41 (s, 2H), 3.42-3.25 (m, 2H), 3.06 - 2.97 (m, 2H), 2.87 - 2.77 (m, 2H), 2.69 - 2.62 (m, 2H), 2.59 (dddd, *J* = 5.8, 5.8, 5.8, 8.1 Hz, 1H), 2.41 - 2.31 (m, 2H), 2.22 - 2.09 (m, 3H), 2.08 - 1.95 (m, 2H), 1.83 (dddd, *J* = 8.1, 8.1, 8.3, 12.2 Hz, 1H), 1.75 - 1.46 (m, 5H); ¹⁹F NMR (376 MHz, dmso) δ-67.24 (s); LCMS (M+H)⁺: 636.3.

### Example 11. {trans-3-(4-{[4-{[(2R)-2-(hydroxymethyl)pyrrolidin-1-yl]methyl}-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

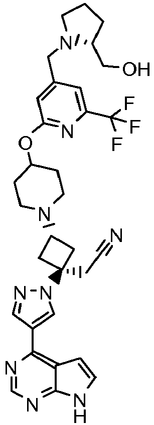

The method of Example 158 of US 2012/0149681 was followed, except that the displacement of mesylate with amine was carried out using (2*R*)-pyrrolidin-2-ylmethanol (20 µL, 0.2 mmol, Aldrich) at room temperature overnight (8.3 mg, 59%). ¹H NMR (400 MHz, dmso) δ 12.14 (br s, 1H), 8.83 (s, 1H), 8.69 (s, 1H), 8.42 (s, 1H), 7.60 (d, *J* = 3.6 Hz, 1H), 7.39 (s, 1H), 7.08 (d, *J* = 3.6 Hz, 1H), 7.03 (s, 1H), 5.04 - 4.94 (m, 1H), 4.52 (t, *J* = 5.4 Hz, 1H), 4.12 (d, *J* = 14.9 Hz, 1H), 3.52 - 3.22 (m, 5H), 3.09 - 2.92 (m, 2H), 2.86 - 2.73 (m, 2H), 2.70 - 2.53 (m, 3H), 2.42 - 2.27 (m, 2H), 2.22 - 2.09 (m, 3H), 2.06 - 1.87 (m, 2H), 1.82 (dddd, *J* = 8.0, 8.0, 8.4, 11.9 Hz, 1H), 1.77 - 1.37 (m, 5H); ¹⁹F NMR (376 MHz, dmso) δ -67.24 (s); LCMS (M+H)⁺: 636.3.

### Example 12. 4-(4-{3-[(Dimethylamino)methyl]-5-fluorophenoxy}piperidin-1-yl)-3-[4-(7H-pyrrolo[2,3-d]pyrimidin-4-yl)-1H-pyrazol-1-yl]butanenitrile (chiral)

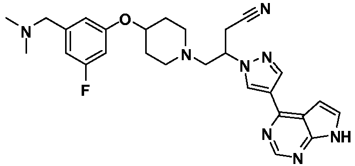

### Step 1. 3-Fluoro-5-hydroxybenzaldehyde

To a suspension of 3-fluoro-5-hydroxybenzonitrile (1.00 g, 7.29 mmol) in toluene (60.0 mL at -78 °C was added 1.0 M diisobutylaluminum hydride in toluene (18.2 mL, 18.2 mmol). The resulting mixture was stirred at -78 °C for 1 hour and allowed to warm to room temperature overnight. The 1:1 mixture of methanol and water (10 mL) was added and stirred for 35 minutes. The solid was filtered and washed with ethyl acetate. The filtrates were washed with water and brine then dried over Na₂SO₄, filtered, and concentrated. The crude product was purified with silica gel column (eluted with 10-50% ethyl acetate/hexanes) to give the desired product (0.77 g, 75%). ¹H NMR (DMSO-d₆) δ 10.49 (s, 1H), 9.88 (s, 1H), 7.10 (m, 2H), 6.87 (d, 1H).

### Step 2. 3-[(Dimethylamino)methyl]-5-fluorophenol

To a mixture of dimethylamine hydrochloride (160 mg, 1.96 mmol) and 3-fluoro-5-hydroxybenzaldehyde (250.0 mg, 1.784 mmol) in methylene chloride (9.0 mL) was added triethylamine (323 µL, 2.32 mmol) and resin of sodium triacetoxyborohydride (1.1 g, 2.7 mmol). The resulting mixture was stirred overnight then filtered and concentrated. The crude was purified by silica gel column (eluting with 0-15% methanol/DCM) to give the desired product (0.21 g, 70%). ¹H NMR (DMSO-d₆) δ 6.55 (m, 2H), 6.42 (d, 1H), 2.15 (s, 6H), 1.89 (s, 2H). LCMS (M+H)⁺: 170.1.

### Step 3. 4-(4-{3-[(Dimethylamino)methyl]-5-fluorophenoxy}piperidin-1-yl)-3-[4-(7H-pyrrolo[2,3-d]pyrimidin-4-yl)-1H-pyrazol-1-yl]butanenitrile

To a mixture of 3-[(dimethylamino)methyl]-5-fluorophenol (158 mg, 0.934 mmol) in methylene chloride (9 mL) was added Resin of triphenylphosphine (578 mg, 1.37 mmol) and di-*tert*-butyl azodicarboxylate (229 mg, 0.996 mmol). The mixture was stirred for 20 minutes before adding a solution of 4-(4-hydroxypiperidin-1-yl)-3-[4-(7-{[2-(trimethylsilyl)ethoxy]methyl}-7H-pyrrolo[2,3-d]pyrimidin-4-yl)-1H-pyrazol-1-yl]butanenitrile (300 mg, 0.6 mmol) in methylene chloride (2 mL). The reaction was stirred at room temperature overnight. Additional resin of triphenylphosphine (0.5 g), di-*tert*-butyl azodicarboxylate (0.23g), and DCM (8mL) were added and stirred for additional 2 hours. The vial and resin were washed with DCM and filtered. The filtrates were washed with 10% aq. NaOH solution. The organic layer was dried over MgSO₄, filtered, and concentrated. The crude was purified by silica gel column (eluted with 0-15% methanol/DCM) to give the SEM protected product. LCMS (M+H)⁺: 633.5. To the purified product was added methylene chloride (1.5 mL) and trifluoroacetic acid (1.5 mL, 19 mmol) and stirred for 2 hours. The solvents were evaporated before adding methanol (3.5 mL) and ethylenediamine (0.70 mL, 10 mmol). The resulting mixture was stirred for 1 hour then concentrated. The concentrate was taken up in DCM and washed with water, dried over Na₂SO₄, filtered, and concentrated to give the crude product which was purified by chiral prep-HPLC (Chiralcel OJ-H column, 4.6 x 250mm, 5 µ, 60% ethanol/Hex, 0.5 ml/min) to afford 2 enantiomers.
enantiomer 1 (first to elute): LCMS (M+H)⁺: 503.3.
enantiomer 2 (second to elute): ¹H NMR (DMSO-d₆) δ 8.78 (s, 1H), 8.67 (s, 1H), 8.35 (s, 1H), 7.59 (d, 1H), 6.96 (d, 1H), 6.64 (t, 3H), 4.94 (m, 1H), 4.36 (m, 1H), 3.39 (m, 2H), 3.19 (d, 3H), 2.77 (m, 3H), 2.60 (m, 1H), 2.32 (m, 2H), 2.10 (s, 6H), 1.83 (m, 2H), 1.54 (m, 2H). LCMS (M+H)⁺: 503.3.

### Example 13. 5-{3-(Cyanomethyl)-3-[4-(7H-pyrrolo[2,3-d]pyrimidin-4-yl)-1H-pyrazol-1-yl]azetidin-1-yl}-N-isopropylpyrazine-2-carboxamide

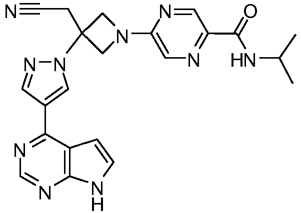

### Step 1: methyl 5-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}pyrazine-2-carboxylate

(R)-(+)-2,2'-Bis(diphenylphosphino)-1,1'-binaphthyl (0.065 g, 0.10 mmol) was added to a mixture of {3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile dihydrochloride (0.50 g, 1.0 mmol), methyl 5-chloropyrazine-2-carboxylate (0.18 g, 1.0 mmol)(Ark Pharm, Inc., Cat. #: AK-23920), and cesium carbonate (1.0 g, 3.1 mmol) in toluene (15.0 mL) under nitrogene, followed by palladium acetate (0.023 g, 0.10 mmol). The reaction mixture was stirred at 120°C for 3 h. After cooled to r.t., the reaction mixture was filtered throught a pad of celite, washed with ethyl acetate. The filtrate was concentrated under reduced pressure. The residue was purified by flash chromatography on a silica gel column with ethyl acetate in dichloromethane (0-70%) to afford the desired product (0.31 g, 55%). LCMS (M+H)⁺ : m/z = 546.3.

### Step 2: 5-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}pyrazine-2-carboxylic acid

A mixture of methyl 5-{3-(cyanomethyl)-3-[4-(7-{[2-(trimethylsilyl)ethoxy]-methyl}-7H-pyrrolo[2,3-d]pyrimidin-4-yl)-1H-pyrazol-1-yl]azetidin-1-yl}pyrazine-2-carboxylate (0.31 g, 0.57 mmol), lithium hydroxide monohydrate (0.060 g, 1.4 mmol) in methanol (6.0 mL) and water (2.5 mL) was stirred at 30°C overnight. The mixture was adjuested to pH = 4 with aqueous HCl, and concentrated under reduced pressure to remove MeOH. The resulted solid was filtered, washed with water and ether, and then dried in vacuum to afford the desired product (0.25 g, 83%). LCMS (M+H)⁺ : m/z = 532.3

### Step 3: 5-{3-(cyanomethyl)-3-[4-(7H-pyrrolo[2,3-d]pyrimidin-4-yl)-1H-pyrazol-1-yl]azetidin-1-yl}-N-isopropylpyrazine-2-carboxamide

Triethylamine (15 µL, 0.11 mmol) was added to a mixture of 5-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}pyrazine-2-carboxylic acid (19.4 mg, 0.0365 mmol) and benzotriazol-1-yloxytris(dimethylamino)phosphonium hexafluorophosphate (19 mg, 0.044 mmol) and 2-propanamine (3.2 mg, 0.055 mmol) in methylene chloride (1.3 mL). The reaction mixture was stirred at r.t. overnight. The reaction mixture was worked up with aqueous NaHCO₃, and extracted with methylene chloride (2 x 2 mL). The combined organic layers were washed with water (1 mL) and concentrated under reduced pressure. The residue was used for next step without further purification. LCMS (M+H)⁺ : m/z = 573.3.

Methylene chloride (1.3 mL) and trifluoroacetic Acid (0.6 mL) were added to the above intermediate. The reaction mixture was stirred at r.t. for 1.5 h. The mixture was concentrated under reduced pressure. The residue was dissolved in methanol (1.3 mL). To the solution was added ethylenediamine (0.086 mL). The reaction mixture was stirred at r.t. for 2 h., and purified by RP-HPLC (pH = 10) to afford the desired product. LCMS (M+H)⁺ : m/z = 443.2. ¹H NMR (400 MHz, DMSO-*d*₆): δ 12.15 (br, 1H), 8.97 (s, 1H), 8.68 (s, 1H), 8.63 (d, J = 1.2 Hz, 1H), 8.46 (s, 1H), 8.12 (d, = 8.4 Hz, 1H), 7.97 (d, J = 1.2 Hz, 1H), 7.60 (dd, J = 3.3, 2.4 Hz, 1H), 7.07 (dd, J = 3.4, 1.7 Hz, 1H), 4.81 (d, J = 9.8 Hz, 2H), 4.53 (d, J = 9.6 Hz, 2H), 4.13-4.02 (m, 1H), 3.78 (s, 2H), 1.14 (d, J = 6.8 Hz, 6H).

### Example 14. 4-{3-(Cyanomethyl)-3-[4-(7H-pyrrolo[2,3-d]pyrimidin-4-yl)-1H-pyrazol-1-yl]azetidin-1-yl}-2,5-difluoro-N-[(1S)-2,2,2-trifluoro-1-methylethyl]benzamide

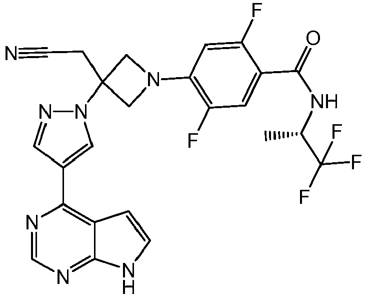

### Step 1: 4-chloro-2,5-difluoro-N-[(1S)-2,2,2-trifluoro-1-methylethyl]benzamide

4-Chloro-2,5-difluorobenzoyl chloride (29.6 mg, 0.140 mmol) (Oakwood, Cat.#: 001628) was added to a mixture of (2S)-1,1,1-trifluoropropan-2-amine hydrochloride (20.0 mg, 0.134 mmol) (SynQuest Lab, Cat.#: 3130-7-S1) and diisopropylethylamine (58 µL, 0.33 mmol) in dichloromethylene (4.0 mL) at 0 °C. The reaction mixture was stirred at room temperature for 30 min., worked up with saturated aqueous NaHCO₃, and extracted with dichloromethylene (3x10 mL). The combined organic layers were washed with brine, dried over MgSO₄, filtered and concentrated under reduced pressure to afford the desired product which was directly used in the next step reaction without further purification. LCMS (M+H)⁺: m/z = 288.0/290.0.

### Step 2: 4-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}-2,5-difluoro-N-[(1S)-2,2,2-trifluoro-1-methylethyl]benzamide

(R)-(+)-2,2'-Bis(diphenylphosphino)-1,1'-binaphthyl (8.3 mg, 0.013 mmol) was added to a mixture of {3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile dihydrochloride (65 mg, 0.13 mmol), 4-chloro-2,5-difluoro-N-[(1S)-2,2,2-trifluoro-1-methylethyl]benzamide (0.14 mmol), and cesium carbonate (0.13 g, 0.40 mmol) in toluene (4.0 mL) under N₂, followed by palladium acetate (3.0 mg, 0.013 mmol). The reaction mixture was stirred at 130 °C for 5 h. After the reaction mixture was cooled to room temperature, the mixture was worked up with water, and extracted with ethyl acetate (3x10 mL). The combined organic layers were washed with brine, dried over MgSO₄, filtered and concentrated under reduced pressure to afford the crude product which was directly used in the next step reaction without further purification. LCMS (M+H)⁺: m/z = 661.2.

### Step 3: 4-{3-(cyanomethyl)-3-[4-(7H-pyrrolo[2,3-d]pyrimidin-4-yl)-1H-pyrazol-1-yl]azetidin-1-yl}-2,5-difluoro-N-[(1S)-2,2,2-trifluoro-1-methylethyl]benzamide

Boron trifluoride etherate (0.051 mL, 0.40 mmol) was added to a solution of 4-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}-2,5-difluoro-N-[(1S)-2,2,2-trifluoro-1-methylethyl]benzamide in acetonitrile (1.0 mL) at 0 °C under N₂. The reaction mixture was stirred at room temperature for 3 h. (LCMS (M+H)⁺: m/z = 561.3). Then the mixture was cooled to 0 °C, water (0.13 mL) was added. After 30 min, 5.0 M ammonium hydroxide in water (0.2 mL, 1 mmol) was added slowly at 0 °C over 5 min. The reaction mixture was stirred at room temperature overnight, and purified by RP-HPLC (pH = 10) to afford the desired product. LCMS (M+H)⁺: m/z = 531.0. ¹H NMR (400 MHz, DMSO-d6): δ 12.62 (br, 1H), 9.07 (s, 1H), 8.84 (s, 1H), 8.55 (s, 1H), 8.51 (dd, J = 8.8, 1.2 Hz, 1H), 7.78 (br, 1H), 7.35 (dd, J = 12.6, 6.5 Hz, 1H), 7.23 (d, J = 1.9 Hz, 1H), 6.65 (dd, J = 11.9, 7.3 Hz, 1H), 4.76 (m, 1H), 4.70 (d, J = 9.3 Hz, 2H), 4.44 (d, J = 9.2 Hz, 2H), 3.76 (s, 2H), 1.30 (d, J = 7.1 Hz, 3H).

### Example 15. 5-{3-(Cyanomethyl)-3-[4-(1H-pyrrolo[2,3-b]pyridin-4-yl)-1H-pyrazol-1-yl]azetidin-1-yl}-N-isopropylpyrazine-2-carboxamide

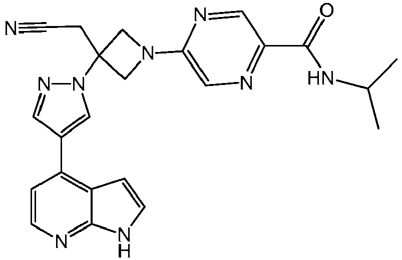

### Step 1: methyl 5-{3-(cyanomethyl)-3-[4-(1-{[2-(trimethylsilyl)ethoxy]methyl}-1H-pyrrolo[2,3-b]pyridin-4-yl)-1H-pyrazol-1-yl]azetidin-1-yl}pyrazine-2-carboxylate

N,N-Diisopropylethylamine (1.0 mL, 6.0 mmol) was added to a mixture of {3-[4-(1-{[2-(trimethylsilyl)ethoxy]methyl}-1H-pyrrolo[2,3-b]pyridin-4-yl)-1H-pyrazol-1-yl]azetidin-3-yl}acetonitrile dihydrochloride (0.96 g, 2.0 mmol) and methyl 5-chloropyrazine-2-carboxylate (0.34 g, 2.0 mmol) in 1,4-dioxane (15 mL). The reaction mixture was stirred at 120 °C overnight. The mixture was worked up with saturated aqueous NaHCO₃, and extracted with dichloromethylene (3x 20 mL). The combined organic layers were washed with brine, dried over MgSO₄, filtered and concentrated under reduced pressure. The residue was purified by flash chromatography on a silica gel column with ethyl acetate in hexanes (0-60%) to afford the desired product (0.13 g, 12%). LCMS (M+H)⁺: m/z = 545.2.

### Step 2: 5-{3-(cyanomethyl)-3-[4-(1-{[2-(trimethylsilyl)ethoxy]methyl}-1H-pyrrolo[2,3-b]pyridin-4-yl)-1H-pyrazol-1-yl]azetidin-1-yl}pyrazine-2-carboxylic acid

A reaction mixture of methyl 5-{3-(cyanomethyl)-3-[4-(1-{[2-(trimethylsilyl)ethoxy]methyl}-1H-pyrrolo[2,3-b]pyridin-4-yl)-1H-pyrazol-1-yl]azetidin-1-yl}pyrazine-2-carboxylate (0.13 g, 0.24 mmol), lithium hydroxide monohydrate (0.025 g, 0.60 mmol) in methanol (4.0 mL), THF (2.0 mL) and water (1.0 mL) was stirred at 40 °C for 3 h. The mixture was adjusted to pH = 4 with 2.0 N HCl aqueous solution, and concentrated under reduced pressure to remove MeOH and THF. The precipitate formed was filtered, washed with water and ether, and dried in vacuum to afford the desired product (0.100 g, 79%). LCMS (M+H)⁺ : m/z = 531.4.

### Step 3: 5-{3-(cyanomethyl)-3-[4-(1H-pyrrolo[2,3-b]pyridin-4-yl)-1H-pyrazol-1-yl]azetidin-1-yl}-N-isopropylpyrazine-2-carboxamide

N,N-Diisopropylethylamine (19 µL, 0.11 mmol) was added to a mixture of 5-{3-(cyanomethyl)-3-[4-(1-{[2-(trimethylsilyl)ethoxy]methyl}-1H-pyrrolo[2,3-b]pyridin-4-yl)-1H-pyrazol-1-yl]azetidin-1-yl}pyrazine-2-carboxylic acid (19.4 mg, 0.0365 mmol), benzotriazol-1-yloxytris(dimethylamino)phosphonium hexafluorophosphate (19 mg, 0.044 mmol) and 2-propanamine (3.2 mg, 0.055 mmol) in DMF (1.0 mL). The reaction mixture was stirred at room temperature overnight. The reaction mixture was worked up with saturated aqueous NaHCO₃, and extracted with dichloromethylene (3x20 mL). The combined organic layers were washed with brine, dried over MgSO₄, filtered and concentrated under reduced pressure. The residue was treated with methylene chloride (1.3 mL) and TFA (1.3 mL). The mixture was stirred at room temperature for 1.5 h., and concentrated under reduced pressure. The residue was dissolved in methanol (1.3 mL), and treated with ethylenediamine (0.086 mL, 1.3 mmol). The resulting mixture was stirred at room temperature for 2 h, and then purified by RP-HPLC (pH = 10) to afford the desired product. LCMS (M+H)⁺: m/z = 442.1. ¹H NMR (400 MHz, DMSO- *d*₆): δ 12.19 (br, 1H), 8.99 (s, 1H), 8.66 (d, J = 1.4 Hz, 1H), 8.47 (s, 1H), 8.32 (d, J = 5.7 Hz, 1H), 8.14 (d, J = 8.4 Hz, 1H), 8.00 (d, J = 1.4 Hz, 1H), 7.67 (dd, J = 3.2, 2.7 Hz, 1H), 7.54 (d, J = 5.5 Hz, 1H), 7.09 (dd, J = 3.5, 2.7 Hz), 4.82 (d, J = 10.0 Hz, 2H), 4.56 (d, J = 10.0 Hz, 2H), 4.10 (m, 1H), 3.79 (s, 2H), 1.17 (d, J = 6.4 Hz, 6H).

### Example 16: {1-(cis-4-{[6-(2-hydroxyethyl)-2-(trifluoromethyl)pyrimidin-4-yl]oxy}cyclohexyl)-3-[4-(7H-pyrrolo[2,3-d]pyrimidin-4-yl)-1H-pyrazol-1-yl]azetidin-3-yl}acetonitrile tris(trifluoroacetate)

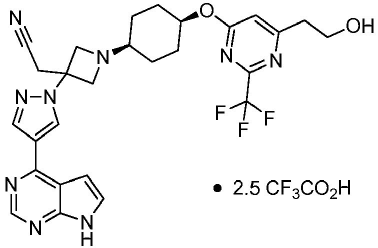

### Step 1: Diethyl [6-(1,4-dioxaspiro[4.5]dec-8-yloxy)-2-(trifluoromethyl)pyrimidin-4yl]malonate

To a mixture of tetrahydrofuran (40 mL) and NaH in mineral oil (1.1 g, 28 mmol) at 0 °C was added ethyl malonate (4.2 mL, 28 mmol), dropwise. 4-chloro-6-(1,4-dioxaspiro[4.5]dec-8-yloxy)-2-(trifluoromethyl)pyrimidine (described in Example 1 of US 2013/0045963, Step 1) (3.75 g, 11.1 mmol), was then added. The reaction mixture was stirred at 64 °C. After 3 hours, HPLC & LCMS analysis showed 70% reaction completion. Heated for another 6 hours, and then cooled to 20 °C. Only a trace of decarboxylation product formed. The reaction was diluted with aqueous bicarbonate, and extracted with EtOAc. The EtOAc extract was washed with brine, dried over Na₂SO₄, and evaporated in vacuo to give 8.5 g oil (includes excess ethyl malonate and mineral oil). The crude product was purified by chromatography on a 120 g silica gel column, using solvent A= hexane; solvent B= EtOAc; flow 60 mL/min; A, 3 min; Gradient to 40%B in 40min; detector set at 254nm; collected 47 mL fractions; retention time, 28 min. The combined fractions were evaporated to give 4.6 g, colorless oil, 90 % yield. ¹H NMR (300 MHz, CDCl₃): δ7.05 (s, 1H); 5.30 (m, 1H, OCH); 4.85 (s, 1H, CH); 4.25 (m, 2H, OCH₂); 3.95 (s, 4H, OCH₂); 1.6-2.1 (m, 8H); 1.28 (t, 3H, CH₃).

### Stp 2: Ethyl [6-(1,4-dioxaspiro[4.5]dec-8-yloxy)-2-(trifluoromethyl)pyrimidin-4-yl]acetate

Diethyl [6-(1,4-dioxaspiro[4.5]dec-8-yloxy)-2-(trifluoromethyl)pyrimidin-4-yl]malonate (4.60 g, 9.95 mmol), was dissolved in ethanol (46 mL). Water (18 µL, 1.0 mmol) and 21% Sodium ethoxide in ethanol (0.37 mL, 1.0 mmol) were added. The reaction mixture was stirred at 75 °C for 1 hour. HPLC & LCMS analysis showed 60% decarboxylation. The heating was continued for another 2 hours (reaction complete). The reaction was diluted with aqueous bicarbonate, and extracted with EtOAc. The EtOAc extract was washed with brine, then dried (Na₂SO₄), and evaporated *in vacuo* to 3.4 g oil (88% yield). LCMS, HPLC, & NMR showed it to be clean enough to proceed. ¹H NMR (400 MHz, DMSO-D₆): δ7.20 (s, 1H); 5.20 (m, 1H, OCH); 4.10 (q, 2H, OCH₂); 3.89 (s, 2H, CH₂); 3.85 (s, 4H, OCH₂); 1.5-2.0 (m, 8H); 1.15 (t, 3H, CH₃). HPLC showed it to have UVₘₐₓ 222 & 252nm.

### Step 3: 2-[6-(1,4-dioxaspiro[4.5]dec-8-yloxy)-2-(trifluoromethyl)pyrimidin-4-yl]ethanol

Ethyl [6-(1,4-dioxaspiro[4.5]dec-8-yloxy)-2-(trifluoromethyl)pyrimidin-4-yl]acetate(3.0g) was dissolved in tetrahydrofuran (40 mL), and cooled in an ice bath. Sodium tetrahydroborate (884 mg, 23.4 mmol) was added followed by methanol (4.8 mL, 120 mmol), in portions. The reaction mixture was stirred for 20 min, removed the ice bath, and stirred at 21 °C for 0.5 hour. HPLC & LCMS showed no remaining ester, and showed conversion to the desired M+H 349; and also showed several over reduction products (at least one of which has no UV absorbance). The reaction mixture was quenched with water and evaporated. The reaction mixture was diluted with aqueous bicarbonate and EtOAc, and stirred for 0.5 hour. The EtOAc layer was washed with brine, dried (Na₂SO₄), and evaporated to give 3.0 g oil. The product was purified by chromatography on a 120 g silica gel column, using solvent A= hexane; solvent B= 3%iPA/EtOAc; flow 60 mL/min; A, 3 min; Gradient to 50%B in 30 min, then 50%B for 15 min; detector set at 254 nm; collected 47 mL fractions; retention time, 34 min. evaporated to yield 1.5 g, a light yellow viscous oil, 56% yield. ¹H NMR (300 MHz, DMSO-D₆): δ7.10 (s, 1H); 5.20 (m, 1H, OCH); 4.71 (t, 1H, OH); 3.85 (s, 4H, OCH₂); 3.72 (q, 2H, OCH₂); 2.85 (t, 2H, CH₂); 1.5-2.0 (m, 8H).

### Step 4: 4-{[6-(2-hydroxyethyl)-2-(trifluoromethyl)pyrimidin-4-yl]oxy}cyclohexanone

2-[6-(1,4-Dioxaspiro[4.5]dec-8-yloxy)-2-(trifluoromethyl)pyrimidin-4-yl]ethanol
was dissolved in acetone (60 mL, 900 mmol), and 5.0 M hydrogen chloride in water (20 mL, 98 mmol) was added and stirred for 17 hours. LCMS and HPLC analysis showed nearly complete conversion to M+H 305. Aqueous bicarbonate was added and the reaction mixture was stirred, then concentrated. This mixture was extracted with EtOAc. The EtOAc was dried (Na₂SO₄), and evaporated in vacuo to give 1.3 g light yellow viscous oil (used in the next reaction without purification). ¹H NMR (300 MHz, CDCl₃): δ6.80 (s, 1H); 5.60 (m, 1H, OCH); 4.06 (t, 2H, OCH₂); 3.04 (t, 2H, CH₂); 2.61 (m, 2H); 2.45 (m, 2H); 2.25 (m, 2H).

### Step 5:{1-(4-{[6-(2-hydroxyethyl)-2-(trifluoromethyl)pyrimidin-4-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile

{3-[4-(7-{[2-(Trimethylsilyl)ethoxy]methyl}-7H-pyrrolo[2,3-d]pyrimidin-4-yl)-1H-pyrazol-1-yl]azetidin-3-yl}acetonitrile dihydrochloride (1.9 g, 3.9 mmol), and 4-{[6-(2-hydroxyethyl)-2-(trifluoromethyl)pyrimidin-4-yl]oxy}cyclohexanone (1.3 g, 4.3 mmol), in dry tetrahydrofuran (36 mL) were stirred for 15 min under nitrogen. Sodium triacetoxyborohydride (1.7 g, 8.2 mmol) was then added. The mixture was stirred at 20 °C for 16 hours. HPLC and LCMS analysis showed clean conversion to the *trans* and *cis* products (M+H 698; 1:1 ratio). The reaction was quenched with water, concentrated, stirred with 20% KHCO₃ and extracted with ethyl acetate, dried (Na₂SO₄), filtered, and evaporated to give 2.8 g. The isomeric products were separated by prep LCMS using a Waters instrument and a 30mm×100mm Xbridge C18 column; 60mL/min; 55% CH₃CN-H₂O (0.1%NH₄OH); 0.5min; 4.5 gradient to 72%; 24 runs; retention time *trans* isomer, 4.6 min; cis isomer, 5.4 min. The isolated *cis* isomer contained <1% residual *trans* isomer. Yield 1.00 g *cis* isomer, 37% yield. ¹H NMR (500 MHz, CDCl₃; also COSY, HSQC, and HMBC): δ8.83 (s, 1H); 8.40 (s, 1H); 8.28 (s, 1H); 7.40 (m, 1H); 6.80 (m, 1H); 6.67 (s, 1H); 5.64 (s, 2H, SEM); 5.17 (m, 1H, OCH); 4.01 (t, 2H, OCH₂); 3.74 (s, 2H, NCH); 3.59 (m, 2H, NCH); 3.55 (t, 2H, SEM); 3.38 (s, 2H, CH₂CN); 2.95 (t, 2H, CH₂); 2.30 (m, 1H, NCH); 2.15 (m, 2H); 1.84 (m, 2H); 1.50 (m, 2H); 1.30 (m, 2H); 0.90 (t, 2H, SEM); -0.92 (s, 9H, SEM).

### Step 6: {1-(cis-4-{[6-(2-hydroxyethyl)-2-(trifluoromethyl)pyrimidin-4-yl]oxy}cyclohexyl)-3-[4-(7H-pyrrolo[2,3-d]pyrimidin-4-yl)-1H-pyrazol-1-yl]azetidin-3-yl}acetonitrile tris(trifluoroacetate)

{1-(4-{[6-(2-Hydroxyethyl)-2-(trifluoromethyl)pyrimidin-4-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile isomer was dissolved in methylene chloride (18 mL) and trifluoroacetic acid (TFA, 18 mL, 230 mmol) and was stirred for 1.0 hour. The solution was concentrated to remove TFA. LCMS showed conversion to the hydroxymethyl intermediate, M+H 598, some of its TFA ester, M+H 694, and <5% residual SEM. The residue was dissolved in methanol (36 mL) and 15.0 M ammonium hydroxide in water (9.0 mL, 130 mmol) was added. The solution was stirred at 21 °C for 18 hours. HPLC & LCMS showed no remaining M+H 598 peak or TFA ester. The solution was evaporated. Ammonium trifluoroacetate was removed by adding aqueous bicarbonate and extracting the product with EtOAc. The combined EtOAc extract was evaporated to give 0.96 g. This was dissolved in 70 mL of 10% H₂O/ACN containing 1.5 equiv TFA (180 µL). The product was isolated by prep LCMS using a Waters Fraction-Linx instrument and a 30mm×100mm Sunfire C18 column; 60 mL/min; 15% ACN-H₂O (0.1%TFA), 0.5 min; 4.5 min gradient to 33%; detector set at m/z 568; 14 runs; retention time 5.0 min. HPLC showed UVₘₐₓ 224, 252, 294, and 318 nm. The combined fractions were freeze-dried. Yield 1.0 g white solid (80% yield). NMR showed it to be the 2.5 TFA salt. ¹H NMR (500 MHz, CD₃CN; also COSY, HSQC, and HMBC): δ10.84 (s, 1H, NH); 9.00 (s, 1H); 8.90 (s, 1H); 8.56 (s, 1H); 7.66 (m, 1H); 7.10 (m, 1H); 6.86 (s, 1H); 5.39 (m, 1H, OCH); 4.86 (brs, 2H, NCH); 4.66 (m, 2H, NCH); 3.90 (t, 2H, OCH₂); 3.78 (s, 2H, CH₂CN); 3.39 (m, 1H, NCH); 2.92 (t, 2H, CH₂); 2.20 (m, 2H); 1.92 (m, 2H); 1.76 (m, 4H). ¹⁹F NMR (400 MHz, DMSO-D₆): δ-69.8 (s); - 74.8 (s, TFA); LCMS calculated for C₂₇H₂₉F₃N₉O₂ (M+H)⁺: m/z =568.24

### Example 17: {1-(cis-4-{[4-[(ethylamino)methyl]-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile tris(trifluoroacetate)

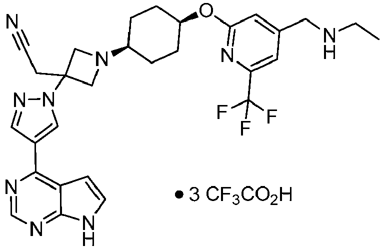

### Step 1: [2-[(cis-4-{3-(cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}cyclohexyl)oxy]-6-(trifluoromethyl)pyridin-4-yl]methyl methanesulfonate

{1-(*cis*-4-{[4-(Hydroxymethyl)-6-(trifluoromethyl)pyridin-2-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile (from Example 64 of US 2013/0045963, 145.0 mg, 0.2124 mmol) was dissolved in methylene chloride (2.93 mL) and was cooled to 0 °C. To that N,N-diisopropylethylamine (60.5 µL, 0.347 mmol) was added followed by methanesulfonyl chloride (23 µL, 0.30 mmol). The reaction was stirred at 0 °C for 1 hour. Then the reaction mixture was worked up with EtOAc and used in the next reaction. MS(ES):761(M+1).

### Step 2:(1-(cis-4-{[4-[(ethylamino)methyl]-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile tris(trifluoroacetate)

[2-[(*cis*-4-{3-(Cyanomethyl)-3-[4-(7-{[2-(trimethylsilyl)ethoxy]methyl}-7H-pyrrolo[2,3-d]pyrimidin-4-yl)-1H-pyrazol-1-yl]azetidin-1-yl}cyclohexyl)oxy]-6-(trifluoromethyl)pyridin-4-yl]methyl methanesulfonate (50 mg, 0.06571 mmol) was dissolved in 1,4-dioxane (2.5 mL) and, 2.0 M ethylamine in THF (300 µL, 0.6 mmol) was added. The reaction was stirred at 25 °C for 16 hours at which time LCMS analysis showed mainly product. The product were purified by LC, evaporated, and deprotected as in Example 1 of US 2013/0045963 and purified by LC to give the product. ¹H NMR (400 MHz, CD₃OD) δ 9.08 (s, 1H), 8.87 (s, 1H), 8.58 (s, 1H), 7.78 (d, 1H), 7.50 (s, 1H), 7.25 (d, 1H), 7.13 (s, 1H), 5.38 (m, 1H), 5.08 (d, 2H), 4.80 (d, 2H), 4.27 (s, 2H), 3.74 (s, 2H), 3.50 (m, 1H), 3.16 (q, 2H), 2.24 (m, 2H), 2.01 (m, 2H), 1.76 (m, 4H), 1.34 (t, 3H). ¹⁹F NMR (376 MHz, CD₃OD) δ-70.52 (s), -77.49 (s). MS(ES):580(M+1).

### Example 18: {1-(cis-4-{[4-(1-hydroxy-1-methylethyl)-6-(trifluoromethyl)pyridin-2-yl] oxy} cyclohexyl)-3- [4-(7H-pyrrolo [2,3-d] pyrimidin-4-yl)-1H-pyrazol-1-yl]azetidin-3-yl}acetonitrile bis(trifluoroacetate)

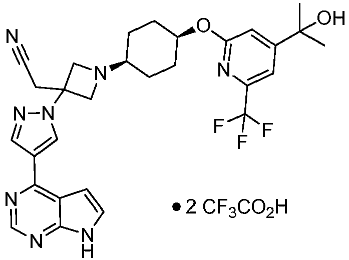

### Step 1: 2-chloro-6-(trifluoromethyl)isonicotinic acid

2-Chloro-6-(trifluoromethyl)pyridine (1.0 g, 5.51 mmol, Oakwood Products) was dissolved in tetrahydrofuran (20 mL) and 1.0 M lithium chloride - chloro(2,2,6,6-tetramethylpiperidin-1-yl)magnesium (1:1) in THF (6.610 mL, 6.610 mmol, Aldrich Co.) was added at 25 °C. The reaction was stirred at 25 °C for 1 hour and was cooled to
-78 °C. The reaction was stirred at -78 °C for 1 hour and allowed to warm to room temperature, quenched with water, and was partitioned between IN NaOH and ether. The phases were separated and the aqueous phase was washed with additional ether and acidified with concentrated HCl to pH∼1 and extracted with ether. The combined organic phase was washed with water, saturated NaCl, dried over MgSO₄, filtered, and evaporated to dryness to provide the crude product. NMR analysis showed that it consisted of a ∼2:1 mixture of the para and meta carboxylic acids. The mixture was carried over to the next reaction. 440 MHz NMR(CDCl₃) δ 8.17 (s, 1H), 8.11 (s, 1H).

### Step 2: ethyl 2-chloro-6-(trifluoromethyl)isonicotinate and ethyl 2-chloro-6-(trifluoromethyl)nicotinate

In a vial, 2-chloro-6-(trifluoromethyl)nicotinic acid (0.98 g, 4.4 mmol) and 2-chloro-6-(trifluoromethyl)isonicotinic acid (1.85 g, 8.2 mmol) were dissolved in ethyl orthoformate (5.0 mL, 30.1 mmol) and heated at 120 °C for 5 hours at which time TLC analysis showed that most of the starting material had been consumed and the products were formed. The reaction mixture was evaporated in vacuo and the residue was purified by silica gel chromatography using 10% EtOAc/hexanes to give the two ethyl ester products. ¹H NMR (400 MHz, CDCl₃): δ 8.14 (s, 1H), 8.08 (s, 1H), 4.47 (q, 2H), 1.44 (t, 3H).

### Step 3: 2-[2-chloro-6-(trifluoromethyl)pyridin-4-yl]propan-2-ol

Ethyl 2-chloro-6-(trifluoromethyl)isonicotinate (0.35 g, 1.4 mmol) was dissolved in tetrahydrofuran (13.8 mL) and was cooled to -78 °C, then 3.0 M methylmagnesium bromide in ether (1.4 mL, 4.1 mmol) was added. The reaction was stirred at -78 °C for 3 hours at which time LCMS analysis showed absence of starting material. The reaction was quenched with saturated NH₄Cl and was partitioned between water/1 N HCl and EtOAc, the phases were separated and the aqueous phase was washed with additional EtOAc. The combined organic phase was washed with water, saturated NaCl, dried over MgSO₄, filtered and evaporated to dryness to provide the crude product. NMR analysis showed that it consisted of a ∼1:1 mixture of the alcohol and the methyl ketone intermediate. The crude material used in the next reaction without purification.. NMR 400 MHz NMR(CDCl₃): δ 7.70 (s, 1H), 7.63 (s, 1H), 1.60 (s, 6H)

### Step 4: 2-[2-(1,4-dioxaspiro[4.5]dec-8-yloxy)-6-(trifluoromethyl)pyridin-4-yl]propan-2-ol

1,4-Dioxaspiro[4.5]decan-8-ol (0.25 g, 1.58 mmol) and 2-[2-chloro-6-(trifluoromethyl)pyridin-4-yl]propan-2-ol (0.2 g, 0.835 mmol) were dissolved in tetrahydrofuran (2 mL) and cooled to 0 °C and a 60% mixture of sodium hydride (70.0 mg, 1.75 mmol) in mineral oil was added and the reaction was stirred for 30 minutes at 0 °C and at 25 °C for 60 hours at which time TLC analysis indicated the presence of some product. The reaction was quenched with water, and was extracted with ethyl acetate and the organic extracts were washed with water, saturated NaCl, dried (MgSO₄), and evaporated in vacuo. The residue was purified by LC (pH 2) to give the product. MS(ES):362 (M+1).

### Step 5: 4-{[4-(1-hydroxy-1-methylethyl)-6-(trifluoromethyl)pyridin-2-yl]oxy}cyclohexanone

2-[2-(1,4-Dioxaspiro[4.5]dec-8-yloxy)-6-(trifluoromethyl)pyridin-4-yl]propan-2-ol (0.049 g, 0.14 mmol) was dissolved in acetone (3.7 mL). A solution of 12.0 M hydrogen chloride in water (0.43 mL, 5.2 mmol) was added and was stirred at 25 °C for 16 hours at which time LCMS showed about 70% reaction was complete. An additional 12.0 M hydrogen chloride in water (0.43 mL, 5.2 mmol) was added and was stirred for 3 hours; LCMS showed ∼90% reaction was complete and
was quenched into excess NaHCO₃, extracted with EtOAc and the organic extract was evaporated to give the product. This was used in the next reaction without purification. MS(ES):318 (M+1).

### Step 5: {1-(cis-4-{[4-(1-hydroxy-1-methylethyl)-6-(trifluoromethyl)pyridin-2-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile

{3-[4-(7-{[2-(Trimethylsilyl)ethoxy]methyl}-7H-pyrrolo[2,3-d]pyrimidin-4-yl)-1H-pyrazol-1-yl]azetidin-3-yl}acetonitrile dihydrochloride (55.3 mg, 0.115 mmol) and 4-{[4-(1-hydroxy-1-methylethyl)-6-(trifluoromethyl)pyridin-2-yl]oxy}cyclohexanone were dissolved in dry 1,2-dichloroethane (1.38 mL) and were stirred for 5 min and sodium triacetoxyborohydride (86.1 mg, 0.406 mmol) was added. The reaction mixture was stirred at 25 °C for 16h, at which time LCMS analysis showed mainly the two diastereomeric products. The reaction was quenched with water, neutralized with NaHCO₃ and extracted with ethyl acetate and the solvent was evaporated. The residue was purified by LCMS (pH 10) and the fractions containing the second peak were combined and evaporated to give {1-(*cis*-4-{[4-(1-hydroxy-1-methylethyl)-6-(trifluoromethyl)pyridin-2-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile. The first peak was also isolated to give {1-(trans-4-{[4-(1-hydroxy-1-methylethyl)-6-(trifluoromethyl)pyridin-2-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile. MS(ES): 712(M+1).

### Step 6: {1-(cis-4-{[4-(1-hydroxy-1-methylethyl)-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile bis(trifluoroacetate)

{1-(*cis*-4-{[4-(1-Hydroxy-1-methylethyl)-6-(trifluoromethyl)pyridin-2-yl]oxy}cyclohexyl)-3-[4-(7-{[2-(trimethylsilyl)ethoxy]methyl}-7H-pyrrolo[2,3-d]pyrimidin-4-yl)-1H-pyrazol-1-yl]azetidin-3-yl}acetonitrile was deprotected as described in Example 1 of US 2013/0045963 and was purified by liquid chromatography (pH 2) to give 1-(*cis*-4-{[4-(1-hydroxy-1-methylethyl)-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile bis(trifluoroacetate. In a similar manner {1-(trans-4-{[4-(1-hydroxy-1-methylethyl)-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile bis(trifluoroacetate) was prepared and characterized. ¹H NMR (400 MHz, CD₃OD) δ 9.07 (s, 1H), 8.87 (s, 1H), 8.59 (s, 1H), 7.78 (d, *J* = 3.7 Hz, 1H), 7.44 (s, 1H), 7.24 (d, *J* = 3.7 Hz, 1H), 7.05 (s, 1H), 5.35 (s, 1H), 5.09 (d, *J* = 12.2 Hz, 2H), 4.82 (d, *J* = 12.2 Hz, 2H), 3.72 (s, 2H), 3.5 (m, 1H), 2.27 (m, 2H), 2.0 (m, 2H), 1.74 (m, 4H), 1.50 (s, 6H). MS(ES): 581(M+1).

### Examples 19 and 20 below were prepared analogously to the procedure of Example 17.

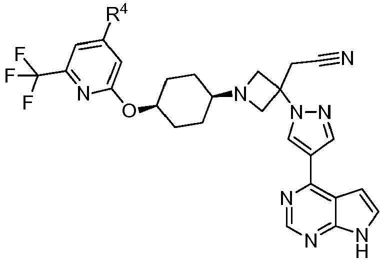

| Example No. | R4 | MS (M+H)⁺ | Name |
|---|---|---|---|
| 19 | 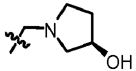 | 622 | {1-(*cis*-4-{[4-{[(3R)-3-hydroxypyrrolidin-1-yl]methyl}-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile pentakis(trifluoroacetate) |
| 20 | 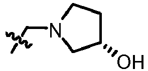 | 622 | {1-(*cis-*4-{[4-{[(3S)-3-hydroxypyrrolidin-1-yl]methyl}-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile tris(trifluoroacetate) |

### Example 21. {trans-3-(4-{[4-({[(1S)-2-hydroxy-1-methylethyl]amino}methyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

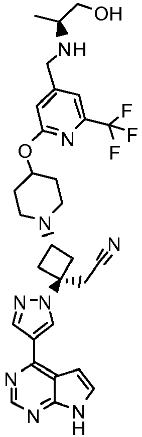

N,N-Diisopropylethylamine (9.4 µL, 0.054 mmol) and methanesulphonic anhydride (7.9 mg, 0.045 mmol) were added to a solution of {trans-3-(4-{[4-(hydroxymethyl)-6-(trifluoromethyl)pyridin-2-yl]oxy}piperidin-1-yl)-1-[4-(7-{[2-(trimethylsilyl)ethoxy]methyl}-7H-pyrrolo[2,3-d]pyrimidin-4-yl)-1H-pyrazol-1-yl]cyclobutyl}acetonitrile (10.0 mg, 0.018 mmol, Peak 1 from Intermediate Example A2 of US 2014/0005166, Step F) in methylene chloride (0.30 mL), and the mesylate formation was stirred for 30 minutes. The solvent was removed in vacuo and the residue was redissolved in a mixture of tetrahydrofuran (0.30 mL) and methanol (0.10 mL) and (2S)-2-aminopropan-1-ol (20. µL, 0.27 mmol, Acros) was added. The reaction mixture was stirred at 40 °C overnight. Solvent was removed *in vacuo* and the crude product was deprotected by stirring with 1:1 TFA:DCM for one hour, then concentrated and stirred with ethylenediamine (0.10 mL) in methanol (1.0 mL) until the deprotection was complete as determined by LCMS. The product was purified using preparative HPLC-MS (C18 eluting with a gradient of MeCN/H₂O containing 0.15% NH₄OH). The eluent was frozen and lyophilized to afford the product as the free base (6.0 mg, 54%). ¹H NMR (400 MHz, CD₃OD) δ 8.74 (s, 1H), 8.67 (s, 1H), 8.40 (s, 1H), 7.51 (d, *J* = 3.6 Hz, 1H), 7.37 (s, 1H), 7.00 - 6.97 (m, 2H), 5.23 - 5.00 (m, 1H), 3.90 (d, *J* = 14.8 Hz, 1H), 3.81 (d, *J* = 14.8 Hz, 1H), 3.50 (dd, *J* = 10.9, 4.9 Hz, 1H), 3.41 (dd, *J* = 10.9, 6.9 Hz, 1H), 3.31 (s, 2H), 3.16 - 3.05 (m, 2H), 2.95 (p, *J* = 7.5 Hz, 1H), 2.83 - 2.63 (m, 3H), 2.56 - 2.42 (m, 2H), 2.39 - 2.23 (m, 2H), 2.19 - 2.04 (m, 2H), 1.93 - 1.75 (m, 2H), 1.05 (d, *J* = 6.4 Hz, 3H). ¹⁹F NMR (376 MHz, CD₃OD) δ -70.30 (s). LCMS (M+H)⁺: 610.3

### Example 22. {trans-3-(4-{[4-({[(2R)-2-hydroxypropyl]amino}methyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

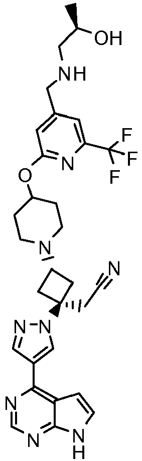

The procedure of Example 9 of US 2014/0005166 was followed, using (2R)-1-aminopropan-2-ol (12 µL, 0.15 mmol, Aldrich) in the displacement step, which was carried out at 50 °C for 2 hours. The product was obtained as the free base (8.7 mg, 46%). ¹H NMR (400 MHz, d₆-DMSO) δ 12.13 (s, 1H), 8.83 (s, 1H), 8.69 (s, 1H), 8.42 (s, 1H), 7.60 (d, *J* = 3.6 Hz, 1H), 7.42 (s, 1H), 7.08 (d, *J* = 3.6 Hz, 1H), 7.04 (s, 1H), 5.11 - 4.90 (m, 1H), 4.49 (d, *J* = 4.4 Hz, 1H), 3.76 (s, 2H), 3.67 (tt, *J* = 10.3, 5.6 Hz, 1H), 3.42 (s, 2H), 3.11 - 2.96 (m, 2H), 2.81 (p, *J* = 7.5 Hz, 1H), 2.74 - 2.56 (m, 2H), 2.46 - 2.25 (m, 4H), 2.24 - 2.09 (m, 2H), 2.09 - 1.90 (m, 2H), 1.81 - 1.51 (m, 2H), 1.03 (d, *J* = 6.2 Hz, 3H). ¹⁹F NMR (376 MHz, d₆-DMSO) δ -67.29 (s). LCMS (M+H)⁺: 610.3.

### Example 23. {trans-3-(4-{[4-({[(2S)-2-hydroxypropyl]amino}methyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

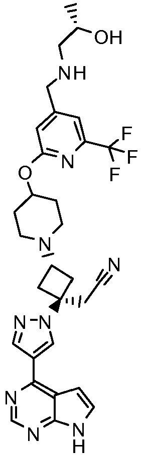

The procedure of Example 9 of US 2014/0005166 was followed, using (2S)-1-aminopropan-2-ol (12 µL, 0.15 mmol, Aldrich) in the displacement step, which was carried out at 50 °C for 2 hours (7.9 mg, 42%). ¹H NMR (400 MHz, d₆-DMSO) δ 12.13 (s, 1H), 8.83 (s, 1H), 8.69 (s, 1H), 8.42 (s, 1H), 7.60 (d, *J* = 3.6 Hz, 1H), 7.42 (s, 1H), 7.08 (d, *J* = 3.6 Hz, 1H), 7.04 (s, 1H), 5.27 - 4.71 (m, 1H), 4.49 (d, *J* = 4.4 Hz, 1H), 3.76 (s, 2H), 3.72 - 3.62 (m, 1H), 3.42 (s, 2H), 3.09 - 2.96 (m, 2H), 2.81 (p, *J* = 7.4 Hz, 1H), 2.72 - 2.55 (m, 2H), 2.43 - 2.25 (m, 4H), 2.25 - 2.08 (m, 2H), 2.08 - 1.96 (m, 2H), 1.78 - 1.57 (m, 2H), 1.03 (d, *J* = 6.2 Hz, 3H). ¹⁹F NMR (376 MHz, d₆-DMSO) δ -67.29 (s). LCMS (M+H)⁺: 610.3.

### Example 24. {trans-3-(4-{[4-(2-hydroxyethyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile

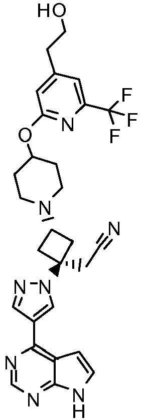

{trans-3-(4-{[4-(2-hydroxyethyl)-6-(trifluoromethyl)pyridin-2-yl]oxy}piperidin-1-yl)-1-[4-(7-{[2-(trimethylsilyl)ethoxy]methyl}-7H-pyrrolo[2,3-d]pyrimidin-4-yl)-1H-pyrazol-1-yl]cyclobutyl}acetonitrile (9.0 mg, 0.013 mmol, Peak 2 from Intermediate Example A4 of US 2014/0005166, Step 3) was deprotected and purified by stirred in a mixture of methylene chloride (0.50 mL) and trifluoroacetic acid (0.50 mL) for one hour. The solvents were removed *in vacuo* and the residue was stirred in methanol (0.1 mL) containing ethylenediamine (0.1 mL). Purification via preparative HPLC-MS (C18 eluting with a gradient of MeCN/H₂O containing 0.15% NH₄OH) afforded product as the free base (5.8 mg, 79%). ¹H NMR (300 MHz, d₆-DMSO) δ 12.12 (s, 1H), 8.83 (s, 1H), 8.69 (s, 1H), 8.42 (s, 1H), 7.60 (d, *J* = 3.6 Hz, 1H), 7.34 (s, 1H), 7.08 (d, *J* = 3.6 Hz, 1H), 6.95 (s, 1H), 4.99 (tt, *J* = 8.2, 4.1 Hz, 1H), 4.73 (t, *J* = 4.9 Hz, 1H), 3.66 (q, *J* = 5.9 Hz, 2H), 3.42 (s, 2H), 3.11 - 2.95 (m, 2H), 2.90 - 2.71 (m, 3H), 2.71 - 2.56 (m, 2H), 2.44 - 2.30 (m, 2H), 2.15 (t, *J* = 9.2 Hz, 2H), 2.09 - 1.82 (m, 2H), 1.83 - 1.58 (m, 2H). ¹⁹F NMR (282 MHz, d₆-DMSO) δ - 67.26 (s). LCMS (M+H)⁺: 567.2.

### Example A: In vitro JAK Kinase Assay

Compounds herein were tested for inhibitory activity of JAK targets according to the following *in vitro* assay described in Park et al., Analytical Biochemistry 1999, 269, 94-104. The catalytic domains of human JAK1 (a.a. 837-1142), JAK2 (a.a. 828-1132) and JAK3 (a.a. 781-1124) with an N-terminal His tag were expressed using baculovirus in insect cells and purified. The catalytic activity of JAK1, JAK2 or JAK3 was assayed by measuring the phosphorylation of a biotinylated peptide. The phosphorylated peptide was detected by homogenous time resolved fluorescence (HTRF). IC_{50S} of compounds were measured for each kinase in the 40 microL reactions that contain the enzyme, ATP and 500 nM peptide in 50 mM Tris (pH 7.8) buffer with 100 mM NaCl, 5 mM DTT, and 0.1 mg/mL (0.01%) BSA. For the 1 mM IC₅₀ measurements, ATP concentration in the reactions was 1 mM. Reactions were carried out at room temperature for 1 hour and then stopped with 20 µL 45 mM EDTA, 300 nM SA-APC, 6 nM Eu-Py20 in assay buffer (Perkin Elmer, Boston, MA). Binding to the Europium labeled antibody took place for 40 minutes and HTRF signal was measured on a Fusion plate reader (Perkin Elmer, Boston, MA). See Table 2 for data for compounds of the examples as tested by the assay of Example A at 1 mM ATP.

### Example B: Cellular Assays

Cancer cell lines dependent on cytokines and hence JAK/STAT signal transduction, for growth, can be plated at 6000 cells per well (96 well plate format) in RPMI 1640, 10% FBS, and 1 nG/mL of appropriate cytokine. Compounds can be added to the cells in DMSO/media (final concentration 0.2% DMSO) and incubated for 72 hours at 37 °C, 5% CO₂. The effect of compound on cell viability is assessed using the CellTiter-Glo Luminescent Cell Viability Assay (Promega) followed by TopCount (Perkin Elmer, Boston, MA) quantitation. Potential off-target effects of compounds are measured in parallel using a non-JAK driven cell line with the same assay readout. All experiments are typically performed in duplicate.

The above cell lines can also be used to examine the effects of compounds on phosphorylation of JAK kinases or potential downstream substrates such as STAT proteins, Akt, Shp2, or Erk. These experiments can be performed following an overnight cytokine starvation, followed by a brief preincubation with compound (2 hours or less) and cytokine stimulation of approximately 1 hour or less. Proteins are then extracted from cells and analyzed by techniques familiar to those schooled in the art including Western blotting or ELISAs using antibodies that can differentiate between phosphorylated and total protein. These experiments can utilize normal or cancer cells to investigate the activity of compounds on tumor cell survival biology or on mediators of inflammatory disease. For example, with regards to the latter, cytokines such as IL-6, IL-12, IL-23, or IFN can be used to stimulate JAK activation resulting in phosphorylation of STAT protein(s) and potentially in transcriptional profiles (assessed by array or qPCR technology) or production and/or secretion of proteins, such as IL-17. The ability of compounds to inhibit these cytokine mediated effects can be measured using techniques common to those schooled in the art.

Compounds herein can also be tested in cellular models designed to evaluate their potency and activity against mutant JAKs, for example, the JAK2V617F mutation found in myeloid proliferative disorders. These experiments often utilize cytokine dependent cells of hematological lineage (e.g. BaF/3) into which the wild-type or mutant JAK kinases are ectopically expressed (James, C., et al. Nature 434:1144-1148; Staerk, J., et al. JBC 280:41893-41899). Endpoints include the effects of compounds on cell survival, proliferation, and phosphorylated JAK, STAT, Akt, or Erk proteins.

Certain compounds herein can be evaluated for their activity inhibiting T-cell proliferation. Such as assay can be considered a second cytokine (*i.e.* JAK) driven proliferation assay and also a simplistic assay of immune suppression or inhibition of immune activation. The following is a brief outline of how such experiments can be performed. Peripheral blood mononuclear cells (PBMCs) are prepared from human whole blood samples using Ficoll Hypaque separation method and T-cells (fraction 2000) can be obtained from PBMCs by elutriation. Freshly isolated human T-cells can be maintained in culture medium (RPMI 1640 supplemented with 10% fetal bovine serum, 100 U/ml penicillin, 100 µg/ml streptomycin) at a density of 2 x 10⁶ cells/ml at 37 °C for up to 2 days. For IL-2 stimulated cell proliferation analysis, T-cells are first treated with Phytohemagglutinin (PHA) at a final concentration of 10 µg/mL for 72 hours. After washing once with PBS, 6000 cells/well are plated in 96-well plates and treated with compounds at different concentrations in the culture medium in the presence of 100 U/mL human IL-2 (ProSpec-Tany TechnoGene; Rehovot, Israel). The plates are incubated at 37 °C for 72h and the proliferation index is assessed using CellTiter-Glo Luminescent reagents following the manufactory suggested protocol (Promega; Madison, WI).

### Assay C. S100A9 Transgenic Mouse Model

It was previously shown that *S100A9* transgenic mice display bone marrow accumulation of MDSC accompanied by development of progressive multilineage cytopenias and cytological dysplasia similar to MDS. Further, early forced maturation of MDSC by either *all*-trans-retinoic acid treatment or active immunoreceptor tyrosine-based activation motif-bearing (ITAM-bearing) adapter protein (DAP12) interruption of CD33 signaling rescued the hematologic phenotype and mitigated the disease. This system can be useful to test the effects on JAK1 inhibition on MDS-like disease in a preclinical model. J. Clin. Invest., 123(11):4595-4611 (2013), Accordingly, a JAK1 selective inhibitor is dosed by oral gavage. The compound's ability to reduce the cytopenias and cytological dysplasia observed in the *S100A9* transgenic mice is monitored.

## Claims

1. A JAK1 selective inhibitor selected from:
3-[1-(6-chloropyridin-2-yl)pyrrolidin-3-yl]-3-[4-(7H-pyrrolo[2,3-d]pyrimidin-4-yl)-1H-pyrazol-1-yl]propanenitrile;
3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile;
4-[(4-{3-cyano-2-[4-(7H-pyrrolo[2,3-d]pyrimidin-4-yl)-1H-pyrazol-1-yl]propyl}piperazin-1-yl)carbonyl]-3-fluorobenzonitrile;
4-[(4-{3-cyano-2-[3-(7H-pyrrolo[2,3-d]pyrimidin-4-yl)-1H-pyrrol-1-yl]propyl}piperazin-1-yl)carbonyl]-3-fluorobenzonitrile;
{1-{1-[3-Fluoro-2-(trifluoromethyl)isonicotinoyl]piperidin-4-yl}-3-[4-(7H-pyrrolo[2,3-d]pyrimidin-4-yl)-1H-pyrazol-1-yl]azetidin-3-yl}acetonitrile;
4-{3-(cyanomethyl)-3-[4-(7H-pyrrolo[2,3-d]pyrimidin-4-yl)-1H-pyrazol-1-yl]azetidin-1-yl}-N-[4-fluoro-2-(trifluoromethyl)phenyl]piperidine-1-carboxamide;
[3-[4-(7H-pyrrolo[2,3-d]pyrimidin-4-yl)-1H-pyrazol-1-yl]-1-(1-{[2-(trifluoromethyl)pyrimidin-4-yl]carbonyl}piperidin-4-yl)azetidin-3-yl]acetonitrile;
*[trans-*1-[4-(7H-pyrrolo[2,3-d]pyrimidin-4-yl)-1H-pyrazol-1-yl]-3-(4-{[2-(trifluoromethyl)pyrimidin-4-yl]carbonyl}piperazin-1-yl)cyclobutyl]acetonitrile;
{*trans*-3-(4-{[4-[(3-hydroxyazetidin-1-yl)methyl]-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile;
{*trans*-3-(4-{[4-{[(2S)-2-(hydroxymethyl)pyrrolidin-1-yl]methyl}-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile;
{*trans*-3-(4-{[4-{[(2R)-2-(hydroxymethyl)pyrrolidin-1-yl]methyl}-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile;
4-(4-{3-[(dimethylamino)methyl]-5-fluorophenoxy}piperidin-1-yl)-3-[4-(7H-pyrrolo[2,3-d]pyrimidin-4-yl)-1H-pyrazol-1-yl]butanenitrile;
5-{3-(cyanomethyl)-3-[4-(7H-pyrrolo[2,3-d]pyrimidin-4-yl)-1H-pyrazol-1-yl]azetidin-1-yl}-N-isopropylpyrazine-2-carboxamide;
4-{3-(cyanomethyl)-3-[4-(7H-pyrrolo[2,3-d]pyrimidin-4-yl)-1H-pyrazol-1-yl]azetidin-1-yl}-2,5-difluoro-N-[(1S)-2,2,2-trifluoro-1-methylethyl]benzamide;
5-{3-(cyanomethyl)-3-[4-(1H-pyrrolo[2,3-b]pyridin-4-yl)-1H-pyrazol-1-yl]azetidin-1-yl}-N-isopropylpyrazine-2-carboxamide;
{1-(*cis*-4-{[6-(2-hydroxyethyl)-2-(trifluoromethyl)pyrimidin-4-yl]oxy}cyclohexyl)-3-[4-(7H-pyrrolo[2,3-d]pyrimidin-4-yl)-1H-pyrazol-1-yl]azetidin-3-yl} acetonitrile;
{1-(*cis*-4-{[4-[(ethylamino)methyl]-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl} acetonitrile;
{1-(*cis*-4-{[4-(1-hydroxy-1-methylethyl)-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl} acetonitrile;
{1-(*cis*-4-{[4-{[(3R)-3-hydroxypyrrolidin-1-yl]methyl}-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile;
{1-(*cis*-4-{[4-{[(3S)-3-hydroxypyrrolidin-1-yl]methyl}-6-(trifluoromethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitrile;
{*trans*-3-(4-{[4-({[(1S)-2-hydroxy-1-methylethyl]amino}methyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile;
{*trans*-3-(4-{[4-({[(2R)-2-hydroxypropyl]amino}methyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile;
{*trans*-3-(4-{[4-({[(2S)-2-hydroxypropyl]amino}methyl)-6-(trifluoromethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitrile; and
{*trans*-3-(4-{[4-(2-hydroxyethyl)-6-(trifluoromethyl)pyridin-2-yl]oxy} piperidin-1-yl)-1-[4-(7H-pyrrolo[2,3-d]pyrimidin-4-yl)-1H-pyrazol-1-yl]cyclobutyl}acetonitrile;
or a pharmaceutically acceptable salt thereof,
for use in a method of treating a myelodysplastic syndrome in a patient in need thereof, the method comprising administering to said patient a therapeutically effective amount of the JAK1 selective inhibitor, or a pharmaceutically acceptable salt thereof.

2. The JAK1 selective inhibitor, or a pharmaceutically acceptable salt thereof, for use according to claim 1, wherein the JAK1 selective inhibitor is selective for JAK1 over JAK2, JAK3, and TYK2.

3. The JAK1 selective inhibitor, or a pharmaceutically acceptable salt thereof, for use according to claim 1 or 2, wherein said myelodysplastic syndrome is refractory cytopenia with unilineage dysplasia (RCUD).

4. The JAK1 selective inhibitor, or a pharmaceutically acceptable salt thereof, for use according to claim 1 or 2, wherein said myelodysplastic syndrome is refractory anemia with ring sideroblasts (RARS).

5. The JAK1 selective inhibitor, or a pharmaceutically acceptable salt thereof, for use according to claim 1 or 2, wherein said myelodysplastic syndrome is refractory cytopenia with multilineage dysplasia.

6. The JAK1 selective inhibitor, or a pharmaceutically acceptable salt thereof, for use according to claim 1 or 2, wherein said myelodysplastic syndrome is refractory anemia with excess blasts-1 (RAEB-1).

7. The JAK1 selective inhibitor, or a pharmaceutically acceptable salt thereof, for use according to claim 1 or 2, wherein said myelodysplastic syndrome is refractory anemia with excess blasts-2 (RAEB-2).

8. The JAK1 selective inhibitor, or a pharmaceutically acceptable salt thereof, for use according to claim 1 or 2, wherein said myelodysplastic syndrome is myelodysplastic syndrome, unclassified (MDS-U).

9. The JAK1 selective inhibitor, or a pharmaceutically acceptable salt thereof, for use according to claim 1 or 2, wherein said myelodysplastic syndrome is myelodysplastic syndrome associated with isolated del(5q).

10. The JAK1 selective inhibitor, or a pharmaceutically acceptable salt thereof, for use according to claim 1 or 2, wherein said myelodysplastic syndrome is refractory to erythropoiesis-stimulating agents.

11. The JAK1 selective inhibitor, or a pharmaceutically acceptable salt thereof, for use according to any one of claims 1 to 10, wherein said patient is red blood cell transfusion dependent.

12. The JAK1 selective inhibitor, or a pharmaceutically acceptable salt thereof, for use according to any one of claims 1 to 11, wherein the method further comprises administering an additional therapeutic agent selected from an IMiD, an anti-IL-6 agent, an anti-TNF-a agent, a hypomethylating agent, or a biologic response modifier (BRM).

13. The JAK1 selective inhibitor, or a pharmaceutically acceptable salt thereof, for use according to claim 12, wherein said anti-TNF-a agent is selected from infliximab and etanercept.

14. The JAK1 selective inhibitor, or a pharmaceutically acceptable salt thereof, for use according to claim 12, wherein said hypomethylating agent is a DNA methyltransferase inhibitor.

15. The JAK1 selective inhibitor, or a pharmaceutically acceptable salt thereof, for use according to claim 14, wherein said DNA methyl transferase inhibitor is selected from 5 azacytidine and decitabine.

16. The JAK1 selective inhibitor, or a pharmaceutically acceptable salt thereof, for use according to claim 12, wherein said IMiD is selected from thalidomide, lenalidomide, pomalidomide, CC-11006, and CC-10015.

17. The JAK1 selective inhibitor, or a pharmaceutically acceptable salt thereof, for use according to any one of claims 1 to 11, wherein the method further comprises administering an additional therapeutic agent selected from anti-thymocyte globulin, recombinant human granulocyte colony-stimulating factor (G CSF), granulocyte-monocyte CSF (GM-CSF), a erythropoiesis-stimulating agent (ESA), and cyclosporine.

18. AJAK1 selective inhibitor which is {1-{1-[3-Fluoro-2-(trifluoromethyl)isonicotinoyl]piperidin-4-yl}-3-[4-(7H-pyrrolo[2,3-d]pyrimidin-4-yl)-1H-pyrazol-1-yl]azetidin-3-yl}acetonitrile, or a pharmaceutically acceptable salt thereof, for use in a method of treating a myelodysplastic syndrome in a patient in need thereof.

19. The JAK1 selective inhibitor, or a pharmaceutically acceptable salt thereof, for use according to claim 18, wherein said myelodysplastic syndrome is refractory cytopenia with unilineage dysplasia (RCUD).

20. The JAK1 selective inhibitor, or a pharmaceutically acceptable salt thereof, for use according to claim 18, wherein said myelodysplastic syndrome is refractory anemia with ring sideroblasts (RARS).

21. The JAK1 selective inhibitor, or a pharmaceutically acceptable salt thereof, for use according to claim 18, wherein said myelodysplastic syndrome is refractory cytopenia with multilineage dysplasia.

22. The JAK1 selective inhibitor, or a pharmaceutically acceptable salt thereof, for use according to claim 18, wherein said myelodysplastic syndrome is refractory anemia with excess blasts-1 (RAEB-1).

23. The JAK1 selective inhibitor, or a pharmaceutically acceptable salt thereof, for use according to claim 18, wherein said myelodysplastic syndrome is refractory anemia with excess blasts-2 (RAEB-2).

24. The JAK1 selective inhibitor, or a pharmaceutically acceptable salt thereof, for use according to claim 18, wherein said myelodysplastic syndrome is myelodysplastic syndrome, unclassified (MDS-U).

25. The JAK1 selective inhibitor, or a pharmaceutically acceptable salt thereof, for use according to claim 18, wherein said myelodysplastic syndrome is myelodysplastic syndrome associated with isolated del(5q).

26. The JAK1 selective inhibitor, or a pharmaceutically acceptable salt thereof, for use according to claim 18, wherein said myelodysplastic syndrome is refractory to erythropoiesis-stimulating agents.

27. The JAK1 selective inhibitor, or a pharmaceutically acceptable salt thereof, for use according to any one of claims 18 to 26, wherein said patient is red blood cell transfusion dependent.

28. The JAK1 selective inhibitor, or a pharmaceutically acceptable salt thereof, for use according to any one of claims 18 to 27, wherein the method further comprises administering an additional therapeutic agent selected from an IMiD, an anti-IL-6 agent, an anti-TNF-a agent, a hypomethylating agent, or a biologic response modifier (BRM).

29. The JAK1 selective inhibitor, or a pharmaceutically acceptable salt thereof, for use according to claim 28, wherein said anti-TNF-a agent is selected from infliximab and etanercept.

30. The JAK1 selective inhibitor, or a pharmaceutically acceptable salt thereof, for use according to claim 28, wherein said hypomethylating agent is a DNA methyltransferase inhibitor.

31. The JAK1 selective inhibitor, or a pharmaceutically acceptable salt thereof, for use according to claim 30, wherein said DNA methyl transferase inhibitor is selected from 5 azacytidine and decitabine.

32. The JAK1 selective inhibitor, or a pharmaceutically acceptable salt thereof, for use according to claim 28, wherein said IMiD is selected from thalidomide, lenalidomide, pomalidomide, CC-11006, and CC-10015.

33. The JAK1 selective inhibitor, or a pharmaceutically acceptable salt thereof, for use according to any one of claims 18 to 27, wherein the method further comprises administering an additional therapeutic agent selected from anti-thymocyte globulin, recombinant human granulocyte colony-stimulating factor (G CSF), granulocyte-monocyte CSF (GM-CSF), a erythropoiesis-stimulating agent (ESA), and cyclosporine.

## Patentansprüche

1. JAK1-selektiver Inhibitor, ausgewählt aus:
3-[1-(6-Chlorpyridin-2-yl)pyrrolidin-3-yl]-3-[4-(7H-pyrrolo[2,3-d]pyrimidin-4-yl)-1H-pyrazol-1-yl]propannitril;
3-(1-[1,3]Oxazolo[5,4-b]pyridin-2-ylpyrrolidin-3-yl)-3-[4-(7H-pyrrolo[2,3-d]pyrimidin-4-yl)-1H-pyrazol-1-yl]propannitril;
4-[(4-{3-Cyano-2-[4-(7H-pyrrolo[2,3-d]pyrimidin-4-yl)-1H-pyrazol-1-yl]propyl}piperazin-1-yl)-carbonyl]-3-fluorbenzonitril;
4-[(4-{3-Cyano-2-[3-(7H-pyrrolo[2,3-d]pyrimidin-4-yl)-1H-pyrrol-1-yl]propyl}piperazin-1-yl)-carbonyl]-3-fluorbenzonitril;
{1-{1-[3-Fluor-2-(trifluormethyl)isonicotinoyl]-piperidin-4-yl}-3-[4-(7H-pyrrolo[2,3-d]pyrimidin-4-yl)-1H-pyrazol-1-yl]azetidin-3-yl}acetonitril;
4-{3-(Cyanomethyl)-3-[4-(7H-pyrrolo[2,3-d]-pyrimidin-4-yl)-1H-pyrazol-1-yl]azetidin-1-yl}-N-[4-fluor-2-(trifluormethyl)phenyl]piperidin-1-carboxamid;
[3-[4-(7H-Pyrrolo[2,3-d]pyrimidin-4-yl)-1H-pyrazol-1-yl]-1-(1-{[2-(trifluormethyl)pyrimidin-4-yl]carbonyl}piperidin-4-yl)azetidin-3-yl]acetonitril;
[*trans*-1-[4-(7H-Pyrrolo[2,3-d]pyrimidin-4-yl)-1H-pyrazol-1-yl]-3-(4-{[2-(trifluormethyl)pyrimidin-4-yl]carbonyl}piperazin-1-yl)cyclobutyl]acetonitril,
{*trans*-3-(4-{[4-[(3-Hydroxyazetidin-1-yl)methyl]-6-(trifluormethyl)pyridin-2-yl]oxy}piperidin-1-yl)-1-[4-(7H-pyrrolo[2,3-d]pyrimidin-4-yl)-1H-pyrazol-1-yl]cyclobutyl}acetonitril;
{*trans*-3-(4-{[4-{[(2S)-2-(Hydroxymethyl)-pyrrolidin-1-yl]methyl}-6-(trifluormethyl)pyridin-2-yl]oxy}piperidin-1-yl)-1-[4-(7H-pyrrolo[2,3-d]-pyrimidin-4-yl)-1H-pyrazol-1-yl]cyclobutyl}acetonitril;
{*trans-*3-(4-{[4-{[(2R)-2-(Hydroxymethyl)-pyrrolidin-1-yl]methyl}-6-(trifluormethyl)pyridin-2-yl]oxy}piperidin-1-yl)-1-[4-(7H-pyrrolo[2,3-d]-pyrimidin-4-yl)-1H-pyrazol-1-yl]cyclobutyl}acetonitril;
4-(4-{3-[(Dimethylamino)methyl]-5-fluorphenoxy}-piperidin-1-yl)-3-[4-(7H-pyrrolo[2,3-d]pyrimidin-4-yl)-1H-pyrazol-1-yl]butannitril;
5-{3-(Cyanomethyl)-3-[4-(7H-pyrrolo[2,3-d]-pyrimidin-4-yl)-1H-pyrazol-1-yl]azetidin-1-yl}-N-isopropylpyrazin-2-carboxamid;
4-{3-(Cyanomethyl)-3-[4-(7H-pyrrolo[2,3-d]-pyrimidin-4-yl)-1H-pyrazol-1-yl]azetidin-1-yl}-2,5-difluor-N-[(1S)-2,2,2-trifluor-1-methylethyl]-benzamid;
5-{3-(Cyanomethyl)-3-[4-(1H-pyrrolo[2,3-b]pyridin-4-yl)-1H-pyrazol-1-yl]azetidin-1-yl}-N-isopropyl-pyrazin-2-carboxamid;
{1-(*cis*-4-{[6-(2-Hydroxyethyl)-2-(trifluormethyl)-pyrimidin-4-yl]oxy}cyclohexyl)-3-[4-(7H-pyrrolo-[2,3-d]pyrimidin-4-yl)-1H-pyrazol-1-yl]azetidin-3-yl}acetonitril;
{1-(*cis*-4-{[4-[(Ethylamino)methyl]-6-(trifluormethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitril;
{1-(*cis*-4-{[4-(1-Hydroxy-1-methylethyl)-6-(trifluormethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitril;
{1-(*cis*-4-{[4-{[(3R)-3-Hydroxypyrrolidin-1-yl]-methyl}-6-(trifluormethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitril;
{1-(*cis*-4-{[4-{[(3S)-3-Hydroxypyrrolidin-1-yl]-methyl}-6-(trifluormethyl)pyridin-2-yl]oxy}cyclohexyl)-3-[4-(7H-pyrrolo[2,3-d]pyrimidin-4-yl)-1H-pyrazol-1-yl]azetidin-3-yl}acetonitril;
{*trans*-3-(4-{[4-({[(1S)-2-Hydroxy-1-methylethyl]-amino}methyl)-6-(trifluormethyl)pyridin-2-yl]oxy}-piperidin-1-yl)-1-[4-(7H-pyrrolo[2,3-d]pyrimidin-4-yl)-1H-pyrazol-1-yl]cyclobutyl}acetonitril;
{*trans*-3-(4-{[4-({[(2R)-2-Hydroxypropyl]amino}-methyl)-6-(trifluormethyl)pyridin-2-yl]oxy}-piperidin-1-yl)-1-[4-(7H-pyrrolo[2,3-d]pyrimidin-4-yl)-1H-pyrazol-1-yl]cyclobutyl}acetonitril;
{*trans*-3-(4-{[4-({[(2S)-2-Hydroxypropyl]amino}-methyl)-6-(trifluormethyl)pyridin-2-yl]oxy}-piperidin-1-yl)-1-[4-(7H-pyrrolo[2,3-d]pyrimidin-4-yl)-1H-pyrazol-1-yl]cyclobutyl}acetonitril und {*trans*-3-(4-{[4-(2-Hydroxyethyl)-6-(trifluormethyl)pyridin-2-yl]oxy}piperidin-1-yl)-1-[4-(7H-pyrrolo[2,3-d]pyrimidin-4-yl)-1H-pyrazol-1-yl]-cyclobutyl}acetonitril;
oder ein pharmazeutisch unbedenkliches Salz davon zur Verwendung bei einer Methode zur Behandlung eines myelodysplastischen Syndroms bei einem Patienten, der einer derartigen Behandlung bedarf, wobei die Methode die Verabreichung einer therapeutisch wirksamen Menge des JAK1-selektiven Inhibitors oder eines pharmazeutisch unbedenklichen Salzes davon an einen Patienten umfasst.

2. JAK1-selektiver Inhibitor oder pharmazeutisch unbedenkliches Salz davon zur Verwendung nach Anspruch 1, wobei der JAK1-selektive Inhibitor gegenüber JAK2, JAK3 und TYK2 für JAK1 selektiv ist.

3. JAK1-selektiver Inhibitor oder pharmazeutisch unbedenkliches Salz davon zur Verwendung nach Anspruch 1 oder 2, wobei es sich bei dem myelodysplastischen Syndrom um refraktäre Zytopenie mit unilineärer Dysplasie (RCUD) handelt.

4. JAK1-selektiver Inhibitor oder pharmazeutisch unbedenkliches Salz davon zur Verwendung nach Anspruch 1 oder 2, wobei es sich bei dem myelodysplastischen Syndrom um refraktäre Anämie mit Ringsideroblasten (RARS) handelt.

5. JAK1-selektiver Inhibitor oder pharmazeutisch unbedenkliches Salz davon zur Verwendung nach Anspruch 1 oder 2, wobei es sich bei dem myelodysplastischen Syndrom um refraktäre Zytopenie mit multilineärer Dysplasie handelt.

6. JAK1-selektiver Inhibitor oder pharmazeutisch unbedenkliches Salz davon zur Verwendung nach Anspruch 1 oder 2, wobei es sich bei dem myelodysplastischen Syndrom um refraktäre Anämie mit Blastenüberschuss 1 (RAEB-1) handelt.

7. JAK1-selektiver Inhibitor oder pharmazeutisch unbedenkliches Salz davon zur Verwendung nach Anspruch 1 oder 2, wobei es sich bei dem myelodysplastischen Syndrom um refraktäre Anämie mit Blastenüberschuss 2 (RAEB-2) handelt.

8. JAK1-selektiver Inhibitor oder pharmazeutisch unbedenkliches Salz davon zur Verwendung nach Anspruch 1 oder 2, wobei es sich bei dem myelodysplastischen Syndrom um unklassifiziertes myelodysplastisches Syndrom (MDS-U) handelt.

9. JAK1-selektiver Inhibitor oder pharmazeutisch unbedenkliches Salz davon zur Verwendung nach Anspruch 1 oder 2, wobei es sich bei dem myelodysplastischen Syndrom um myelodysplastisches Syndrom mit isolierter del(5q) handelt.

10. JAK1-selektiver Inhibitor oder pharmazeutisch unbedenkliches Salz davon zur Verwendung nach Anspruch 1 oder 2, wobei das myelodysplastische Syndrom gegenüber die Erythropoese stimulierenden Mitteln refraktär ist.

11. JAK1-selektiver Inhibitor oder pharmazeutisch unbedenkliches Salz davon zur Verwendung nach einem der Ansprüche 1 bis 10, wobei der Patient erythrozytentransfusionsabhängig ist.

12. JAK1-selektiver Inhibitor oder pharmazeutisch unbedenkliches Salz davon zur Verwendung nach einem der Ansprüche 1 bis 11, wobei die Methode ferner die Verabreichung eines zusätzlichen Therapeutikums, das aus einem ImiD, einem Anti-IL-6-Mittel, einem Anti-TNF-α-Mittel, einem Hypomethylierungsmittel und einem Biological Response Modifier (BRM) ausgewählt ist, umfasst.

13. JAK1-selektiver Inhibitor oder pharmazeutisch unbedenkliches Salz davon zur Verwendung nach Anspruch 12, wobei das Anti-TNF-α-Mittel aus Infliximab und Etanercept ausgewählt ist.

14. JAK1-selektiver Inhibitor oder pharmazeutisch unbedenkliches Salz davon zur Verwendung nach Anspruch 12, wobei es sich bei dem Hypomethylierungsmittel um einen DNA-Methyltransferase-Inhibitor handelt.

15. JAK1-selektiver Inhibitor oder pharmazeutisch unbedenkliches Salz davon zur Verwendung nach Anspruch 14, wobei der DNA-Methyltransferase-Inhibitor aus 5-Azacytidin und Decitabin ausgewählt ist.

16. JAK1-selektiver Inhibitor oder pharmazeutisch unbedenkliches Salz davon zur Verwendung nach Anspruch 12, wobei das IMiD aus Thalidomid, Lenalidomid, Pomalidomid, CC-11006 und CC-10015 ausgewählt ist.

17. JAK1-selektiver Inhibitor oder pharmazeutisch unbedenkliches Salz davon zur Verwendung nach einem der Ansprüche 1 bis 11, wobei die Methode ferner die Verabreichung eines zusätzlichen Therapeutikums, das aus Antithymozytenglobulin, rekombinantem humanem Granulozyten-Koloniestimulierenden Faktor (G CSF), Granulozyten-Monozyten-CSF (GM-CSF), einem die Erythropoese stimulierenden Mittel (ESA) und Cyclosporin ausgewählt ist, umfasst.

18. JAK1-selektiver Inhibitor, bei dem es sich um {1-{1-[3-Fluor-2-(trifluormethyl)isonicotinoyl]-piperidin-4-yl}-3-[4-(7H-pyrrolo[2,3-d]pyrimidin-4-yl)-1H-pyrazol-1-yl]azetidin-3-yl}acetonitril handelt, oder ein pharmazeutisch unbedenkliches Salz davon zur Verwendung bei einer Methode zur Behandlung eines myelodysplastischen Syndroms bei einem Patienten, der einer derartigen Behandlung bedarf.

19. JAK1-selektiver Inhibitor oder pharmazeutisch unbedenkliches Salz davon zur Verwendung nach Anspruch 18, wobei es sich bei dem myelodysplastischen Syndrom um refraktäre Zytopenie mit unilineärer Dysplasie (RCUD) handelt.

20. JAK1-selektiver Inhibitor oder pharmazeutisch unbedenkliches Salz davon zur Verwendung nach Anspruch 18, wobei es sich bei dem myelodysplastischen Syndrom um refraktäre Anämie mit Ringsideroblasten (RARS) handelt.

21. JAK1-selektiver Inhibitor oder pharmazeutisch unbedenkliches Salz davon zur Verwendung nach Anspruch 18, wobei es sich bei dem myelodysplastischen Syndrom um refraktäre Zytopenie mit multilineärer Dysplasie handelt.

22. JAK1-selektiver Inhibitor oder pharmazeutisch unbedenkliches Salz davon zur Verwendung nach Anspruch 18, wobei es sich bei dem myelodysplastischen Syndrom um refraktäre Anämie mit Blastenüberschuss 1 (RAEB-1) handelt.

23. JAK1-selektiver Inhibitor oder pharmazeutisch unbedenkliches Salz davon zur Verwendung nach Anspruch 18, wobei es sich bei dem myelodysplastischen Syndrom um refraktäre Anämie mit Blastenüberschuss 2 (RAEB-2) handelt.

24. JAK1-selektiver Inhibitor oder pharmazeutisch unbedenkliches Salz davon zur Verwendung nach Anspruch 18, wobei es sich bei dem myelodysplastischen Syndrom um unklassifiziertes myelodysplastisches Syndrom (MDS-U) handelt.

25. JAK1-selektiver Inhibitor oder pharmazeutisch unbedenkliches Salz davon zur Verwendung nach Anspruch 18, wobei es sich bei dem myelodysplastischen Syndrom um myelodysplastisches Syndrom mit isolierter del(5q) handelt.

26. JAK1-selektiver Inhibitor oder pharmazeutisch unbedenkliches Salz davon zur Verwendung nach Anspruch 18, wobei das myelodysplastische Syndrom gegenüber die Erythropoese stimulierenden Mitteln refraktär ist.

27. JAK1-selektiver Inhibitor oder pharmazeutisch unbedenkliches Salz davon zur Verwendung nach einem der Ansprüche 18 bis 26, wobei der Patient erythrozytentransfusionsabhängig ist.

28. JAK1-selektiver Inhibitor oder pharmazeutisch unbedenkliches Salz davon zur Verwendung nach einem der Ansprüche 18 bis 27, wobei die Methode ferner die Verabreichung eines zusätzlichen Therapeutikums, das aus einem ImiD, einem Anti-IL-6-Mittel, einem Anti-TNF-α-Mittel, einem Hypomethylierungsmittel und einem Biological Response Modifier (BRM) ausgewählt ist, umfasst.

29. JAK1-selektiver Inhibitor oder pharmazeutisch unbedenkliches Salz davon zur Verwendung nach Anspruch 28, wobei das Anti-TNF-α-Mittel aus Infliximab und Etanercept ausgewählt ist.

30. JAK1-selektiver Inhibitor oder pharmazeutisch unbedenkliches Salz davon zur Verwendung nach Anspruch 28, wobei es sich bei dem Hypomethylierungsmittel um einen DNA-Methyltransferase-Inhibitor handelt.

31. JAK1-selektiver Inhibitor oder pharmazeutisch unbedenkliches Salz davon zur Verwendung nach Anspruch 30, wobei der DNA-Methyltransferase-Inhibitor aus 5-Azacytidin und Decitabin ausgewählt ist.

32. JAK1-selektiver Inhibitor oder pharmazeutisch unbedenkliches Salz davon zur Verwendung nach Anspruch 28, wobei das IMiD aus Thalidomid, Lenalidomid, Pomalidomid, CC-11006 und CC-10015 ausgewählt ist.

33. JAK1-selektiver Inhibitor oder pharmazeutisch unbedenkliches Salz davon zur Verwendung nach einem der Ansprüche 18 bis 27, wobei die Methode ferner die Verabreichung eines zusätzlichen Therapeutikums, das aus Antithymozytenglobulin, rekombinantem humanem Granulozyten-Koloniestimulierenden Faktor (G CSF), Granulozyten-Monozyten-CSF (GM-CSF), einem die Erythropoese stimulierenden Mittel (ESA) und Cyclosporin ausgewählt ist, umfasst.

## Revendications

1. Inhibiteur sélectif de JAK1 choisi parmi :
3-[1-(6-chloropyridin-2-yl)pyrrolidin-3-yl]-3-[4-(7H-pyrrolo[2,3-d]pyrimidin-4-yl)-1H-pyrazol-1-yl]propanenitrile ;
3-(1-[1,3]oxazolo[5,4-b]pyridin-2-ylpyrrolidin-3-yl)-3-[4-(7H-pyrrolo[2,3-d]pyrimidin-4-yl)-1H-pyrazol-1-yl]propanenitrile ;
4-[(4-{3-cyano-2-[4-(7H-pyrrolo[2,3-d]pyrimidin-4-yl)-1H-pyrazol-1-yl]propyl}pipérazin-1-yl)carbonyl]-3-fluorobenzonitrile ;
4-[(4-{3-cyano-2-[3-(7H-pyrrolo[2,3-d]pyrimidin-4-yl)-1H-pyrrol-1-yl]propyl}pipérazin-1-yl)carbonyl]-3-fluorobenzonitrile ;
{1-{1-[3-Fluoro-2-(trifluorométhyl)isonicotinoyl]pipéridin-4-yl}-3-[4-(7H-pyrrolo[2,3-d]pyrimidin-4-yl)-1H-pyrazol-1-yl]azétidin-3-yl}acétonitrile ;
4-{3-(cyanométhyl)-3-[4-(7H-pyrrolo[2,3-d]pyrimidin-4-yl)-1H-pyrazol-1-yl]azétidin-1-yl}-N-[4-fluoro-2-(trifluorométhyl)phényl]pipéridine-1-carboxamide ;
[3-[4-(7H-pyrrolo[2,3-d]pyrimidin-4-yl)-1H-pyrazol-1-yl]-1-(1-{[2-(trifluorométhyl)pyrimidin-4-yl]carbonyl}pipéridin-4-yl)azétidin-3-yl]acétonitrile ;
[*trans*-1-[4-(7H-pyrrolo[2,3-d]pyrimidin-4-yl)-1H-pyrazol-1-yl]-3-(4-{[2-(trifluorométhyl)pyrimidin-4-yl]carbonyl}pipérazin-1-yl)cyclobutyl]acétonitrile ;
{*trans*-3-(4-{[4-[(3-hydroxyazétidin-1-yl)méthyl]-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ;
{*trans*-3-(4-{[4-{[(2S)-2-(hydroxyméthyl)pyrrolidin-1-yl]méthyl}-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ;
{*trans*-3-(4-{[4-{[(2R)-2-(hydroxyméthyl)pyrrolidin-1-yl]méthyl}-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ;
4-(4-{3-[(diméthylamino)méthyl]-5-fluorophenoxy}pipéridin-1-yl)-3-[4-(7H-pyrrolo[2,3-d]pyrimidin-4-yl)-1H-pyrazol-1-yl]butanenitrile ;
5-{3-(cyanométhyl)-3-[4-(7H-pyrrolo[2,3-d]pyrimidin-4-yl)-1H-pyrazol-1-yl]azétidin-1-yl}-N-isopropylpyrazine-2-carboxamide ;
4-{3-(cyanométhyl)-3-[4-(7H-pyrrolo[2,3-d]pyrimidin-4-yl)-1H-pyrazol-1-yl]azétidin-1-yl}-2,5-difluoro-N-[(1S)-2,2,2-trifluoro-1-méthyléthyl]benzamide ;
5-{3-(cyanométhyl)-3-[4-(1H-pyrrolo[2,3-b]pyridin-4-yl)-1H-pyrazol-1-yl]azétidin-1-yl}-N-isopropylpyrazine-2-carboxamide ;
{1-(*cis*-4-{[6-(2-hydroxyéthyl)-2-(trifluorométhyl)pyrimidin-4-yl]oxy}cyclohexyl)-3-[4-(7H-pyrrolo[2,3-d]pyrimidin-4-yl)-1H-pyrazol-1-yl]azétidin-3-yl}acétonitrile ;
{1-(*cis*-4-{[4-[(éthylamino)méthyl]-6-(trifluorométhyl)pyridin-2-yl]oxy}cyclohexyl)-3-[4-(7H-pyrrolo[2,3-d]pyrimidin-4-yl)-1H-pyrazol-1-yl]azétidin-3-yl}acétonitrile ;
{1-(*cis*-4-{[4-(1-hydroxy-1-méthyléthyl)-6-(trifluorométhyl)pyridin-2-yl]oxy}cyclohexyl)-3-[4-(7H-pyrrolo[2,3-d]pyrimidin-4-yl)-1H-pyrazol-1-yl]azétidin-3-yl}acétonitrile ;
{1-(*cis*-4-{[4-{[(3R)-3-hydroxypyrrolidin-1-yl]méthyl}-6-(trifluorométhyl)pyridin-2-yl]oxy}cyclohexyl)-3-[4-(7H-pyrrolo[2,3-d]pyrimidin-4-yl)-1H-pyrazol-1-yl]azétidin-3-yl}acétonitrile ;
{1-(*cis*-4-{[4-{[(3S)-3-hydroxypyrrolidin-1-yl]méthyl}-6-(trifluorométhyl)pyridin-2-yl]oxy}cyclohexyl)-3-[4-(7H-pyrrolo[2,3-d]pyrimidin-4-yl)-1H-pyrazol-1-yl]azétidin-3-yl}acétonitrile ;
{*trans-*3-(4-{[4-({[(1S)-2-hydroxy-1-méthyléthyl]amino}méthyl)-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ;
{*trans*-3-(4-{[4-({[(2R)-2-hydroxypropyl]amino}méthyl)-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ;
{*trans*-3-(4-{[4-({[(2S)-2-hydroxypropyl]amino}méthyl)-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ; et
{*trans*-3-(4-{[4-(2-hydroxyéthyl)-6-(trifluorométhyl)pyridin-2-yl]oxy}pipéridin-1-yl)-1-[4-(7H-pyrrolo[2,3-d]pyrimidin-4-yl)-1H-pyrazol-1-yl]cyclobutyl}acétonitrile ;
ou sel pharmaceutiquement acceptable de celui-ci,
pour utilisation dans un procédé de traitement d'un syndrome myélodysplasique chez un patient en ayant besoin, le procédé comprenant l'administration audit patient d'une quantité thérapeutiquement efficace de l'inhibiteur sélectif de JAK1, ou un sel pharmaceutiquement acceptable de celui-ci.

2. Inhibiteur sélectif de JAK1, ou sel pharmaceutiquement acceptable de celui-ci, pour utilisation selon la revendication 1, l'inhibiteur sélectif de JAK1 étant sélectif pour JAK1 par rapport à JAK2, JAK3 et TYK2.

3. Inhibiteur sélectif de JAK1, ou sel pharmaceutiquement acceptable de celui-ci, pour utilisation selon la revendication 1 ou 2, ledit syndrome myélodysplasique étant la cytopénie réfractaire avec dysplasie unilignée (RCUD).

4. Inhibiteur sélectif de JAK1, ou sel pharmaceutiquement acceptable de celui-ci, pour utilisation selon la revendication 1 ou 2, ledit syndrome myélodysplasique étant l'anémie réfractaire avec sidéroblastes en couronne (RARS).

5. Inhibiteur sélectif de JAK1, ou sel pharmaceutiquement acceptable de celui-ci, pour utilisation selon la revendication 1 ou 2, ledit syndrome myélodysplasique étant la cytopénie réfractaire avec dysplasie multilignée.

6. Inhibiteur sélectif de JAK1, ou sel pharmaceutiquement acceptable de celui-ci, pour utilisation selon la revendication 1 ou 2, ledit syndrome myélodysplasique étant l'anémie réfractaire avec excès de blastes 1 (AREB-1).

7. Inhibiteur sélectif de JAK1, ou sel pharmaceutiquement acceptable de celui-ci, pour utilisation selon la revendication 1 ou 2, ledit syndrome myélodysplasique étant l'anémie réfractaire avec excès de blastes 2 (AREB-2).

8. Inhibiteur sélectif de JAK1, ou sel pharmaceutiquement acceptable de celui-ci, pour utilisation selon la revendication 1 ou 2, ledit syndrome myélodysplasique étant un syndrome myélodysplasique non classé (MDS-U).

9. Inhibiteur sélectif de JAK1, ou sel pharmaceutiquement acceptable de celui-ci, pour utilisation selon la revendication 1 ou 2, ledit syndrome myélodysplasique étant un syndrome myélodysplasique associé à del(5q) isolé.

10. Inhibiteur sélectif de JAK1, ou sel pharmaceutiquement acceptable de celui-ci, pour utilisation selon la revendication 1 ou 2, ledit syndrome myélodysplasique étant réfractaire aux agents stimulant l'érythropoïèse.

11. Inhibiteur sélectif de JAK1, ou sel pharmaceutiquement acceptable de celui-ci, pour utilisation selon l'une quelconque des revendications 1 à 10, ledit patient étant dépendant d'une transfusion de globules rouges.

12. Inhibiteur sélectif de JAK1, ou sel pharmaceutiquement acceptable de celui-ci, pour utilisation selon l'une quelconque des revendications 1 à 11, le procédé comprenant en outre l'administration d'un agent thérapeutique supplémentaire choisi parmi un IMiD, un agent anti-IL-6, un agent anti-TNF-α, un agent d'hypométhylation, ou un modificateur de réponse biologique (MRB).

13. Inhibiteur sélectif de JAK1, ou sel pharmaceutiquement acceptable de celui-ci, pour utilisation selon la revendication 12, ledit agent anti-TNF-α étant choisi parmi l'infliximab et l'étanercept.

14. Inhibiteur sélectif de JAK1, ou sel pharmaceutiquement acceptable de celui-ci, pour utilisation selon la revendication 12, ledit agent d'hypométhylation étant un inhibiteur d'ADN méthyl-transférase.

15. Inhibiteur sélectif de JAK1, ou sel pharmaceutiquement acceptable de celui-ci, pour utilisation selon la revendication 14, ledit inhibiteur d'ADN méthyl-transférase étant choisi parmi la 5-azacytidine et la décitabine.

16. Inhibiteur sélectif de JAK1, ou sel pharmaceutiquement acceptable de celui-ci, pour utilisation selon la revendication 12, ledit IMiD étant choisi parmi la thalidomide, le lénalidomide, le pomalidomide, CC-11006 et CC-10015.

17. Inhibiteur sélectif de JAK1, ou sel pharmaceutiquement acceptable de celui-ci, pour utilisation selon l'une quelconque des revendications 1 à 11, le procédé comprenant en outre l'administration d'un agent thérapeutique supplémentaire choisi parmi une globuline anti-thymocyte, un facteur stimulant les colonies de granulocytes humains (G-CSF) recombinant, le facteur stimulant les colonies de granulocytes-monocytes (GM-CSF), un agent stimulant l'érythropoïèse (ESA) et une cyclosporine.

18. Inhibiteur sélectif de JAK1 qui est le {1-{1-[3-fluoro-2-(trifluorométhyl)isonicotinoyl]pipéridin-4-yl}-3-[4-(7H-pyrrolo[2,3-d]pyrimidin-4-yl)-1H-pyrazol-1-yl]azétidin-3-yl}acétonitrile, ou sel pharmaceutiquement acceptable de celui-ci, pour utilisation dans un procédé de traitement d'un syndrome myélodysplasique chez un patient en ayant besoin.

19. Inhibiteur sélectif de JAK1, ou sel pharmaceutiquement acceptable de celui-ci, pour utilisation selon la revendication 18, ledit syndrome myélodysplasique étant la cytopénie réfractaire avec dysplasie unilignée (RCUD).

20. Inhibiteur sélectif de JAK1, ou sel pharmaceutiquement acceptable de celui-ci, pour utilisation selon la revendication 18, ledit syndrome myélodysplasique étant l'anémie réfractaire avec sidéroblastes en couronne (RARS).

21. Inhibiteur sélectif de JAK1, ou sel pharmaceutiquement acceptable de celui-ci, pour utilisation selon la revendication 18, ledit syndrome myélodysplasique étant la cytopénie réfractaire avec dysplasie multilignée.

22. Inhibiteur sélectif de JAK1, ou sel pharmaceutiquement acceptable de celui-ci, pour utilisation selon la revendication 18, ledit syndrome myélodysplasique étant l'anémie réfractaire avec excès de blastes 1 (AREB-1).

23. Inhibiteur sélectif de JAK1, ou sel pharmaceutiquement acceptable de celui-ci, pour utilisation selon la revendication 18, ledit syndrome myélodysplasique étant l'anémie réfractaire avec excès de blastes 2 (AREB-2).

24. Inhibiteur sélectif de JAK1, ou sel pharmaceutiquement acceptable de celui-ci, pour utilisation selon la revendication 18, ledit syndrome myélodysplasique étant un syndrome myélodysplasique non classé (MDS-U).

25. Inhibiteur sélectif de JAK1, ou sel pharmaceutiquement acceptable de celui-ci, pour utilisation selon la revendication 18, ledit syndrome myélodysplasique étant un syndrome myélodysplasique associé à del(5q) isolé.

26. Inhibiteur sélectif de JAK1, ou sel pharmaceutiquement acceptable de celui-ci, pour utilisation selon la revendication 18, ledit syndrome myélodysplasique étant réfractaire aux agents stimulant l'érythropoïèse.

27. Inhibiteur sélectif de JAK1, ou sel pharmaceutiquement acceptable de celui-ci, pour utilisation selon l'une quelconque des revendications 18 à 26, ledit patient étant dépendant d'une transfusion de globules rouges.

28. Inhibiteur sélectif de JAK1, ou sel pharmaceutiquement acceptable de celui-ci, pour utilisation selon l'une quelconque des revendications 18 à 27, le procédé comprenant en outre l'administration d'un agent thérapeutique supplémentaire choisi parmi un IMiD, un agent anti-IL-6, un agent anti-TNF-α, un agent d'hypométhylation, ou un modificateur de réponse biologique (MRB).

29. Inhibiteur sélectif de JAK1, ou sel pharmaceutiquement acceptable de celui-ci, pour utilisation selon la revendication 28, ledit agent anti-TNF-α étant choisi parmi l'infliximab et l'étanercept.

30. Inhibiteur sélectif de JAK1, ou sel pharmaceutiquement acceptable de celui-ci, pour utilisation selon la revendication 28, ledit agent d'hypométhylation étant un inhibiteur d'ADN méthyl-transférase.

31. Inhibiteur sélectif de JAK1, ou sel pharmaceutiquement acceptable de celui-ci, pour utilisation selon la revendication 30, ledit inhibiteur d'ADN méthyl-transférase étant choisi parmi la 5-azacytidine et la décitabine.

32. Inhibiteur sélectif de JAK1, ou sel pharmaceutiquement acceptable de celui-ci, pour utilisation selon la revendication 28, ledit IMiD étant choisi parmi la thalidomide, le lénalidomide, le pomalidomide, CC-11006 et CC-10015.

33. Inhibiteur sélectif de JAK1, ou sel pharmaceutiquement acceptable de celui-ci, pour utilisation selon l'une quelconque des revendications 18 à 27, le procédé comprenant en outre l'administration d'un agent thérapeutique supplémentaire choisi parmi une globuline anti-thymocyte, un facteur stimulant les colonies de granulocytes humains (G-CSF) recombinant, le facteur stimulant les colonies de granulocytes-monocytes (GM-CSF), un agent stimulant l'érythropoïèse (ESA) et une cyclosporine.
